Europäisches Patentamt

European Patent Office

Office européen des brevets

(19)

(11) **EP 1 705 249 A1**

(12) **EUROPEAN PATENT APPLICATION**

(43) Date of publication:
**27.09.2006 Bulletin 2006/39**

(51) Int Cl.:
*C12N 15/82* (2006.01)        *C12N 9/02* (2006.01)
*A01H 5/02* (2006.01)

(21) Application number: **05290639.3**

(22) Date of filing: **23.03.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(71) Applicant: **Genoplante-Valor**
**91025 Evry Cedex (FR)**

(72) Inventors:
- **Tahir Abdulrazzak, Nawroz,
District 412
Rezgari,
Sulaimanyah,
Kurdistan (IQ)**
- **Werck, Danièle
67460 Souffelweyersheim (FR)**

(74) Representative: **Colombet, Alain André et al
Cabinet Lavoix,
2, Place d'Estienne d'Orves
75441 Paris Cedex 09 (FR)**

(54) **Method for producing plants with increased biomass**

(57)    The invention relates to a method for producing a plant with increased growth, size or weight, or increased growth speed as well as an increased seed yield, which method comprises transforming a plant cell with a nucleic acid that encodes the p-coumaroyl ester 3'-hydroxylase (C3'H), and generating therefrom a plant that overexpresses C3'H.

EP 1 705 249 A1

**Description**

The present invention relates to a method for obtaining plants with increased biomass, especially plants with increased growth, size and/or weight, increased seed yield, or with increased growth speed, by transgenesis, and the resulting plants, as well as to the use of an expression cassette to obtain increased expression of *p*-coumaroyl ester 3'-hydroxylase in plants.

The activity of *p*-coumaroyl ester 3'-hydroxylase (C3'H) is thought to be essential for the biosynthesis of lignin and many other phenylpropanoid pathway products in plants. This enzyme was recently characterized as a cytochrome P450-dependent monooxygenase, referred to as CYP98A3 in Arabidopsis thaliana, (Schoch et al., 2001, and Franke et al., 2002a).

A mutant of this gene, that shows a single nucleotide substitution (the *Ref8* mutant), was reported in Franke et al., 2002b, and the international patent application WO 02/14497.

The authors of the present invention have now surprisingly found that overexpression of the C3'H protein leads to a dramatic increase of growth, size and weight of the plants, an increased growth speed as well as an increased seed yield.

A subject matter of the present invention is thus the use of a nucleic acid that encodes the *p*-coumaroyl ester 3'-hydroxylase (C3'H) and the encoded C3'H protein, for obtaining a plant that shows increased growth, size and/or weight, increased growth speed or increased seed yield by overexpressing C3'H.

The invention provides a method for obtaining increased growth, increased size, increased weight, increased growth speed, or increased seed yield in a plant, comprising the step of overexpressing a C3'H in cells of said plant.

A particular subject of the invention is a method for producing a plant or a part of a plant with increased growth or increased growth speed, which method comprises transforming a plant cell with a nucleic acid that encodes the p-coumaroyl ester 3'-hydroxylase (C3'H), and generating therefrom a plant that overexpresses C3'H.

Another subject of the invention is a method for producing a plant cell, plant or part of a plant, with increased size or weight, which method comprises the step of transforming a plant cell with the gene that encodes the p-coumaroyl ester 3'-hydroxylase (C3'H), and can comprise the further step of generating therefrom a plant that overexpresses C3'H.

The plant parts can be roots, leaves, trunk, stem, and flowers for instance.

The invention further provides a method for obtaining an increased seed yield, which method comprises transforming a plant cell with a nucleic acid that encodes the *p*-coumaroyl ester 3'-hydroxylase (C3'H), generating therefrom a plant that overexpresses C3'H, and culturing the plant to maturation.

A further subject of the invention is a plant cell, plant or part of a plant, that is transgenic for the nucleic acid that encodes the *p*-coumaroyl ester 3'-hydroxylase (C3'H), and overexpresses it.

More particularly, the invention provides a method, wherein the nucleic acid i) has a sequence showing at least 55% similarity with SEQ ID NO: 1 or its complementary sequence, or ii) has a sequence that hybridizes to SEQ ID No. 1 or its complementary sequence under stringency conditions, and encodes a p-coumaroyl ester 3'-hydroxylase enzyme.

The invention further provides an expression cassette comprising a plant-expressible promoter operably linked to a coding region encoding a C3'H, wherein said promoter is not a C3'H promoter. Said promoter may be a 35S promoter, a ubiquitin promoter or an actin promoter, for example.

The term "transgenic" means that the plant cell or plant comprises within its genome a C3'H encoding nucleic acid that is foreign to such plant or plant cell, or that comprises within its genome an endogenous C3'H coding sequence functionally linked to at least one regulatory region, e.g., promoter, which is not present in the endogenous C3'H gene in that plant or plant cell. Generally the foreign nucleic acid is stably integrated within the genome such that the polynucleotide is passed on to successive generations. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

The term "overexpression" is here intended to mean both an increase in the amount of C3'H compared to the amount expressed in a normal plant, and an ectopic expression of this enzyme, in a tissue or a compartment and/or at a developmental stage where it is not normally expressed. It encompasses the situation when the C3'H is endogenous or heterologous, i.e., when it comes from an organism, such as a plant, different from the host cell, or where at least one transcription regulatory region of the C3'H is not present in the endogenous C3'H gene. The amount of C3'H protein expressed in a plant or plant cell can be measured by measuring the C3'H enzymatic quantity of plant cell extracts, by using for instance Q-PCR.

An "increased" growth, size and/or weight, an "increased" seed yield, or "increased" growth speed means that the growth, size, weight, seed yield or growth speed observed in the transgenic plants that overexpress C3'H is significantly superior to the normal plants of the same species that have not been transformed with a C3'H encoding nucleic acid, when cultivated under the same growth conditions.

In the methods or plants of the invention, the C3'H encoding nucleic acid may be a sequence heterologous with respect to said plant, or may belong to the same species, as long as it can be expressed in plants in amounts greater than the amount conventionally produced by a non transformed plant, any C3'H encoding nucleotide sequence can be used (i.e. wild-type to the C3'H gene).

Advantageously, the C3'H encoding nucleic acid used to transform the cells or plants of the invention comprises a nucleic acid encoding the protein of SEQ ID No. 2, e.g., the CYP98A3 cDNA sequence of SEQ ID No. 1.

The nucleic acid to be transferred or the protein to be overexpressed can also be a CYP98A3 homologous nucleic acid or protein.

The term "homologous" refers to any nucleic acid or protein having one or more sequence modification(s) with respect to all or part of the CYP98A3 sequence, while retaining most or all of the C3'H enzyme activity.

In one embodiment of this invention, overexpression of C3'H in plants can be achieved using nucleic acids or proteins comprising any one of the following nucleotide or amino acid sequences (encoding C3'H of other plant species than *Arabidopsis thaliana*): CYP98A1, *Sorghum bicolor* Genbank access number : 048956 (SEQ ID No: 15 and 16, for the nucleotide and the aminoacid sequence respectively) ; CYP98A2, *Glycine max* Genbank access number : AAB94587 (SEQ ID No: 17 and 18, for the nucleotide and the aminoacid sequence respectively), CYP98A4, *Oryza sativa* Genbank access number : AAU44038 (SEQ ID No: 19 and 20, for the nucleotide and the aminoacid sequence respectively) ; CYP98A6, *Lithospermum erythrorhizon* Genbank access number : AB017418 (SEQ ID No: 21 and 22, for the nucleotide and the aminoacid sequence respectively) ; CYP98A10 *Triticum aestivum* Genbank access number : CAE47489 (SEQ ID No: 23 and 24, for the nucleotide and the aminoacid sequence respectively); CYP98A11 *Triticum aestivum* Genbank access number : CAE47490 (SEQ ID No: 25 and 26, for the nucleotide and the aminoacid sequence respectively) ; and CYP98A12 *Triticum aestivum* Genbank access number No: CAE47491, (SEQ ID No: 27 and 28, for the nucleotide and the aminoacid sequence respectively) ; CYP98A13v1 Genbank access number : AAL99200, (SEQ ID No: 29 and 30, for the nucleotide and the aminoacid sequence respectively) and v2 Genbank access number: AAL99201 (SEQ ID No: 31 and 32, for the nucleotide and the aminoacid sequence respectively), *Ocimum basilicum*) ; CYP98A14, *Solenostemon scutellarioides* Genbank access number : CAD20576 (SEQ ID No: 33 and 34, for the nucleotide and the aminoacid sequence respectively) ; CYP98A19, *Pinus taeda Genbank access number :* AAL47685 (SEQ ID No: 35 and 36, for the nucleotide and the aminoacid sequence respectively) ; CYP98A21, *Ammi majus* Genbank access number : AAT06912, (SEQ ID No: 37 and 38, for the nucleotide and the aminoacid sequence respectively) ; CYP98A23, *Camptotheca acuminata* Genbank access number : AAS57921 (SEQ ID No: 39 and 40, for the nucleotide and the aminoacid sequence respectively).

One embodiment of the invention includes the overexpression of C3'H in a monocot, particularly rice (Oryza sativa), by overexpressing the C3'H of SEQ ID No. 20, 24, 26 or 28 in rice plant cells.

The skilled person knows how to identify C3'H enzymes in other species, by comparing SEQ ID No. 1 with the sequences in other species, with a computer program such as Blast (www.ncbi.nlm.nih.gov) and Fast DB with parameters by defaults.

Those algorithms are described in "Current methods in sequencing and synthesis Methods and Applications", pages 127-149, 1988, Ala. R. Liss, Inc.

Homologous sequences are preferably defined as:

i) DNA or protein sequences that show a similarity or identity of at least 55%, preferably at least 70%, preferably at least 80%, even more preferably at least 90% to the sequence SEQ ID No. 1 or 2, respectively; or
ii) sequences which hybridize with the sequence of SEQ ID No. 1, or the sequence complementary thereto, under stringency hybridization conditions, e. g. low stringency, or
iii) sequences encoding a C3'H enzyme comprising the amino acid sequence of SEQ ID No. 2, or a homologous amino acid sequence, e.g., any amino acid sequence with C3'H enzymatic activity and having at least 60%, preferably at least 70%, preferably at least 80%, even more preferably at least 90%, sequence identity to the sequence of SEQ ID No. 2.

Preferentially, such a homologous nucleotide sequence hybridizes specifically to the sequences complementary to the sequence SEQ ID No. 1, under stringent conditions. The parameters which define stringency conditions depend on the temperature at which 50% of the paired strands separate (Tm).

For sequences comprising more than 30 bases, Tm is defined by the equation:

$$Tm=81.5+0.41(\%G+C)+16.6 Log \text{ (cation concentration)} -0.63(\%formamide) - (600/number\ of\ bases) \text{ (Sambrook } et\ al., 1989).$$

For sequences less than 30 bases long, Tm is defined by the equation:

$$Tm = 4(G+C) + 2(A+T).$$

Low stringency hybridization conditions are those wherein hybridization is observed using a hybridization temperature of 5 to 10°C below Tm, and the hybridization buffers are solutions of high ionic strength, such as a 6 x SSC solution.

The term "sequence similarity" used above differs from complete resemblance, or identity, between nucleotide or amino acid sequences compared, in that different but functionally or structurally similar residues (nucleotides or amino acids) are included in the determination of the percentage similarity. A search for similarity in nucleic acid sequences distinguishes, for example, purines and pyrimidines. The above standard programs can determine the percentage of similarity or identity.

A nucleic acid is homologous to the cDNA represented in SEQ ID No. 1, as used herein, when it comprises a nucleotide sequence which differs from sequence SEQ ID No. 1 by mutation, insertion, deletion or substitution of one or more bases, or by degeneracy of the genetic code, as long as it encodes a polypeptide having the activity of the C3'H enzyme. A protein is homologous to the C3'H represented in SEQ ID No. 2, as used herein, when it includes an amino acid sequence which differs from sequence SEQ ID No. 2, by mutation, insertion, deletion or substitution of one or more amino acids, as long it is a polypeptide having the activity of the C3'H enzyme.

Homology is generally determined using a sequence analysis program (for example, Sequence Analysis Software Package of the Genetics Computer Group, University of Wisconsin Biotechnology Center, 1710 University Avenue, Madison, WI 53705). Similar amino acid sequences are aligned so as to obtain the maximum degree of homology (i.e., identity or similarity, as defined above). For this purpose, it may be necessary to artificially introduce gaps into the sequence. Once the optical alignment has been carried out, the degree of homology is established by recording all the positions for which the amino acids of the two sequences compared are identical, relative to the total number of positions.

The expression "the activity of the C3'H enzyme" or "the C3'H enzymatic activity", as used herein, refers in particular to its activity of 3'-hydroxylation of phenolic esters in plants, which can be determined by incubating the homologous protein with 5-O-(4-coumaroyl)D-quinate or 5-O-(4-coumaroyl)shikimate, in the presence of NADPH, and assaying the conversion of the quinate and shikimate esters into a more hydrophilic product, as described in Schoch et al., 2001 (incorporated herein by reference).

The nucleic acid that encodes C3'H is typically inserted into a nucleotide construct, called expression cassette, in which it is functionally linked to elements which allow the expression thereof and, optionally, the regulation thereof.

Among these elements, mention may in particular be made of transcription promoters, activators and/or terminators.

In a particular embodiment, the plant cell is transformed with an expression cassette comprising a C3'H encoding sequence and a tissue-specific promoter. Expression in lignin-containing tissues or in inflorescences may be of particular interest, as well as expression selectively or preferentially in flowers or seed. A root-specific promoter may also be useful. Expression in root tissue could be accomplished by utilizing the acid chitinase gene (Samac et al., 1990), or the root specific subdomains of the CaMV35S promoter that have been identified (Benfield et al., 1989).

Among the transcription promoters as can be used, one may cite :

- A 35S promoter, or the double constitutive 35S promoter (pd35S) of the cauliflower mosaic virus (CaMV), e;g., as described in Kay et al., 1987 ;
- the PCRU promoter of the radish cruciferin that directs the expression of the associated sequences only in seeds of the transgenic plant ;
- the PGEA1 and PGEA6 promoters that correspond to the 5' non coding region of the genes of the stocking protein of seeds (GEA1 and GEA6 respectively), in *Arabidopsis thaliana* (Gaubier et al., 1993), that direct a specific expression in seeds ;
- the PSP chimeric promoter (Ni et al., 1995) that is a fusion of a triple repetition of a transcription activating element of the promoter of the octopin synthase gene in *Agrobacterium tumefaciens,* with a transcription activating element of the mannopin synthase promoter and the mannopin synthase promoter in *Agrobacterium tumefaciens ;*
- a rice actin promoter, optionally followed by the rice actin intron (PAR-IAR), e.g., the promoter contained in plasmid pAct1-F4 described by Mc Elroy et al., 1991 ; Mol. Gen. Genet., 231, 150-160)
- the wheat HMGW promoter (High Molecular Weight Glutenin) ;
- the promoter of the γzein gene in corn (Pγzein) contained in plasmid pγ63, that directs the expression in endosperm of seeds.

Other suitable plant-expressible promoters in accordance with this invention include, but are not limited to: promoters from the ubiquitin family (e.g., the maize ubiquitin promoter of EP 0 342 926), a rice actin promoter such as the promoter described by Me Elroy et al., (already mentioned above) or the promoter described in US 5,641,876; any one of promoters of the Cassava vein mosaic virus (WO 97/48819), any one of the pPLEX series of promoters from Subterranean Clover Stunt Virus (WO 96/06932), or an alcohol dehydrogenase promoter, e.g., pAdh 1S (GenBank accession numbers X04049, X00581).

Among the terminators which can be used in the constructs of the invention, mention may in particular be made of the 3' end of the nopaline synthase gene of *Agrobacterium tumefaciens.*

The expression cassette is inserted into a nucleotide vector, such as a plasmid, which may also comprise a marker gene, for example a gene for selecting a transformed plant from a plant which does not contain the foreign DNA transfected. As a marker gene, mention may in particular be made of a gene which confers resistance to an antibiotic (Herrera-Estrella et al., 1983) or resistance to a herbicide (EP 242 246).

The vector thus constructed can be used to transform host cells, according to techniques known to those skilled in the art.

Mention may in particular be made of the methods of direct transfer of genes into plant cells, such as direct microinjection into plant embryoids (Neuhaus et al., 1987), infiltration under vacuum (Bechtold et al., 1993) or electroporation (Chupeau et al., 1989), or alternatively direct precipitation using PEG (Schocher et al., 1986) or bombarding particles covered with the plasmid DNA of interest, using a gun (M. Fromm et al., 1990).

According to another embodiment of the method of the invention, the plant cells are transformed with a vector as defined above, transferred into a cellular host capable of infecting said plant cells while allowing the integration, into the genome of the latter, of the nucleotide sequences of interest initially contained in the abovementioned vector genome. Advantageously, the cellular host used is a bacterial strain, such as *Agrobacterium tumefaciens,* in particular according to the method described in the article by An et al., (1986), or *Agrobacterium rhizogenes,* in particular according to the method described in the article by Guerche et al., (1987).

For example, the plant cells may be transformed by transferring the T region of the tumor-inducing extrachromosomal circular plasmid Ti of *Agrobacterium tumefaciens,* using a binary system (Watson et al., 1994). To do this, two vectors are constructed. In one of these vectors, the T region has been removed by deletion, with the exception of the right and left borders, the gene of interest and a marker gene being inserted between them (the latter to allow easy selection of transformed plant cells). The other partner in the binary system is an auxiliary Ti plasmid, which is a modified plasmid which no longer has a T region but still contains the virulence genes *vir* required for transformation of the plant cell.

To transform monocots such as rice *(Oryza sativa),* the method described by Ishida et al., (1996) may be used or any one of the methods described in Hiei *et al.* (1994); Hiei *et al.* (1997), in US Patent 5,641,664 or 5,679,558, or in Christou *et al.* (1991).

According to another protocol, the transformation is carried out according to the method described by Finer et al., (1992) using a particle gun with tungsten or gold particles.

The plants so produced overexpress C3'H.

Such plants or parts of a plant are advantageously obtainable by the method described above, wherein a plant cell is transformed with a nucleic acid that encodes the C3'H enzyme, and cultured, whereby a plant that overexpresses C3'H is obtained.

As examples of transgenic plants, mention may be made of oilseed rape , canola, or cereals, in particular maize, wheat, barley, sorghum, rye and rice, preferentially maize, and also pea, soybean, cotton, potato, tomato, tobacco, coffee and cacao tree.

It is indeed of particular interest to increase yields of such crops.

Other plants of interest include trees, in particular poplar, eucalyptus, pine, Ocotea, Piper, Cumaru, Pau rosa. Such trees are useful for paper, wood or perfume industry.

The increase of root growth favours the collection of secondary metabolites with therapeutic and other industrial applications produced in medicinal and aromatic plants (e.g. Taxus, Catharanthus, Ruta, Datura species, etc). In that respect, WO 01/33942 describes a method for producing molecules from plants, wherein the plants are cultivated in soil-less conditions and are supplied with a nutrient solution and/or sprayed with a leaching liquid. The solution or liquid is subsequently recovered and the molecules that it contains are extracted therefrom.

The increased growth and growth speed of inflorescences is advantageous for the production of flowers, in the flower industry, horticulture and in perfume industry, especially for obtaining methyleugenol perfumes.

These transgenic plants encompass both the first generation plants and their descendants that contain the C3'H expression cassette of the invention (lines or hybrids, in particular).

The parts of the plant include any tissue or organ, such as roots, flowers, stems, trunk, leaves, fruits or seeds.

The present invention particularly comprises the seeds that show an increased expression of C3'H, obtained by specific expression of a C3'H encoding sequence in the seed.

In one embodiment of the invention, the expression cassette of the invention is used to overexpress C3'H in a plant or plant cell. Such use leads to increased biomass, plant growth, size, weight, yield and/or growth speed.

In another embodiment of the invention, the expression cassette of the invention is used to increase biomass, plant growth, size, weight, yield and/or growth speed in a plant cell, a plant or a part of plant.

The figures and examples illustrate the invention without limiting its scope.

## DESCRIPTION OF THE DRAWINGS :

Figure 1 is a comparison of two photographs of 15-day-old plants *(Arabidopsis* Wassilewskija WS) Figure 1A : wild-type ; Figure 1B : CYP98A3 overexpression. This figure shows the increase in root growth as well as increased growth of the rosette.

Figure 2 is a photograph of leaves from wild-type and CYP98A3 overexpressing 15-day-old *Arabidopsis* WS plants, showing the increase in leaf surface in the transgenic plant.

Figure 3 is a photograph of wild-type and CYP98A3 overexpressing 11-week-old WS plants, showing the increased size of the transgenic plant. Increased size is also observed for transformed Col-0 pants.

Figure 4 is a graph representing the biomass of 15-day-old wild-type and overexpressing WS plantlets (mean on 38 plantlets).

Figure 5 is a graph representing the biomass of the seeds collected from mature wild-type and overexpressing WS plants (mean on 15 plants).

Figure 6 includes a graph (Figure 6A) and photographs showing the increase in cell size in the overexpressing WS plants (Figure 6C) compared to wild-type (Figure 6B).

Figure 7 is a photograph of phloroglucinol-stained stem cuts that shows that there is no major modification in lignified tissues in the transformed plants.

Figure 8 is graph showing that the increase in plant biomass depends on the level of expression of *CYP98A3,* as measured in different lines of 15-day-old WS overexpressing plants (average of 20 plants per line) by real-time quantitative PCR.

Figure 9 is a graph showing that the increase in *CYP98A3* expression in the 15-days-old WS overexpressing plants is correlated with an increased expression of the other genes of the phenylpropanoid pathway, as measured by real-time including phenylalanine ammonia lyase (PAL1), cinnamate 4-hydroxylase (C4H), and hydroxycinnamoyl-CoA:shikimate hydrodroxycinnamoyl transferase (HCT). Data are average of three experiments.

Figure 10 is a map of the expression plasmid pB7WG2.0.

## EXAMPLES :

### Example 1. Overexpression of C3'H in a dicot plant.

#### A - Plant growth

Seeds from *Arabidopsis thaliana* Wassilewskija (WS) or Columbia 0 (Col-0) were kept at 4°C for four days, and then washed 5 minutes in 70% ethanol. After 20 min incubation with gentle shaking in 20% bleach supplemented with 0.1 % Triton X100, they were washed 4 times in sterile distilled water, and germinated on Murashige and Skoog (Duchefa) medium containing 0.8% Pastagar (Sigma Aldrich), 1% sucrose (Carlo Erba Reagenti), with or without 10 $\mu$glmL Basta (DL-Phophinotricine, Duchefa), and 500$\mu$g/mL carbenicillin (Duchefa). For vertical growth, 8-day-old plants were transferred on square plates containing Murashige and Skoog medium with 0.8% Pastagar and 1% sucrose. Plates are placed vertically in a 21°C growth chamber with a photoperiod of 16h light and 8h dark for 7 days.

For plant transformation, Col-0 or WS seeds were grown on soil at 21°C under a photoperiod of 16h light and 8h dark. After 13 weeks, watering was interrupted and seeds were collected from the dry plants.

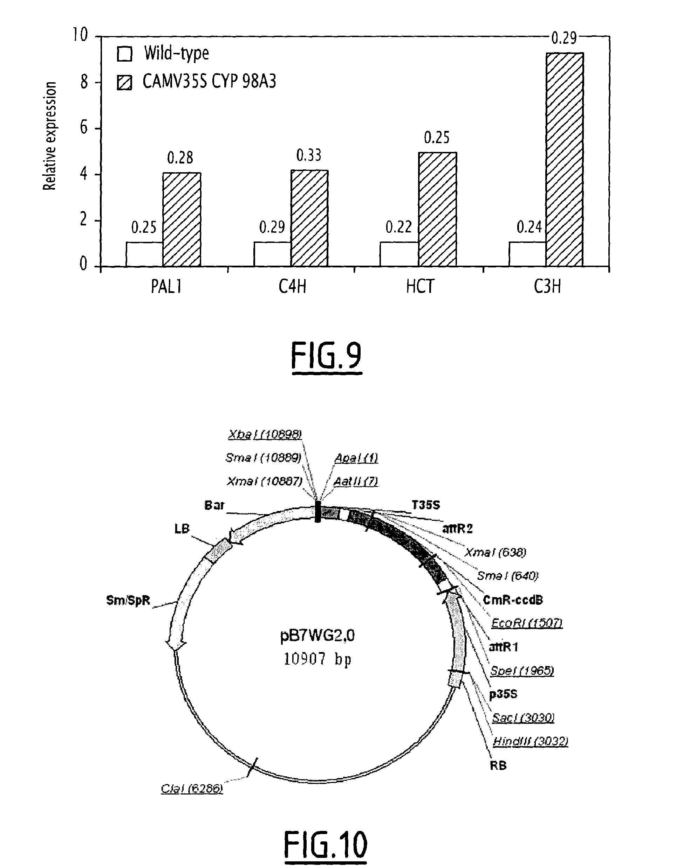

#### B - Construction of the expression vector (pB7WG2 *CAMV 35-CYP98A3,* Figure 10).

The *CYP98A3* cDNA (SEQ ID NO°1) in a pGEM-T vector was amplified using specific primers with added *attB* recombination sites (Gateway™ Technology), sense primer 98A3b1: 5'-GGGGACAAGTTTGTACAAAAAA GCAGGCTATGTCGTGGTTTCTAATAGCGGTG-3' (SEQ ID No. 3), antisense primer 98A3b2: 5'-GGGGACCACTTTGTACAAGAAAGCTGGGTTTACATAT CGTAAGGCACGCGTTT -3' (SEQID No. 4) (Karimi et al., 2002).

The PCR reaction was carried out using as template 200ng of the plasmid DNA, 10$\mu$M of each primer, and High Fidelity PCR Master mix (Roche) according to the recommendations of the manufacturer. After 3 min of heating at 96°C, 18 cycles of amplification were carried out as follows : 1 min denaturation at 95°C, 1 min at 60°C, and 2 min extension at 72°C. The reaction was completed by a 10 min extension at 72°C. The PCR product was purified on a 1 % agarose gel, and cloned into the pDONR201 donor vector (Invitrogen, Gateway™ Technology). The ligation mix (20 μL) contained 300ng of the PCR product, 300ng of the pDONR201 vector, BP Clonase™ reaction buffer, the volume being adjusted to 16 μL with sterile water according to the instructions of the manufacturer. The reaction was started with 4 μL of BP Clonase™ enzyme mix. It was incubated at 25°C for 18h, and stopped by 10 min incubation at 37°C with proteinase K (4 μg/20μL of ligation medium). Electrocompetent bacteria (DH5α) were transformed by electroporation (McCormac et al. 1998) with 1 μL of the ligation medium. Plasmid DNA of the positive colonies selected on kanamycin (100μg/mL) was prepared (NucleoSpin[R] Plasmid) and sequenced for selection of a clone corresponding to the expected sequence. After extraction of the pDONR201 plasmid from the selected clone, the insert was transferred to the destination expression vector pB7WG2 (Karimi et al., 2002), for insertion of the *CYP98A3* cDNA dowstream of the 35S promoter of the Cauliflower mosaic virus (CaMV), in a reaction mix (20 μL) containing 300ng of pDONR201 with the *CYP98A3* insert, 800ng of pB7WG2, LR Clonase™ buffer, the volume being adjusted to 16 μL with sterile water. Four μL of LR Clonase™ enzyme mix were added, before incubation at 25°C for 18h according to the protocol recommended by Invitrogen. The reaction was stopped by 10 min incubation at 37°C in the presence of proteinase K (4 μg/20 μL of reaction medium). The plasmid DNA prepared (NucleoSpin[R] Plasmid) from the positive colonies selected on spectino-mycin (100μg/mL) was double-checked by digestion and PCR.

**C - Transformation of *Arabidopsis thaliana.***

The pB7WG2 vector was introduced into *Agrobacterium tumefaciens* LBA 4404 virG by electroporation, and stabilization of the construct in A. *tumefaciens* was confirmed by digestion of the plasmid isolated from *Agrobacterium,* and by PCR with specific primers. The plants were then transformed using the « floral dip » method (Clough and Bent, 1998).

D - Selection of the transformed plants.

The T0 seeds were collected and grown on soil at a density of 1500 plants per 25 x 50 cm pot. Plantlets were sprayed with 250 mg/L of Basta (AgroEvo) 5, 14 and 21 days after germination. Transgenic plants, resistant to the herbicide were transferred into individual pots and seeds collected as above. For *in vitro* selection, seeds of the T1 plants were grown on Murashige and Skoog medium supplemented with 0.8% Pastagar, 1% sucrose, 10 μg/mL Basta and 500μg/mL carbenicillin.

**E - Confirmation of CYP98A3 overexpression in the selected transformants.**

**1- RNA preparation.**

Total RNAs from 15-day-old plants were extracted using the Trizol method described by Chomczynski and Sacchi (1987).

**2- cDNA synthesis (RT).**

Approximately 2.5 μg RNA from all samples were first retro-transcribed into cDNA. RNA was mixed with 0.5 μg oligo(dT) primers, dNTPs (0.83 mM final), made up to 12 μL with sterile DEPC water. The mix was incubated at 65°C for 5 min and kept on ice, before addition of first strand buffer, 10 mM DTT, sterile water to obtain a final volume of 19 μL. One μL of Superscriptll (200u/μL) was added, and the reaction incubated 50 min at 42°C, and then 15 min at 70°C for inaction of the enzyme.

**3- Quantitative PCR**

The cDNA resulting from RT is used for real-time quantitative PCR (performed on Applied Biosystems GeneAmp 5700 Sequence Detection System). Couple of primers, specific of the genes studied were designed, based on sequences available in GenBank and according to the following criteria: a size of 20-30 nt, a Tm around 60°C, and an amplicon size of about 50-200 nt. Primers were chosen with help of the software Primer Express :

PAL1 forward (AY303128):
5'-TCTGCGAAAAGGATTTACTCAAAGTT-3' (SEQ 10 No. 5)
PAL1 reverse (AY303128):
5'-CTACAAGGATCATCCGCGTATGT-3' (SEQ ID No.6)

C4H forward (D78596)
5'-CCTCCAGGACAGTCTAAAGTGGATACTA-3' (SEQ ID No.7)
C4H reverse (D78596):
5'-CGATTATGGAGTGGTTAAGGATGTG-3'(SEQ ID No.8)
HCT forward (BX830476):
5'-TCTTTATGGGACCTGGTGGAATT-3'(SEQ ID No.9)
HCT reverse (BX830476):
5'-GATAAGCTGCCATCATTAGTAGGACTTG-3' (SEQ ID No.10)
C3'H forward (AC002409, T20B5.9):
5'-AAGTCTAGTGGAGCGAAACAGCATT-3' (SEQ ID No.11)
C3'H reverse (AC002409, T20B5.9):
5'-TCCTCACTAAGATCATACTGATCCTTCA-3' (SEQ ID No.12)
ACTIN II forward (AY096397):
5'-TTCAATGTCCCTGCCATGTATG-3' (SEQ ID No.13)
ACTIN II reverse (AY096397) :
5'-AATACCGGTTGTACGACCAC-3' (SEQ ID No.14)

For each quantitative PCR, 5μL of a 5 fold dilution of the cDNA were mixed with the primers, 0.3 μM each, and 13 μL of SYBR Green mix (Applied Biosystems). The volume was adjusted to 25 μl with sterile water, and the mix maintained on ice. The PCR was initiated with a « hot start », and a first step at 95°C for 10 min for denaturation, followed by 40 cycles of 15 sec at 95°C and 1 min at 60°C. A first step of sample standardization by quantitative PCR on a gene constitutively expressed was performed for adjustment of cDNA amounts in the PCR reaction. The reference gene used was *Actin II.* The adjusted cDNA dilutions were used for quantification of the gene expression. Each quantitative PCR was repeated 3 times.

**F- Lignin histochemistry**

Inflorescence stems of 8 week-old plants were cleared in ethanol and sectioned with a razor-blade (section cut to approximately 200 μm thickness). The stem sections were stained with phloroglucinol-HCl (Wiesner reagent: 1 % (w/v) phloroglucinol in 6N HCl) (Adler et al., 1948) and observed under a microscope (objective: 10 x).

**G- Determination of the area of the epidermal cells**

Leaves were cleared in successive baths in ethyl alcohol 30, 50, 70, 90, 100, 100, 100 % (30 min each), and left overnight in 100 % ethyl alcohol. They were then incubated for two hours in the solution of Hoyer (HCl / Arabic gum / glycerol / water; 100:7.5:5:60). The cells of the epidermal layer was visualized in Nomarsky (microscope Nikon E800, objective 40x in immersion of oil) and photographed with a camera Sony DXC950. Average cell area in comparable in different and comparable leaf zones in wild-type and mutant was analyzed using the software Image J.

**H - Results**

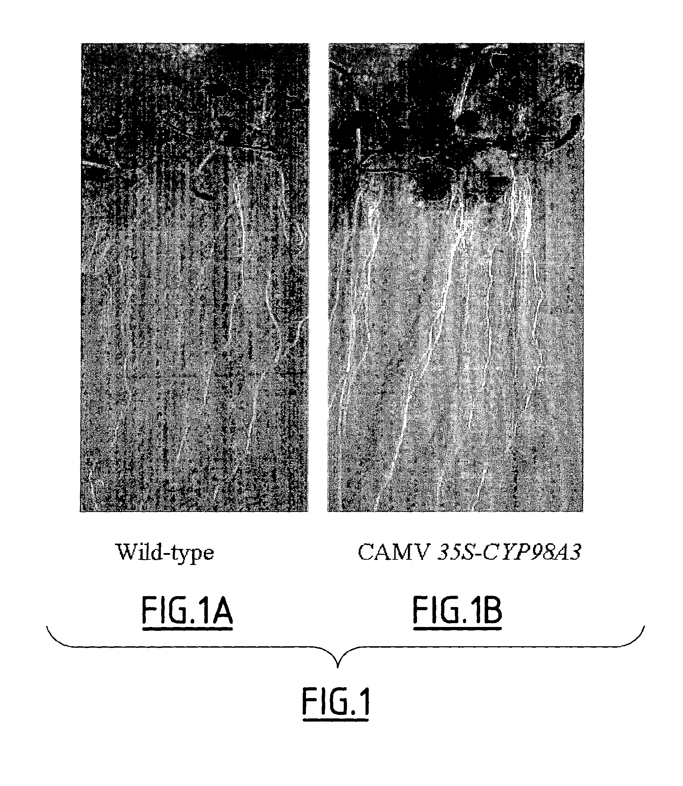

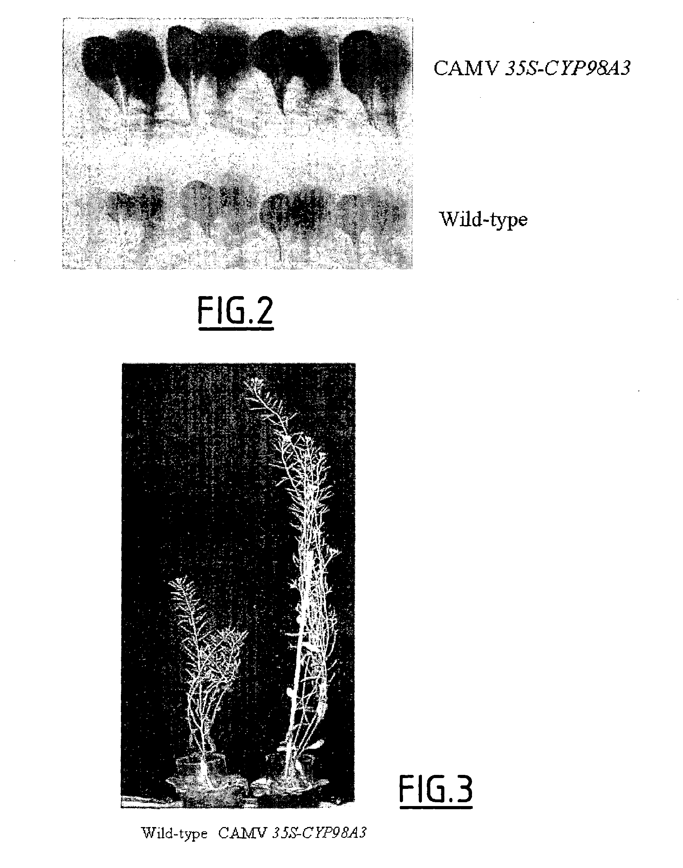

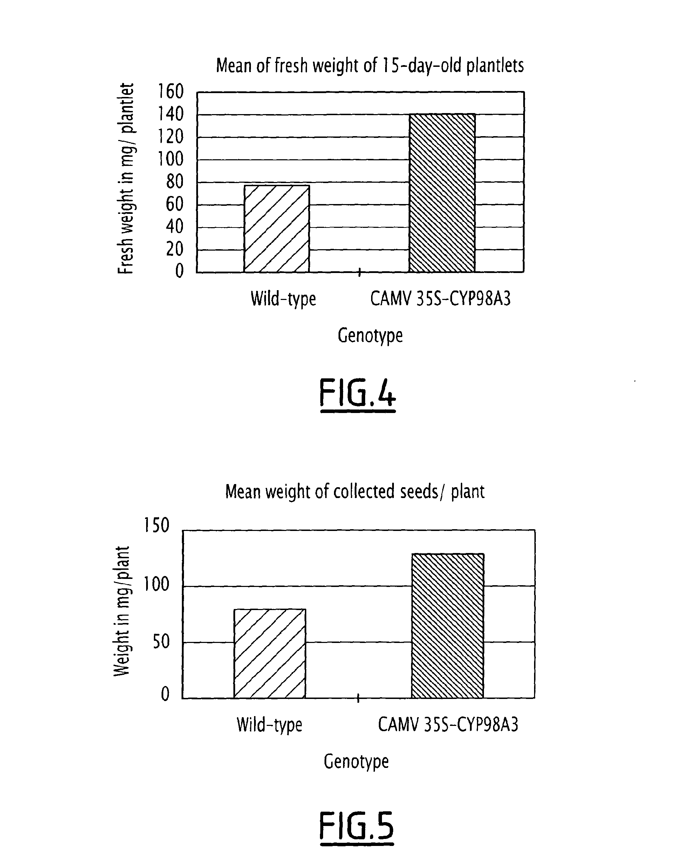

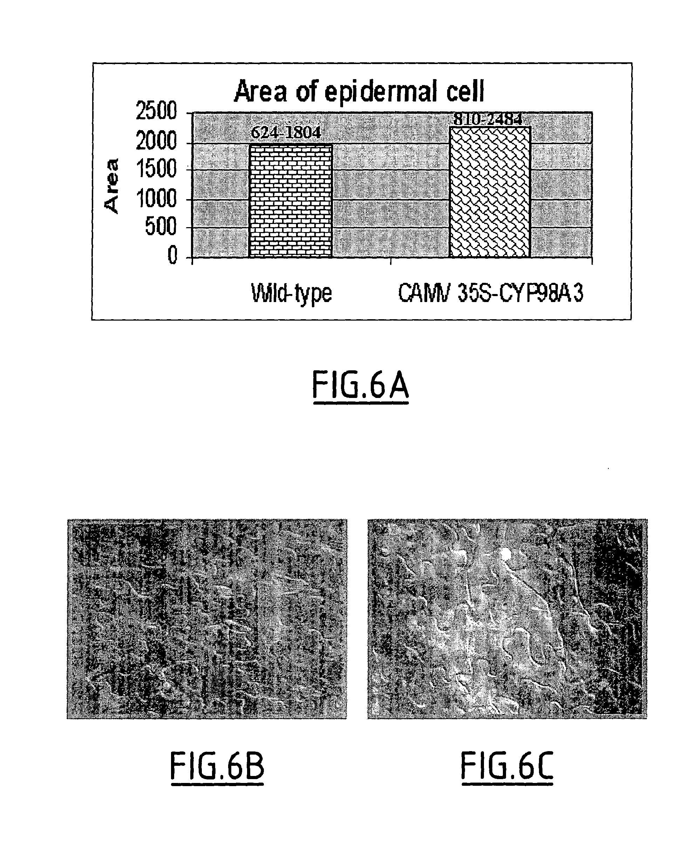

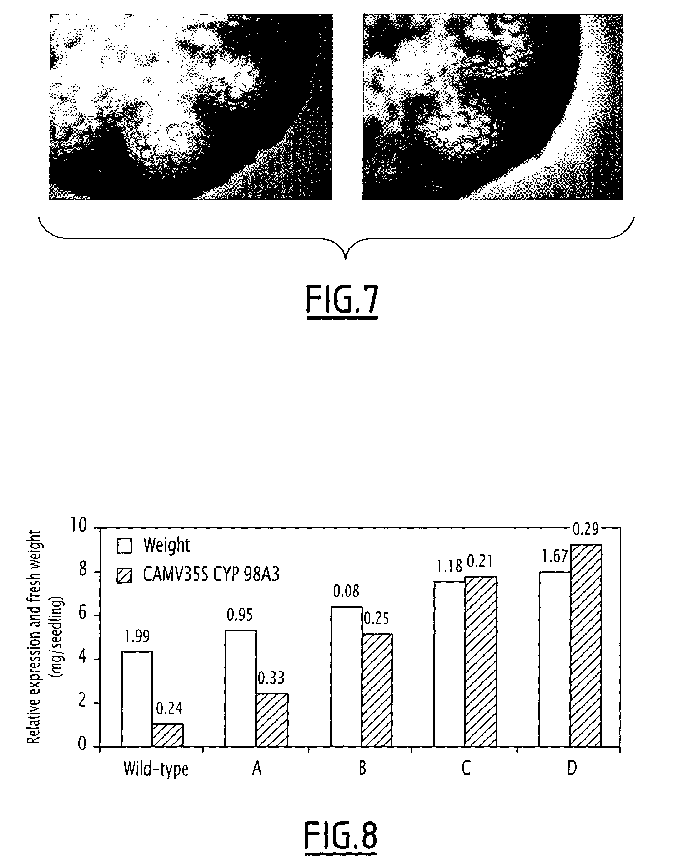

The results are shown on Figures 1 to 7. The CYP98A3 overexpression leads to an increase in root growth (Figure 1), as well as in leaf surface (Figure 2). The size of the transgenic plant is superior to the size of the wild-type plant of same age (Figure 3). The fresh weight of the plantlet is also significantly increased (Figure 4). The seed yield in the CYP98A3 overexpressing plants was also shown to be superior (x 1.6) to the wild-type plants (Figure 5). In addition to increased plant development, a small increase in the average size of the epidermal cells can be detected (Figure 6). No increased development in lignified tissues or lignin accumulation is detected in phloroglucinol stained stem cuts (Figure 7). The increase in plant growth directly depends on the level of expression of CYP98A3 (Figure 8). Increased CYP98A3 expression triggers an increased expression of several other genes in the upstream phenylpropanoid pathway (Figure 9).
The C3'H gene overexpression thus causes an increased cell growth and an increased growth and development of the whole plant.

**Example 2. Overexpression of C3'H in a monocot plant.**

In another aspect of the invention different expression cassettes are used to transform rice plants and obtain overexpression of C3'H in a monocot plant.
Among the three sequences of wheat (SEQ ID N°23, 25 and 27, that encodes proteins of SEQ ID N°24, 26 and 28 respectively), the SEQ ID N°25 was chosen to transform rice. SEQ ID N°24 shows 67% identity with SEQ ID N°2. SEQ ID N°26 shows 67% identity with SEQ ID N°2. SEQ ID N°28 shows 65% identity with SEQ ID N°2. All three proteins were experimentally confirmed to have C3'H activity, the most active being the protein resulting from SEQ 26.

An expression cassette comprising the coding sequence of SEQ ID No. 25 under control of a 35S promoter and the nopaline synthase transcription termination signal was used together with a glufosinate resistance expression cassette to transform rice plants using the procedure described in Hiei *et al.* (1997). The expression of the CYP98A11 protein in rice plants lead to a significantly enhanced growth and yield (i.e., more seed weight) compared to control plants only containing the glufosinate resistance gene (planted at the same time under the same conditions).

Using similar procedures also the coding sequence of SEQ ID No. 19 was overexpressed in rice and a significant effect on growth and yield is found in those plants with an increased amount of C3'H, when compared to control plants, grown under the same conditions.

Expression cassettes containing the rice actin promoter (Zhang et a/.,1991) are also constructed, wherein the coding sequence is a DNA encoding the protein of SEQ ID No. 20 or 26, and the nopaline synthase transcription termination sequence is present. A glufosinate resistance gene is used herein as marker gene. The growth and yield of rice plants transformed with this expression cassette are evaluated.

<u>REFERENCES</u>

- An et al., 1986, Plant Physiol., 81: 86-91
- Adler E., Bjorkquist, K.J., and Haggroth, S. (1948) Acta. Chem. Scand., 2,93-94.
- Bechtold et al., 1993, C.R. Acad. Sci. Paris, Life sciences, 316: 1194-1199
- Benfey et al., 1989 EMBO J, 5:2195-2202
- Chomczynski et al. (1987). Analytical Biochem. 162: 1256-159.
- Christou et al. (1991) Biotechnology 9:957
- Clough et al. (1998) Plant J. 16(6), 735-743.
- Chupeau et al., 1989, Biotechnology, 7 (5): 503-508
- Finer J., (1992), Plant Cell Report, 11: 323-328
- Franke et al., the Plant Journal, 2002a, 30(1):47-59
- Franke et al., 2002, The Plant Journal, 2002b, 30(1):33-45
- Fromm M. et al., 1990, Biotechnology, 8: 833-839
- Gaubier et al., 1993 Mol. Gen., 238, 409-418
- Goujon T., Sibout R., Pollet B., Maba B., Nussaume L., Bechtold N., Lu F., Ralph J., Mila I., Barrière Y., Lapierre C., and Jouanin L. (2003), Plant Mol. Biol, 2003, 51, 973-989.
- Guerche et al., (1987), Mol. Gen. Genet., 206: 382
- Herrera-Estrella et al., 1983, EMBO J. 2, 987-995
- Hiei et al. (1994) Plant J 6:271-282;
- Hiei et al. (1997) Plant Mol Biol. 35:205-218
- Ishida et al., (1996), Nature Biotechnology, 14: 745-750
- Karimi et al., (2002) Trends Plant Sci. 7(5):193-5.
- Kay et al., 1987, Science, 236: 1299-1302
- McCormac et al. (1998) Mol Biotechnol. 9:155-159. McElroy et al., 1991, Mol. Gen. Genet., 231: 150-160 Morant et al., 2005, submitted
- Neuhaus et al., 1987, Theoretical and Applied Genetics, 75 (1): 30-36
- Ni et al., 1995, Plant J., 7, 661-676
- Samac et al., 1990, Plant Physiol. 93 :907-914
- Schoch et al., 2001, The Journal of Biological Chemistry, 276(39):36566-36574
- Schocher et al., 1986, Biotechnology, 4: 1093-1096
- Watson et al., 1994, ADN recombinant [Recombinant DNA], Pub. De Boeck University, 273-292
- Zhang et al., 1991, The Plant Cell 3, 1155-1165.

## Claims

1. A method for producing a plant or part of a plant with increased growth or increased growth speed, which method comprises transforming a plant cell with a nucleic acid that encodes a *p*-coumaroyl ester 3'-hydroxylase (C3'H), and generating therefrom a plant that overexpresses C3'H.

2. A method for producing a plant or part of a plant, with increased size or weight, which method comprises transforming a plant cell with a nucleic acid that encodes the *p*-coumaroyl ester 3'-hydroxylase (C3'H), and generating therefrom a plant that overexpresses C3'H.

3. The method of claim 1 or 2, wherein the plant part is a root.

4. The method of claim 1 or 2, wherein the plant part is a leaf.

5. A method for obtaining an increased seed yield, which method comprises transforming a plant cell with a nucleic acid that encodes a p-coumaroyl ester 3'-hydroxylase (C3'H), generating therefrom a plant that overexpresses C3'H, and culturing the plant to maturation.

6. A method for obtaining increased growth, increased size, increased weight, increased growth speed, or increased seed yield in a plant, comprising the step of overexpressing a C3'H in cells of said plant.

7. The method of any of claims 1 to 5, wherein the C3'H comprises the amino acid sequence of SEQ ID No. 2.

8. The method of any of claims 1 to 5, wherein the nucleic acid i) has a sequence showing at least 55% similarity with SEQ ID NO:1 or its complementary sequence, or ii) has a sequence that hybridizes to SEQ ID No. 1 or its complementary sequence under stringency conditions, and encodes a p-coumaroyl ester 3'-hydroxylase enzyme.

9. The method of any of claims 1 to 7, wherein the C3'H encoding nucleic acid is heterologous with respect to said plant.

10. The method of any of claims 1 to 8, wherein the plant cell is transformed with an expression cassette comprising a C3'H encoding sequence and a tissue-specific promoter.

11. The method of any of claims 1 to 9, wherein the plant is oilseed rape .

12. The use of a nucleic acid that encodes a p-coumaroyl ester 3'-hydroxylase (C3'H), for obtaining a plant or plant part that overexpresses C3'H, and shows increased growth, increased size, increased weight, increased growth speed, or increased seed yield.

13. The use of a nucleic acid that encodes a *p*-coumaroyl ester 3'-hydroxylase (C3'H), for obtaining increased growth, increased size, increased weight, increased growth speed, or increased seed yield in a plant or plant part.

14. The use of a C3'H for obtaining increased growth, increased size, increased weight, increased growth speed, or increased seed yield in plants.

15. The use of any of claims 12 to 13, wherein the nucleic acid i) has a sequence showing at least 55% similarity with SEQ ID NO:1 or its complementary sequence, or ii) has a sequence that hybridizes to SEQ ID No. 1 or its complementary sequence under stringent conditions, and encodes a p-coumaroyl ester 3'-hydroxylase enzyme.

16. The use of claim 14, wherein the C3'H comprises the amino acid sequence of SEQ ID No. 2.

17. A plant cell, a plant, or part of a plant that is transgenic for a nucleic acid that encodes a p-coumaroyl ester 3'-hydroxylase (C3'H).

18. The plant cell, plant or plant part of claim 17, that comprises an expression cassette comprising a constitutive plant promoter operably linked to a nucleic acid encoding a p-coumaroyl ester 3'-hydroxylase (C3'H).

19. The plant or part of a plant according to claim 17 or 18, that is obtainable by the method of any one of claims 1 to 10.

20. A part of the plant according to any of claims 17 to 19, that is a root, stem, trunk, flower, leaf or seed.

21. An expression cassette comprising a plant-expressible promoter operably linked to a coding region encoding a C3'H, wherein said promoter is not a C3'H promoter.

22. The expression cassette of claim 21, wherein said promoter is a 35S promoter, a ubiquitin promoter or an actin promoter.

Wild-type                    CAMV *35S-CYP98A3*

**FIG.1A**                    **FIG.1B**

**FIG.1**

CAMV *35S-CYP98A3*

Wild-type

## FIG.2

## FIG.3

Wild-type  CAMV *35S-CYP98A3*

Mean of fresh weight of 15-day-old plantlets

FIG.4

Mean weight of collected seeds/ plant

FIG.5

**Area of epidermal cell**

624-1804   810-2484

Wild-type   CAMV 35S-CYP98A3

FIG.6A

FIG.6B

FIG.6C

## FIG.7

## FIG.8

## FIG.9

## FIG.10

**European Patent Office**

**EUROPEAN SEARCH REPORT**

Application Number

EP 05 29 0639

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | FRANKE R ET AL: "The Arabidopsis REF8 gene encodes the 3-hydroxylase of phenylpropanoid metabolism" PLANT JOURNAL, BLACKWELL SCIENTIFIC PUBLICATIONS, OXFORD, GB, vol. 30, no. 1, April 2002 (2002-04), pages 33-45, XP002223726 ISSN: 0960-7412 * page 38, right-hand column; figures 1,6(c) * * page 42, paragraph 4 * ----- | 1-22 | C12N15/82 C12N9/02 A01H5/02 |
| X,D A | WO 02/14497 A (PURDUE RESEARCH FOUNDATION) 21 February 2002 (2002-02-21) see SEQ ID NO: 1 and 4 * page 7, paragraphs 3,5; claims * * page 23, paragraph 2 * * page 27, paragraph 4 - page 29, paragraph 1 * ----- | 17-22 7,8,15, 16 | |
| A | WO 99/24561 A (BOARD OF CONTROL OF MICHIGAN TECHNOLOGICAL UNIVERSITY; CHIANG, VINCENT) 20 May 1999 (1999-05-20) * the whole document * ----- | | **TECHNICAL FIELDS SEARCHED (Int.Cl.7)** C12N A01H |

The present search report has been drawn up for all claims

| Place of search | Date of completion of the search | Examiner |
|---|---|---|
| The Hague | 19 August 2005 | Oderwald, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 29 0639

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2005

| Patent document cited in search report | | Publication date | Patent family member(s) | | Publication date |
|---|---|---|---|---|---|
| WO 0214497 | A | 21-02-2002 | AU | 8495201 A | 25-02-2002 |
| | | | CA | 2419524 A1 | 21-02-2002 |
| | | | WO | 0214497 A2 | 21-02-2002 |
| | | | US | 2002062496 A1 | 23-05-2002 |
| WO 9924561 | A | 20-05-1999 | US | 6455762 B1 | 24-09-2002 |
| | | | AU | 762576 B2 | 26-06-2003 |
| | | | AU | 1402499 A | 31-05-1999 |
| | | | AU | 2003248405 A1 | 06-11-2003 |
| | | | BR | 9814154 A | 13-11-2001 |
| | | | CA | 2309337 A1 | 20-05-1999 |
| | | | EP | 1034283 A2 | 13-09-2000 |
| | | | NZ | 504395 A | 31-01-2003 |
| | | | WO | 9924561 A2 | 20-05-1999 |
| | | | US | 2003172395 A1 | 11-09-2003 |
| | | | US | 6831208 B1 | 14-12-2004 |
| | | | ZA | 9810305 A | 20-10-2000 |

EPO FORM P0459

For more details about this annex : see Official Journal of the European Patent Office, No. 12/82

## REFERENCES CITED IN THE DESCRIPTION

**Patent documents cited in the description**

- WO 0214497 A
- EP 0342926 A
- US 5641876 A
- WO 9748819 A
- WO 9606932 A
- EP 242246 A
- US 5641664 A
- US 5679558 A
- WO 0133942 A

**Non-patent literature cited in the description**

- Current methods in sequencing and synthesis Methods and Applications. Ala. R. Liss, Inc, 1988, 127-149
- **MC ELROY et al.** *Mol. Gen. Genet.,* 1991, vol. 231, 150-160
- **AN et al.** *Plant Physiol.,* 1986, vol. 81, 86-91
- **ADLER E. ; BJORKQUIST, K.J. ; HAGGROTH, S.** *Acta. Chem. Scand.,* 1948, vol. 2, 93-94
- **BECHTOLD et al.** *C.R. Acad. Sci. Paris, Life sciences,* 1993, vol. 316, 1194-1199
- **BENFEY et al.** *EMBO J,* 1989, vol. 5, 2195-2202
- **CHOMCZYNSKI et al.** *Analytical Biochem.,* 1987, vol. 162, 1256-159
- **CHRISTOU et al.** *Biotechnology,* 1991, vol. 9, 957
- **CLOUGH et al.** *Plant J.,* 1998, vol. 16 (6), 735-743
- **CHUPEAU et al.** *Biotechnology,* 1989, vol. 7 (5), 503-508
- **FINER J.** *Plant Cell Report,* 1992, vol. 11, 323-328
- **FRANKE et al.** *the Plant Journal,* 2002, vol. 30 (1), 47-59
- **FRANKE et al.** *The Plant Journal,* 2002, vol. 30 (1), 33-45
- **FROMM M. et al.** *Biotechnology,* 1990, vol. 8, 833-839
- **GAUBIER et al.** *Mol. Gen.,* 1993, vol. 238, 409-418
- **GOUJON T. ; SIBOUT R. ; POLLET B. ; MABA B. ; NUSSAUME L. ; BECHTOLD N. ; LU F. ; RALPH J. ; MILA I. ; BARRIÈRE Y.** *Plant Mol. Biol,* 2003, vol. 51, 973-989
- **GUERCHE et al.** *Mol. Gen. Genet.,* 1987, vol. 206, 382
- **HERRERA-ESTRELLA et al.** *EMBO J.,* 1983, vol. 2, 987-995
- **HIEI et al.** *Plant J,* 1994, vol. 6, 271-282
- **HIEI et al.** *Plant Mol Biol.,* 1997, vol. 35, 205-218
- **ISHIDA et al.** *Nature Biotechnology,* 1996, vol. 14, 745-750
- **KARIMI et al.** *Trends Plant Sci.,* 2002, vol. 7 (5), 193-5
- **KAY et al.** *Science,* 1987, vol. 236, 1299-1302
- **MCCORMAC et al.** *Mol Biotechnol.,* 1998, vol. 9, 155-159
- **MCELROY et al.** *Mol. Gen. Genet.,* 1991, vol. 231, 150-160
- **NEUHAUS et al.** *Theoretical and Applied Genetics,* 1987, vol. 75 (1), 30-36
- **NI et al.** *Plant J.,* 1995, vol. 7, 661-676
- **SAMAC et al.** *Plant Physiol.,* 1990, vol. 93, 907-914
- **SCHOCH et al.** *The Journal of Biological Chemistry,* 2001, vol. 276 (39), 36566-36574
- **SCHOCHER et al.** *Biotechnology,* 1986, vol. 4, 1093-1096
- **WATSON et al.** ADN recombinant [Recombinant DNA. De Boeck University, 1994, 273-292
- **ZHANG et al.** *The Plant Cell,* 1991, vol. 3, 1155-1165

SEQUENCE LISTING

<110> Genoplante

<120> Method for producing plants with increased biomass

<130> BET 05P0002

<140>
<141>

<160> 40

<170> PatentIn Ver. 2.1

<210> 1
<211> 1527
<212> DNA
<213> Arabidopsis thaliana

<220>
<221> CDS
<222> (1)..(1527)

<400> 1
```
atg tcg tgg ttt cta ata gcg gtg gcg aca atc gcc gcc gtc gta tcc      48
Met Ser Trp Phe Leu Ile Ala Val Ala Thr Ile Ala Ala Val Val Ser
  1               5                  10                  15

tac aag cta atc caa cgg cta aga tac aag ttc cca cca ggc cca agc      96
Tyr Lys Leu Ile Gln Arg Leu Arg Tyr Lys Phe Pro Pro Gly Pro Ser
             20                  25                  30

ccc aag ccg atc gtc ggt aac ctc tac gac ata aaa ccg gtc cgg ttc     144
Pro Lys Pro Ile Val Gly Asn Leu Tyr Asp Ile Lys Pro Val Arg Phe
         35                  40                  45

aga tgt tac tac gag tgg gct caa tct tat gga cca atc ata tcg gtc     192
Arg Cys Tyr Tyr Glu Trp Ala Gln Ser Tyr Gly Pro Ile Ile Ser Val
     50                  55                  60

tgg atc ggt tca att cta aac gtg gtc gta tct agc gcc gag cta gca     240
Trp Ile Gly Ser Ile Leu Asn Val Val Val Ser Ser Ala Glu Leu Ala
 65                  70                  75                  80

aaa gaa gtt ctg aaa gaa cac gac cag aaa ctc gcc gac cgg cac cgg     288
Lys Glu Val Leu Lys Glu His Asp Gln Lys Leu Ala Asp Arg His Arg
                 85                  90                  95

aac aga tcg acg gaa gca ttt agc cgc aac ggt cag gat ctt ata tgg     336
Asn Arg Ser Thr Glu Ala Phe Ser Arg Asn Gly Gln Asp Leu Ile Trp
                100                 105                 110

gcc gat tat ggg cct cat tac gtg aag gtg aga aaa gtt tgc acg ctt     384
Ala Asp Tyr Gly Pro His Tyr Val Lys Val Arg Lys Val Cys Thr Leu
            115                 120                 125

gag ctc ttc aca ccg aaa cga ctc gag tct ctc aga cct atc cgt gaa     432
Glu Leu Phe Thr Pro Lys Arg Leu Glu Ser Leu Arg Pro Ile Arg Glu
            130                 135                 140
```

```
gat gaa gtc acc gcc atg gtt gaa tcc gtc ttc aga gac tgt aac ctt    480
Asp Glu Val Thr Ala Met Val Glu Ser Val Phe Arg Asp Cys Asn Leu
145             150             155             160

cct gaa aac aga gca aaa ggt tta caa ctg agg aag tac tta gga gcg    528
Pro Glu Asn Arg Ala Lys Gly Leu Gln Leu Arg Lys Tyr Leu Gly Ala
            165             170             175

gtt gcg ttc aac aac ata acg cgg cta gcc ttt ggg aag cgt ttt atg    576
Val Ala Phe Asn Asn Ile Thr Arg Leu Ala Phe Gly Lys Arg Phe Met
            180             185             190

aac gct gaa ggt gtt gtg gac gag caa ggg ctt gag ttc aag gcc ata    624
Asn Ala Glu Gly Val Val Asp Glu Gln Gly Leu Glu Phe Lys Ala Ile
            195             200             205

gta tcc aac ggt ctg aag cta ggt gct tca ctg tca ata gct gaa cac    672
Val Ser Asn Gly Leu Lys Leu Gly Ala Ser Leu Ser Ile Ala Glu His
            210             215             220

atc ccg tgg ctc agg tgg atg ttt ccg gct gat gag aag gcg ttt gct    720
Ile Pro Trp Leu Arg Trp Met Phe Pro Ala Asp Glu Lys Ala Phe Ala
225             230             235             240

gag cac ggg gct cgt cgt gac cgc ctc act cga gct atc atg gag gag    768
Glu His Gly Ala Arg Arg Asp Arg Leu Thr Arg Ala Ile Met Glu Glu
                245             250             255

cat act ttg gcc cgt caa aag tct agt gga gcg aaa cag cat ttc gtt    816
His Thr Leu Ala Arg Gln Lys Ser Ser Gly Ala Lys Gln His Phe Val
                260             265             270

gat gcg ttg cta acg ttg aag gat cag tat gat ctt agt gag gat act    864
Asp Ala Leu Leu Thr Leu Lys Asp Gln Tyr Asp Leu Ser Glu Asp Thr
            275             280             285

atc att ggt ctt cta tgg gat atg atc acg gca ggg atg gac acg aca    912
Ile Ile Gly Leu Leu Trp Asp Met Ile Thr Ala Gly Met Asp Thr Thr
            290             295             300

gcg ata aca gcg gaa tgg gcg atg gcg gaa atg atc aag aat cca aga    960
Ala Ile Thr Ala Glu Trp Ala Met Ala Glu Met Ile Lys Asn Pro Arg
305             310             315             320

gtg caa caa aaa gtg caa gaa gag ttc gac aga gtg gtt gga ctt gac    1008
Val Gln Gln Lys Val Gln Glu Glu Phe Asp Arg Val Val Gly Leu Asp
            325             330             335

cgg atc tta acc gag gca gat ttc tcc cgc tta cct tac ttg caa tgc    1056
Arg Ile Leu Thr Glu Ala Asp Phe Ser Arg Leu Pro Tyr Leu Gln Cys
            340             345             350

gtg gtg aaa gag tca ttc agg ctg cat cct cca acg cct cta atg cta    1104
Val Val Lys Glu Ser Phe Arg Leu His Pro Pro Thr Pro Leu Met Leu
            355             360             365

cct cac cga agc aac gca gat gtc aag atc gga ggc tat gat att ccc    1152
Pro His Arg Ser Asn Ala Asp Val Lys Ile Gly Gly Tyr Asp Ile Pro
370             375             380

aaa gga tca aac gtt cat gtg aat gtg tgg gct gtg gct aga gac ccg    1200
Lys Gly Ser Asn Val His Val Asn Val Trp Ala Val Ala Arg Asp Pro
```

11

```
Lys Gly Ser Asn Val His Val Asn Val Trp Ala Val Ala Arg Asp Pro
385                 390                 395                 400

gct gta tgg aaa aat cca ttt gag ttt aga cca gag aga ttc ttg gaa    1248
Ala Val Trp Lys Asn Pro Phe Glu Phe Arg Pro Glu Arg Phe Leu Glu
                405                 410                 415

gaa gat gtt gac atg aag ggt cat gat ttt agg ctg ctt ccg ttt gga    1296
Glu Asp Val Asp Met Lys Gly His Asp Phe Arg Leu Leu Pro Phe Gly
                420                 425                 430

gct gga aga cgg gtt tgt ccc ggt gca caa ctt ggt atc aat ttg gta    1344
Ala Gly Arg Arg Val Cys Pro Gly Ala Gln Leu Gly Ile Asn Leu Val
            435                 440                 445

act tcg atg atg agt cat ttg ctt cac cat ttt gtt tgg aca cct cct    1392
Thr Ser Met Met Ser His Leu Leu His His Phe Val Trp Thr Pro Pro
            450                 455                 460

caa ggg act aaa ccg gag gag att gac atg tct gaa aac cct gga ctc    1440
Gln Gly Thr Lys Pro Glu Glu Ile Asp Met Ser Glu Asn Pro Gly Leu
465                 470                 475                 480

gtt act tac atg cgt acc cct gtg caa gcg gtt gca acg cct cgg ttg    1488
Val Thr Tyr Met Arg Thr Pro Val Gln Ala Val Ala Thr Pro Arg Leu
                485                 490                 495

cct tcg gat ctg tac aaa cgc gtg cct tac gat atg taa                1527
Pro Ser Asp Leu Tyr Lys Arg Val Pro Tyr Asp Met
            500                 505


<210> 2
<211> 508
<212> PRT
<213> Arabidopsis thaliana

<400> 2
Met Ser Trp Phe Leu Ile Ala Val Ala Thr Ile Ala Ala Val Val Ser
  1             5                 10                  15

Tyr Lys Leu Ile Gln Arg Leu Arg Tyr Lys Phe Pro Pro Gly Pro Ser
                20                  25                  30

Pro Lys Pro Ile Val Gly Asn Leu Tyr Asp Ile Lys Pro Val Arg Phe
            35                  40                  45

Arg Cys Tyr Tyr Glu Trp Ala Gln Ser Tyr Gly Pro Ile Ile Ser Val
        50                  55                  60

Trp Ile Gly Ser Ile Leu Asn Val Val Val Ser Ser Ala Glu Leu Ala
 65                  70                  75                  80

Lys Glu Val Leu Lys Glu His Asp Gln Lys Leu Ala Asp Arg His Arg
                85                  90                  95

Asn Arg Ser Thr Glu Ala Phe Ser Arg Asn Gly Gln Asp Leu Ile Trp
            100                 105                 110

Ala Asp Tyr Gly Pro His Tyr Val Lys Val Arg Lys Val Cys Thr Leu
            115                 120                 125
```

```
Glu Leu Phe Thr Pro Lys Arg Leu Glu Ser Leu Arg Pro Ile Arg Glu
    130             135             140

Asp Glu Val Thr Ala Met Val Glu Ser Val Phe Arg Asp Cys Asn Leu
145             150             155                 160

Pro Glu Asn Arg Ala Lys Gly Leu Gln Leu Arg Lys Tyr Leu Gly Ala
            165             170             175

Val Ala Phe Asn Asn Ile Thr Arg Leu Ala Phe Gly Lys Arg Phe Met
            180             185             190

Asn Ala Glu Gly Val Val Asp Glu Gln Gly Leu Glu Phe Lys Ala Ile
        195             200             205

Val Ser Asn Gly Leu Lys Leu Gly Ala Ser Leu Ser Ile Ala Glu His
    210             215             220

Ile Pro Trp Leu Arg Trp Met Phe Pro Ala Asp Glu Lys Ala Phe Ala
225             230             235                 240

Glu His Gly Ala Arg Arg Asp Arg Leu Thr Arg Ala Ile Met Glu Glu
            245             250             255

His Thr Leu Ala Arg Gln Lys Ser Ser Gly Ala Lys Gln His Phe Val
        260             265             270

Asp Ala Leu Leu Thr Leu Lys Asp Gln Tyr Asp Leu Ser Glu Asp Thr
        275             280             285

Ile Ile Gly Leu Leu Trp Asp Met Ile Thr Ala Gly Met Asp Thr Thr
    290             295             300

Ala Ile Thr Ala Glu Trp Ala Met Ala Glu Met Ile Lys Asn Pro Arg
305             310             315                 320

Val Gln Gln Lys Val Gln Glu Glu Phe Asp Arg Val Val Gly Leu Asp
            325             330             335

Arg Ile Leu Thr Glu Ala Asp Phe Ser Arg Leu Pro Tyr Leu Gln Cys
            340             345             350

Val Val Lys Glu Ser Phe Arg Leu His Pro Pro Thr Pro Leu Met Leu
        355             360             365

Pro His Arg Ser Asn Ala Asp Val Lys Ile Gly Gly Tyr Asp Ile Pro
    370             375             380

Lys Gly Ser Asn Val His Val Asn Val Trp Ala Val Ala Arg Asp Pro
385             390             395                 400

Ala Val Trp Lys Asn Pro Phe Glu Phe Arg Pro Glu Arg Phe Leu Glu
            405             410             415

Glu Asp Val Asp Met Lys Gly His Asp Phe Arg Leu Leu Pro Phe Gly
        420             425             430

Ala Gly Arg Arg Val Cys Pro Gly Ala Gln Leu Gly Ile Asn Leu Val
        435             440             445
```

13

```
Thr Ser Met Met Ser His Leu Leu His His Phe Val Trp Thr Pro Pro
    450             455             460

Gln Gly Thr Lys Pro Glu Glu Ile Asp Met Ser Glu Asn Pro Gly Leu
465             470             475             480

Val Thr Tyr Met Arg Thr Pro Val Gln Ala Val Ala Thr Pro Arg Leu
            485             490             495

Pro Ser Asp Leu Tyr Lys Arg Val Pro Tyr Asp Met
            500             505
```

```
<210> 3
<211> 53
<212> DNA
<213> Artificial Sequence

<220>
<223> Description of Artificial Sequence:
      oligonucleotide

<400> 3
ggggacaagt ttgtacaaaa aagcaggcta tgtcgtggtt tctaatagcg gtg          53
```

```
<210> 4
<211> 53
<212> DNA
<213> Artificial Sequence

<220>
<223> Description of Artificial Sequence:
      oligonucleotide

<400> 4
ggggaccact ttgtacaaga aagctgggtt tacatatcgt aaggcacgcg ttt          53
```

```
<210> 5
<211> 26
<212> DNA
<213> Artificial Sequence

<220>
<223> Description of Artificial Sequence:
      oligonucleotide

<400> 5
tctgcgaaaa ggatttactc aaagtt                                        26
```

```
<210> 6
<211> 23
<212> DNA
<213> Artificial Sequence

<220>
<223> Description of Artificial Sequence:
      oligonucleotide
```

```
<400> 6
ctacaaggat catccgcgta tgt                                          23


<210> 7
<211> 28
<212> DNA
<213> Artificial Sequence

<220>
<223> Description of Artificial Sequence:
      oligonucleotide

<400> 7
cctccaggac agtctaaagt ggatacta                                     28


<210> 8
<211> 25
<212> DNA
<213> Artificial Sequence

<220>
<223> Description of Artificial Sequence:
      oligonucleotide

<400> 8
cgattatgga gtggttaagg atgtg                                        25


<210> 9
<211> 23
<212> DNA
<213> Artificial Sequence

<220>
<223> Description of Artificial Sequence:
      oligonucleotide

<400> 9
tctttatggg acctggtgga att                                          23


<210> 10
<211> 28
<212> DNA
<213> Artificial Sequence

<220>
<223> Description of Artificial Sequence:
      oligonucleotide

<400> 10
gataagctgc catcattagt aggacttg                                     28


<210> 11
<211> 25
<212> DNA
<213> Artificial Sequence
```

```
<220>
<223> Description of Artificial Sequence:
      oligonucleotide

<400> 11
aagtctagtg gagcgaaaca gcatt                                    25


<210> 12
<211> 28
<212> DNA
<213> Artificial Sequence

<220>
<223> Description of Artificial Sequence:
      oligonucleotide

<400> 12
tcctcactaa gatcatactg atccttca                                 28


<210> 13
<211> 22
<212> DNA
<213> Artificial Sequence

<220>
<223> Description of Artificial Sequence:
      oligonucleotide

<400> 13
ttcaatgtcc ctgccatgta tg                                       22


<210> 14
<211> 20
<212> DNA
<213> Artificial Sequence

<220>
<223> Description of Artificial Sequence:
      oligonucleotide

<400> 14
aataccggtt gtacgaccac                                          20


<210> 15
<211> 1539
<212> DNA
<213> Sorghum bicolor

<220>
<221> CDS
<222> (1)..(1539)

<400> 15
atg gac gcc tcc ctc ctc ctc tcc gtc gcg ctc gcg gtg gtg ctg atc    48
Met Asp Ala Ser Leu Leu Leu Ser Val Ala Leu Ala Val Val Leu Ile
  1               5                  10                  15
```

```
ccg ctc tcg ctc gcg ctc ctc aac cgg ctc cgc ctc ggc cgc ctg ccg      96
Pro Leu Ser Leu Ala Leu Leu Asn Arg Leu Arg Leu Gly Arg Leu Pro
            20              25              30

ccg ggc ccg cgg ccg tgg ccc gtc ctg ggc aac ctg cgc cag atc aag      144
Pro Gly Pro Arg Pro Trp Pro Val Leu Gly Asn Leu Arg Gln Ile Lys
        35              40              45

ccg atc cgg tgc cgc tgc ttc cag gag tgg gcg gag cgg tac ggg ccc      192
Pro Ile Arg Cys Arg Cys Phe Gln Glu Trp Ala Glu Arg Tyr Gly Pro
        50              55              60

gtc atc tcc gtc tgg ttc ggg tcg ggg ctc acc gtg gtc gtc tcc acc      240
Val Ile Ser Val Trp Phe Gly Ser Gly Leu Thr Val Val Val Ser Thr
65              70              75              80

tcc gag ctc gcc aag gag gtg ctc aag gag aat gac cag cag ctc gcg      288
Ser Glu Leu Ala Lys Glu Val Leu Lys Glu Asn Asp Gln Gln Leu Ala
            85              90              95

gac cgg ccg cgg aac cgc tcc acc cag cgg ttc agc cgc aac ggg cag      336
Asp Arg Pro Arg Asn Arg Ser Thr Gln Arg Phe Ser Arg Asn Gly Gln
            100             105             110

gac ctg atc tgg gcc gac tac ggc ccg cac tac atc aag gtg cgc aag      384
Asp Leu Ile Trp Ala Asp Tyr Gly Pro His Tyr Ile Lys Val Arg Lys
        115             120             125

ctc tgc aac ctc gag ctc ttc acg ccc aag agg ctc gag gcg ctg cgc      432
Leu Cys Asn Leu Glu Leu Phe Thr Pro Lys Arg Leu Glu Ala Leu Arg
    130             135             140

ccc atc cgc gag gac gag gtc acc gcc atg gtc gag tcc gtt tac cgc      480
Pro Ile Arg Glu Asp Glu Val Thr Ala Met Val Glu Ser Val Tyr Arg
145             150             155             160

gcc gcc act gcc ccc ggt aat gaa gga aag cca atg gta gtg agg aac      528
Ala Ala Thr Ala Pro Gly Asn Glu Gly Lys Pro Met Val Val Arg Asn
            165             170             175

cac ctt tct atg gtg gcc ttc aac aac ata aca aga ctg gca ttt ggg      576
His Leu Ser Met Val Ala Phe Asn Asn Ile Thr Arg Leu Ala Phe Gly
            180             185             190

aag cgg ttc atg aat gca aac ggt gac att gac gaa caa ggg cgt gag      624
Lys Arg Phe Met Asn Ala Asn Gly Asp Ile Asp Glu Gln Gly Arg Glu
            195             200             205

ttt aag act att gtg aac aac ggg atc aag att ggt gca tct ctc tct      672
Phe Lys Thr Ile Val Asn Asn Gly Ile Lys Ile Gly Ala Ser Leu Ser
            210             215             220

gtt gct gag ttt att tgg tat ttg aga tgg ttg tgt cca ctt aat gag      720
Val Ala Glu Phe Ile Trp Tyr Leu Arg Trp Leu Cys Pro Leu Asn Glu
225             230             235             240

gag ctt tac aaa act cac aat gag aga agg gac cgc ttg aca atg aag      768
Glu Leu Tyr Lys Thr His Asn Glu Arg Arg Asp Arg Leu Thr Met Lys
            245             250             255
```

```
atc att gag gag cac gca aag tct ctc aag gag agt ggt gcc aag cag    816
Ile Ile Glu Glu His Ala Lys Ser Leu Lys Glu Ser Gly Ala Lys Gln
            260                 265                 270

cac ttt gtg gat gca ctc ttc acc ctc aaa caa cag tac gac ctt agt    864
His Phe Val Asp Ala Leu Phe Thr Leu Lys Gln Gln Tyr Asp Leu Ser
            275                 280                 285

gaa gac aca gtt att gga ctt cta tgg gac atg atc act gct gga atg    912
Glu Asp Thr Val Ile Gly Leu Leu Trp Asp Met Ile Thr Ala Gly Met
            290                 295                 300

gac aca aca gtc atc tcc gtt gag tgg gca atg gca gag ctg gtc agg    960
Asp Thr Thr Val Ile Ser Val Glu Trp Ala Met Ala Glu Leu Val Arg
305                 310                 315                 320

aac ccc agg gtg cag aag aag ctg caa gag gag ctc gac cgt gtg gtt   1008
Asn Pro Arg Val Gln Lys Lys Leu Gln Glu Glu Leu Asp Arg Val Val
                325                 330                 335

ggc cgt gac cgc gtc atg tta gag acc gac ttc cag aac ctc ccg tac   1056
Gly Arg Asp Arg Val Met Leu Glu Thr Asp Phe Gln Asn Leu Pro Tyr
            340                 345                 350

cta cag gcc gtc gtc aag gag tcg ctc cgg ctg cac ccg ccg acg cca   1104
Leu Gln Ala Val Val Lys Glu Ser Leu Arg Leu His Pro Pro Thr Pro
            355                 360                 365

ctc atg ctc ccc cac aag gcc agc acg aat gtc aag atc ggt ggc tac   1152
Leu Met Leu Pro His Lys Ala Ser Thr Asn Val Lys Ile Gly Gly Tyr
            370                 375                 380

gac atc ccc aag ggc gcc aac gtg atg gtg aac gtg tgg gca gtg gca   1200
Asp Ile Pro Lys Gly Ala Asn Val Met Val Asn Val Trp Ala Val Ala
385                 390                 395                 400

cgc gat ccc aag gtc tgg agc aac ccg ctg gag tac agg ccg gag cgc   1248
Arg Asp Pro Lys Val Trp Ser Asn Pro Leu Glu Tyr Arg Pro Glu Arg
                405                 410                 415

ttc ctg gag gag aac atc gac atc aag ggc agc gac ttc agg gtg ttg   1296
Phe Leu Glu Glu Asn Ile Asp Ile Lys Gly Ser Asp Phe Arg Val Leu
            420                 425                 430

ccg ttt ggt gcc ggc cgg cgt gtc tgc cct ggt gcg cag ctc ggc atc   1344
Pro Phe Gly Ala Gly Arg Arg Val Cys Pro Gly Ala Gln Leu Gly Ile
            435                 440                 445

aac ctc gtg gcc tcc atg atc ggg cac ctt ctg cac cac ttc gag tgg   1392
Asn Leu Val Ala Ser Met Ile Gly His Leu Leu His His Phe Glu Trp
            450                 455                 460

tcc ctg ccg gaa ggc acc aga ccg gag gac gtc aac atg atg gag tcc   1440
Ser Leu Pro Glu Gly Thr Arg Pro Glu Asp Val Asn Met Met Glu Ser
465                 470                 475                 480

ccc ggg ctc gtc acg ttc atg ggc aca cca cta caa gct gtc gcc aag   1488
Pro Gly Leu Val Thr Phe Met Gly Thr Pro Leu Gln Ala Val Ala Lys
            485                 490                 495

ccg cgc ctg gag aag gag gag ctg tac aat agg gtc ccc gtt gag atg   1536
```

```
Pro Arg Leu Glu Lys Glu Glu Leu Tyr Asn Arg Val Pro Val Glu Met
        500                 505             510

tga                                                         1539
```

```
<210> 16
<211> 512
<212> PRT
<213> Sorghum bicolor

<400> 16
Met Asp Ala Ser Leu Leu Leu Ser Val Ala Leu Ala Val Val Leu Ile
  1           5               10                  15

Pro Leu Ser Leu Ala Leu Leu Asn Arg Leu Arg Leu Gly Arg Leu Pro
            20                  25                  30

Pro Gly Pro Arg Pro Trp Pro Val Leu Gly Asn Leu Arg Gln Ile Lys
        35                  40                  45

Pro Ile Arg Cys Arg Cys Phe Gln Glu Trp Ala Glu Arg Tyr Gly Pro
    50                  55                  60

Val Ile Ser Val Trp Phe Gly Ser Gly Leu Thr Val Val Val Ser Thr
 65                 70                  75                  80

Ser Glu Leu Ala Lys Glu Val Leu Lys Glu Asn Asp Gln Gln Leu Ala
                85                  90                  95

Asp Arg Pro Arg Asn Arg Ser Thr Gln Arg Phe Ser Arg Asn Gly Gln
            100                 105                 110

Asp Leu Ile Trp Ala Asp Tyr Gly Pro His Tyr Ile Lys Val Arg Lys
            115                 120                 125

Leu Cys Asn Leu Glu Leu Phe Thr Pro Lys Arg Leu Glu Ala Leu Arg
        130                 135                 140

Pro Ile Arg Glu Asp Glu Val Thr Ala Met Val Glu Ser Val Tyr Arg
145                 150                 155                 160

Ala Ala Thr Ala Pro Gly Asn Glu Gly Lys Pro Met Val Val Arg Asn
                165                 170                 175

His Leu Ser Met Val Ala Phe Asn Asn Ile Thr Arg Leu Ala Phe Gly
            180                 185                 190

Lys Arg Phe Met Asn Ala Asn Gly Asp Ile Asp Glu Gln Gly Arg Glu
            195                 200                 205

Phe Lys Thr Ile Val Asn Asn Gly Ile Lys Ile Gly Ala Ser Leu Ser
    210                 215                 220

Val Ala Glu Phe Ile Trp Tyr Leu Arg Trp Leu Cys Pro Leu Asn Glu
225                 230                 235                 240

Glu Leu Tyr Lys Thr His Asn Glu Arg Arg Asp Arg Leu Thr Met Lys
                245                 250                 255
```

```
                Ile Ile Glu Glu His Ala Lys Ser Leu Lys Glu Ser Gly Ala Lys Gln
                        260                 265                 270

                His Phe Val Asp Ala Leu Phe Thr Leu Lys Gln Gln Tyr Asp Leu Ser
                        275                 280                 285

                Glu Asp Thr Val Ile Gly Leu Leu Trp Asp Met Ile Thr Ala Gly Met
                    290                 295                 300

                Asp Thr Thr Val Ile Ser Val Glu Trp Ala Met Ala Glu Leu Val Arg
                305                 310                 315                 320

                Asn Pro Arg Val Gln Lys Lys Leu Gln Glu Glu Leu Asp Arg Val Val
                            325                 330                 335

                Gly Arg Asp Arg Val Met Leu Glu Thr Asp Phe Gln Asn Leu Pro Tyr
                        340                 345                 350

                Leu Gln Ala Val Val Lys Glu Ser Leu Arg Leu His Pro Pro Thr Pro
                        355                 360                 365

                Leu Met Leu Pro His Lys Ala Ser Thr Asn Val Lys Ile Gly Gly Tyr
                    370                 375                 380

                Asp Ile Pro Lys Gly Ala Asn Val Met Val Asn Val Trp Ala Val Ala
                385                 390                 395                 400

                Arg Asp Pro Lys Val Trp Ser Asn Pro Leu Glu Tyr Arg Pro Glu Arg
                            405                 410                 415

                Phe Leu Glu Glu Asn Ile Asp Ile Lys Gly Ser Asp Phe Arg Val Leu
                            420                 425                 430

                Pro Phe Gly Ala Gly Arg Arg Val Cys Pro Gly Ala Gln Leu Gly Ile
                        435                 440                 445

                Asn Leu Val Ala Ser Met Ile Gly His Leu Leu His His Phe Glu Trp
                    450                 455                 460

                Ser Leu Pro Glu Gly Thr Arg Pro Glu Asp Val Asn Met Met Glu Ser
                465                 470                 475                 480

                Pro Gly Leu Val Thr Phe Met Gly Thr Pro Leu Gln Ala Val Ala Lys
                            485                 490                 495

                Pro Arg Leu Glu Lys Glu Glu Leu Tyr Asn Arg Val Pro Val Glu Met
                        500                 505                 510
```

<210> 17
<211> 1530
<212> DNA
<213> Glycine max

<220>
<221> CDS
<222> (1)..(1530)

<400> 17
atg gcg ctg ctt ctg ata att ccc atc tca ctg gtc acc ctc tgg ctc     48

```
Met Ala Leu Leu Leu Ile Ile Pro Ile Ser Leu Val Thr Leu Trp Leu
 1               5                  10                 15

ggt tac acc cta tac cag cga tta aga ttc aag ctc cct ccg ggt cca    96
Gly Tyr Thr Leu Tyr Gln Arg Leu Arg Phe Lys Leu Pro Pro Gly Pro
             20                  25                 30

cgg ccc tgg ccg gta gtc ggt aac ctc tac gac ata aaa ccc gtc cgc   144
Arg Pro Trp Pro Val Val Gly Asn Leu Tyr Asp Ile Lys Pro Val Arg
         35                  40                 45

ttc cgg tgc ttc gcg gag tgg gcg cag tct tac ggc ccc ata ata tcg   192
Phe Arg Cys Phe Ala Glu Trp Ala Gln Ser Tyr Gly Pro Ile Ile Ser
     50                  55                 60

gtt tgg ttc ggt tcg acc cta aac gtc atc gtt tcg aac tcg gag ctg   240
Val Trp Phe Gly Ser Thr Leu Asn Val Ile Val Ser Asn Ser Glu Leu
 65                  70                 75                 80

gcg aag gag gtg ctg aag gag cac gat cag ctg ctg gcg gac cgc cac   288
Ala Lys Glu Val Leu Lys Glu His Asp Gln Leu Leu Ala Asp Arg His
                 85                  90                 95

cgg agc cgg tcg gcg gcg aag ttc agc cgc gac ggg aag gat cta att   336
Arg Ser Arg Ser Ala Ala Lys Phe Ser Arg Asp Gly Lys Asp Leu Ile
            100                 105                110

tgg gcc gat tat ggg ccg cac tac gtg aag gtg agg aag gtt tgc acg   384
Trp Ala Asp Tyr Gly Pro His Tyr Val Lys Val Arg Lys Val Cys Thr
        115                 120                 125

ctc gag ctt ttc tcg ccg aag cgc ctc gag gcc ctg agg ccc att agg   432
Leu Glu Leu Phe Ser Pro Lys Arg Leu Glu Ala Leu Arg Pro Ile Arg
        130                 135                 140

gag gac gag gtc acc tcc atg gtt gac tcc gtt tac aat cac tgc acc   480
Glu Asp Glu Val Thr Ser Met Val Asp Ser Val Tyr Asn His Cys Thr
145                 150                 155                160

agc act gaa aat ttg ggg aaa gga ata ttg ttg agg aag cac ttg ggg   528
Ser Thr Glu Asn Leu Gly Lys Gly Ile Leu Leu Arg Lys His Leu Gly
                165                 170                 175

gtt gtg gca ttc aac aac ata acc agg ttg gca ttt ggg aaa aga ttt   576
Val Val Ala Phe Asn Asn Ile Thr Arg Leu Ala Phe Gly Lys Arg Phe
            180                 185                 190

gtg aac tca gaa ggt gtg atg gat gag caa gga gta gaa ttc aag gcc   624
Val Asn Ser Glu Gly Val Met Asp Glu Gln Gly Val Glu Phe Lys Ala
            195                 200                 205

att gtg gaa aat ggg tta aag cta gga gca tct cta gcc atg gca gaa   672
Ile Val Glu Asn Gly Leu Lys Leu Gly Ala Ser Leu Ala Met Ala Glu
        210                 215                 220

cac atc cct tgg ctt cgc tgg atg ttc cca ctg gaa gaa gga gct ttt   720
His Ile Pro Trp Leu Arg Trp Met Phe Pro Leu Glu Glu Gly Ala Phe
225                 230                 235                240

gcc aag cat gga gcc cgc cgc gac cga ctc acc aga gcc atc atg gca   768
Ala Lys His Gly Ala Arg Arg Asp Arg Leu Thr Arg Ala Ile Met Ala
```

21

```
                      245                    250                       255

      gag cac act gaa gca cgc aag aaa tct ggt ggt gcc aag caa cat ttt    816
      Glu His Thr Glu Ala Arg Lys Lys Ser Gly Gly Ala Lys Gln His Phe
                  260                 265                 270

      gtt gat gcc ctc ctc aca ttg caa gac aaa tat gac ctt agt gaa gac    864
      Val Asp Ala Leu Leu Thr Leu Gln Asp Lys Tyr Asp Leu Ser Glu Asp
                  275                 280                 285

      acc atc att ggt ctc ctt tgg gat atg atc aca gca ggg atg gac aca    912
      Thr Ile Ile Gly Leu Leu Trp Asp Met Ile Thr Ala Gly Met Asp Thr
                  290                 295                 300

      act gca att tca gtt gag tgg gcc atg gct gag ttg ata aga aac cca    960
      Thr Ala Ile Ser Val Glu Trp Ala Met Ala Glu Leu Ile Arg Asn Pro
      305                 310                 315                 320

      agg gtg caa caa aag gtc caa gag gag cta gac agg gta att ggg ctt   1008
      Arg Val Gln Gln Lys Val Gln Glu Glu Leu Asp Arg Val Ile Gly Leu
                      325                 330                 335

      gaa agg gtg atg act gaa gca gac ttc tca aat ctc cct tac cta caa   1056
      Glu Arg Val Met Thr Glu Ala Asp Phe Ser Asn Leu Pro Tyr Leu Gln
                  340                 345                 350

      tgt gtg acc aaa gaa gca atg agg ctt cac cca cca acc cca cta atg   1104
      Cys Val Thr Lys Glu Ala Met Arg Leu His Pro Pro Thr Pro Leu Met
                  355                 360                 365

      ctc cca cac cgt gcc aat gcc aat gtc aaa gtt gga ggc tat gac att   1152
      Leu Pro His Arg Ala Asn Ala Asn Val Lys Val Gly Gly Tyr Asp Ile
                  370                 375                 380

      ccc aaa ggg tcc aat gtg cat gtg aat gtg tgg gcg gtg gcc cgc gac   1200
      Pro Lys Gly Ser Asn Val His Val Asn Val Trp Ala Val Ala Arg Asp
      385                 390                 395                 400

      ccg gcc gtg tgg aag gat cca ttg gag ttc cga ccc gaa agg ttc ctt   1248
      Pro Ala Val Trp Lys Asp Pro Leu Glu Phe Arg Pro Glu Arg Phe Leu
                  405                 410                 415

      gag gag gat gta gac atg aag ggc cat gac ttt agg cta ctt cca ttc   1296
      Glu Glu Asp Val Asp Met Lys Gly His Asp Phe Arg Leu Leu Pro Phe
                  420                 425                 430

      ggg tcg ggt cga cga gta tgc ccg ggt gcc caa ctt ggt atc aac ttg   1344
      Gly Ser Gly Arg Arg Val Cys Pro Gly Ala Gln Leu Gly Ile Asn Leu
                  435                 440                 445

      gca gca tcc atg ttg ggc cac ctc ttg cac cat ttc tgt tgg acc cca   1392
      Ala Ala Ser Met Leu Gly His Leu Leu His His Phe Cys Trp Thr Pro
                  450                 455                 460

      cct gaa gga atg aag cct gag gaa att gac atg gga gag aat cca ggg   1440
      Pro Glu Gly Met Lys Pro Glu Glu Ile Asp Met Gly Glu Asn Pro Gly
      465                 470                 475                 480

      cta gtc aca tac atg agg act cca ata caa gct gtg gtt tct cct agg   1488
      Leu Val Thr Tyr Met Arg Thr Pro Ile Gln Ala Val Val Ser Pro Arg
                  485                 490                 495
```

```
ctc ccc tca cat tta tac aaa cgt gtg cct gct gag atc taa          1530
Leu Pro Ser His Leu Tyr Lys Arg Val Pro Ala Glu Ile
        500                 505             510


<210> 18
<211> 509
<212> PRT
<213> Glycine max

<400> 18
Met Ala Leu Leu Leu Ile Ile Pro Ile Ser Leu Val Thr Leu Trp Leu
  1               5                  10                  15

Gly Tyr Thr Leu Tyr Gln Arg Leu Arg Phe Lys Leu Pro Pro Gly Pro
            20                  25                  30

Arg Pro Trp Pro Val Val Gly Asn Leu Tyr Asp Ile Lys Pro Val Arg
        35                  40                  45

Phe Arg Cys Phe Ala Glu Trp Ala Gln Ser Tyr Gly Pro Ile Ile Ser
    50                  55                  60

Val Trp Phe Gly Ser Thr Leu Asn Val Ile Val Ser Asn Ser Glu Leu
65                  70                  75                  80

Ala Lys Glu Val Leu Lys Glu His Asp Gln Leu Leu Ala Asp Arg His
                85                  90                  95

Arg Ser Arg Ser Ala Ala Lys Phe Ser Arg Asp Gly Lys Asp Leu Ile
            100                 105                 110

Trp Ala Asp Tyr Gly Pro His Tyr Val Lys Val Arg Lys Val Cys Thr
        115                 120                 125

Leu Glu Leu Phe Ser Pro Lys Arg Leu Glu Ala Leu Arg Pro Ile Arg
    130                 135                 140

Glu Asp Glu Val Thr Ser Met Val Asp Ser Val Tyr Asn His Cys Thr
145                 150                 155                 160

Ser Thr Glu Asn Leu Gly Lys Gly Ile Leu Leu Arg Lys His Leu Gly
                165                 170                 175

Val Val Ala Phe Asn Asn Ile Thr Arg Leu Ala Phe Gly Lys Arg Phe
            180                 185                 190

Val Asn Ser Glu Gly Val Met Asp Glu Gln Gly Val Glu Phe Lys Ala
            195                 200                 205

Ile Val Glu Asn Gly Leu Lys Leu Gly Ala Ser Leu Ala Met Ala Glu
    210                 215                 220

His Ile Pro Trp Leu Arg Trp Met Phe Pro Leu Glu Glu Gly Ala Phe
225                 230                 235                 240

Ala Lys His Gly Ala Arg Arg Asp Arg Leu Thr Arg Ala Ile Met Ala
            245                 250                 255

Glu His Thr Glu Ala Arg Lys Lys Ser Gly Gly Ala Lys Gln His Phe
```

```
                    260                      265                      270

        Val Asp Ala Leu Leu Thr Leu Gln Asp Lys Tyr Asp Leu Ser Glu Asp
                275                  280                  285

        Thr Ile Ile Gly Leu Leu Trp Asp Met Ile Thr Ala Gly Met Asp Thr
                290                  295                  300

        Thr Ala Ile Ser Val Glu Trp Ala Met Ala Glu Leu Ile Arg Asn Pro
        305                  310                  315                  320

        Arg Val Gln Gln Lys Val Gln Glu Glu Leu Asp Arg Val Ile Gly Leu
                        325                  330                  335

        Glu Arg Val Met Thr Glu Ala Asp Phe Ser Asn Leu Pro Tyr Leu Gln
                    340                  345                  350

        Cys Val Thr Lys Glu Ala Met Arg Leu His Pro Pro Thr Pro Leu Met
                    355                  360                  365

        Leu Pro His Arg Ala Asn Ala Asn Val Lys Val Gly Gly Tyr Asp Ile
                370                  375                  380

        Pro Lys Gly Ser Asn Val His Val Asn Val Trp Ala Val Ala Arg Asp
        385                  390                  395                  400

        Pro Ala Val Trp Lys Asp Pro Leu Glu Phe Arg Pro Glu Arg Phe Leu
                        405                  410                  415

        Glu Glu Asp Val Asp Met Lys Gly His Asp Phe Arg Leu Leu Pro Phe
                    420                  425                  430

        Gly Ser Gly Arg Arg Val Cys Pro Gly Ala Gln Leu Gly Ile Asn Leu
                    435                  440                  445

        Ala Ala Ser Met Leu Gly His Leu Leu His His Phe Cys Trp Thr Pro
                450                  455                  460

        Pro Glu Gly Met Lys Pro Glu Glu Ile Asp Met Gly Glu Asn Pro Gly
        465                  470                  475                  480

        Leu Val Thr Tyr Met Arg Thr Pro Ile Gln Ala Val Val Ser Pro Arg
                        485                  490                  495

        Leu Pro Ser His Leu Tyr Lys Arg Val Pro Ala Glu Ile
                        500                  505
```

```
<210> 19
<211> 1422
<212> DNA
<213> Oryza sativa

<220>
<221> CDS
<222> (1)..(1422)

<400> 19
atg gtg ggg aac ctg tgg cag atc aag ccg gtg cgg tgc cgc ggc ttc      48
Met Val Gly Asn Leu Trp Gln Ile Lys Pro Val Arg Cys Arg Gly Phe
```

```
          1                    5                        10                         15

        ctg gag tgg gcg gag agg tac ggc ccg atc gtg tcg gtg tgg ttc ggc       96
        Leu Glu Trp Ala Glu Arg Tyr Gly Pro Ile Val Ser Val Trp Phe Gly
                    20                      25                      30

        tcg tcg ctg aac gtg gtg gtg tcg acg tcg gag ctc gcc aag gag gtg       144
        Ser Ser Leu Asn Val Val Val Ser Thr Ser Glu Leu Ala Lys Glu Val
                35                      40                      45

        ctc aag gag aac gac cag ctg ctc gcc gac cgg ccg cgc aac cgc tcc       192
        Leu Lys Glu Asn Asp Gln Leu Leu Ala Asp Arg Pro Arg Asn Arg Ser
            50                      55                      60

        acg cag cgc ttc agc cgc aac ggg atg gac ctg atc tgg gcg gac tac       240
        Thr Gln Arg Phe Ser Arg Asn Gly Met Asp Leu Ile Trp Ala Asp Tyr
        65                      70                      75                      80

        ggc ccg cac tac atc aag gtg cgc aag ctc tgc aac ctc gag ctc ttc       288
        Gly Pro His Tyr Ile Lys Val Arg Lys Leu Cys Asn Leu Glu Leu Phe
                        85                      90                      95

        acc ccc aag cgc ctc gag gcc ctc cgc ccc atc cgc gag gac gag gtc       336
        Thr Pro Lys Arg Leu Glu Ala Leu Arg Pro Ile Arg Glu Asp Glu Val
                    100                     105                     110

        acc gcc atg gtc gag tcc gtc cac cgc gcc gtc acc cag cca ggt agt       384
        Thr Ala Met Val Glu Ser Val His Arg Ala Val Thr Gln Pro Gly Ser
                115                     120                     125

        gaa cat aaa cca atc gta gtg agg aat cac ctt gct atg gtg gcc ttc       432
        Glu His Lys Pro Ile Val Val Arg Asn His Leu Ala Met Val Ala Phe
                130                     135                     140

        aac aat ata aca agg cta gct ttc ggc aag agg ttc atg aat gca aat       480
        Asn Asn Ile Thr Arg Leu Ala Phe Gly Lys Arg Phe Met Asn Ala Asn
        145                     150                     155                     160

        ggt gac att gat gaa caa ggg cgt gag ttt aag act att gtc aac aat       528
        Gly Asp Ile Asp Glu Gln Gly Arg Glu Phe Lys Thr Ile Val Asn Asn
                        165                     170                     175

        gga atc aag atc ggt gca tct ctt tct gtt gct gag tac att tgg tat       576
        Gly Ile Lys Ile Gly Ala Ser Leu Ser Val Ala Glu Tyr Ile Trp Tyr
                    180                     185                     190

        ttg agg tgg ttg tgt cca ctt aac gag gag ctt tac aag acc cac aac       624
        Leu Arg Trp Leu Cys Pro Leu Asn Glu Glu Leu Tyr Lys Thr His Asn
                195                     200                     205

        gag aga agg gat cgg ctg acc aag aag atc ata gat gag cat gcg aag       672
        Glu Arg Arg Asp Arg Leu Thr Lys Lys Ile Ile Asp Glu His Ala Lys
                210                     215                     220

        gcc ctc aag gag agt ggt gcc aag cag cac ttt gtg gat gct ctg ttc       720
        Ala Leu Lys Glu Ser Gly Ala Lys Gln His Phe Val Asp Ala Leu Phe
        225                     230                     235                     240

        act ctc aga gaa cag tat gac ctt agt gac gac aca gtt att ggg ctt       768
        Thr Leu Arg Glu Gln Tyr Asp Leu Ser Asp Asp Thr Val Ile Gly Leu
                245                     250                     255
```

```
cta tgg gac atg atc act gct gga atg gac acc aca gtg ata tca gtc      816
Leu Trp Asp Met Ile Thr Ala Gly Met Asp Thr Thr Val Ile Ser Val
        260             265             270

gag tgg gca atg gca gag ctg gtc agg aac ccc agg gtg cag aag aag      864
Glu Trp Ala Met Ala Glu Leu Val Arg Asn Pro Arg Val Gln Lys Lys
        275             280             285

ctg cag gag gag ctg gac cgc gtc gtc ggc cgc gac cgc gtc atg tcg      912
Leu Gln Glu Glu Leu Asp Arg Val Val Gly Arg Asp Arg Val Met Ser
        290             295             300

gag acc gac ttc cag agc ctc cct tac ctg aac gcc gtc gtc aag gag      960
Glu Thr Asp Phe Gln Ser Leu Pro Tyr Leu Asn Ala Val Val Lys Glu
305             310             315             320

tcg ctc cgg ctg cac ccg ccg acg ccg ctg atg ctc ccg cac aag gcc     1008
Ser Leu Arg Leu His Pro Pro Thr Pro Leu Met Leu Pro His Lys Ala
        325             330             335

agc acg aac gtc aag atc ggc ggg tac aac atc ccc aag ggc gcc aac     1056
Ser Thr Asn Val Lys Ile Gly Gly Tyr Asn Ile Pro Lys Gly Ala Asn
        340             345             350

gtg atg gtg aac gtg tgg gcg atc gcc agg gac ccc aag gtg tgg agc     1104
Val Met Val Asn Val Trp Ala Ile Ala Arg Asp Pro Lys Val Trp Ser
        355             360             365

aac ccg ctg gag tac agg ccg gag cgg ttc atc gag gag aac atc gac     1152
Asn Pro Leu Glu Tyr Arg Pro Glu Arg Phe Ile Glu Glu Asn Ile Asp
        370             375             380

atc aag ggc agc gac ttc agg gtg ctc ccc ttc ggc gcc ggc cgc cgc     1200
Ile Lys Gly Ser Asp Phe Arg Val Leu Pro Phe Gly Ala Gly Arg Arg
385             390             395             400

gtg tgc ccc ggc gcc cag ctc ggc atc aac ctc gtc gcc tcc atg atc     1248
Val Cys Pro Gly Ala Gln Leu Gly Ile Asn Leu Val Ala Ser Met Ile
        405             410             415

ggg cac ctc ctc cac cag ttc gag tgg tct ctc ccc gag ggc acc agg     1296
Gly His Leu Leu His Gln Phe Glu Trp Ser Leu Pro Glu Gly Thr Arg
        420             425             430

ccg gag gac gtc aac atg atg gag tcc aac ggc gtc gtc acg ttc atg     1344
Pro Glu Asp Val Asn Met Met Glu Ser Asn Gly Val Val Thr Phe Met
        435             440             445

agc acg tcg ctg cag gtc atc gcc aag ccc agg ctc gac aac ccg gac     1392
Ser Thr Ser Leu Gln Val Ile Ala Lys Pro Arg Leu Asp Asn Pro Asp
        450             455             460

ctg tac aag agg ttc cct gtc gag atg tga                             1422
Leu Tyr Lys Arg Phe Pro Val Glu Met
465             470
```

```
<210> 20
<211> 473
<212> PRT
```

26

<213> Oryza sativa

<400> 20

```
Met Val Gly Asn Leu Trp Gln Ile Lys Pro Val Arg Cys Arg Gly Phe
 1               5                  10                  15

Leu Glu Trp Ala Glu Arg Tyr Gly Pro Ile Val Ser Val Trp Phe Gly
            20                  25                  30

Ser Ser Leu Asn Val Val Val Ser Thr Ser Glu Leu Ala Lys Glu Val
            35                  40                  45

Leu Lys Glu Asn Asp Gln Leu Leu Ala Asp Arg Pro Arg Asn Arg Ser
        50                  55                  60

Thr Gln Arg Phe Ser Arg Asn Gly Met Asp Leu Ile Trp Ala Asp Tyr
65                  70                  75                  80

Gly Pro His Tyr Ile Lys Val Arg Lys Leu Cys Asn Leu Glu Leu Phe
                85                  90                  95

Thr Pro Lys Arg Leu Glu Ala Leu Arg Pro Ile Arg Glu Asp Glu Val
            100                 105                 110

Thr Ala Met Val Glu Ser Val His Arg Ala Val Thr Gln Pro Gly Ser
        115                 120                 125

Glu His Lys Pro Ile Val Val Arg Asn His Leu Ala Met Val Ala Phe
        130                 135                 140

Asn Asn Ile Thr Arg Leu Ala Phe Gly Lys Arg Phe Met Asn Ala Asn
145                 150                 155                 160

Gly Asp Ile Asp Glu Gln Gly Arg Glu Phe Lys Thr Ile Val Asn Asn
            165                 170                 175

Gly Ile Lys Ile Gly Ala Ser Leu Ser Val Ala Glu Tyr Ile Trp Tyr
            180                 185                 190

Leu Arg Trp Leu Cys Pro Leu Asn Glu Glu Leu Tyr Lys Thr His Asn
        195                 200                 205

Glu Arg Arg Asp Arg Leu Thr Lys Lys Ile Ile Asp Glu His Ala Lys
        210                 215                 220

Ala Leu Lys Glu Ser Gly Ala Lys Gln His Phe Val Asp Ala Leu Phe
225                 230                 235                 240

Thr Leu Arg Glu Gln Tyr Asp Leu Ser Asp Asp Thr Val Ile Gly Leu
            245                 250                 255

Leu Trp Asp Met Ile Thr Ala Gly Met Asp Thr Thr Val Ile Ser Val
            260                 265                 270

Glu Trp Ala Met Ala Glu Leu Val Arg Asn Pro Arg Val Gln Lys Lys
        275                 280                 285

Leu Gln Glu Glu Leu Asp Arg Val Val Gly Arg Asp Arg Val Met Ser
        290                 295                 300

Glu Thr Asp Phe Gln Ser Leu Pro Tyr Leu Asn Ala Val Val Lys Glu
```

```
                 305                  310                  315                  320
     Ser Leu Arg Leu His Pro Pro Thr Pro Leu Met Leu Pro His Lys Ala
                     325              330              335

     Ser Thr Asn Val Lys Ile Gly Gly Tyr Asn Ile Pro Lys Gly Ala Asn
                 340              345              350

     Val Met Val Asn Val Trp Ala Ile Ala Arg Asp Pro Lys Val Trp Ser
             355              360              365

     Asn Pro Leu Glu Tyr Arg Pro Glu Arg Phe Ile Glu Glu Asn Ile Asp
         370              375              380

     Ile Lys Gly Ser Asp Phe Arg Val Leu Pro Phe Gly Ala Gly Arg Arg
     385              390              395              400

     Val Cys Pro Gly Ala Gln Leu Gly Ile Asn Leu Val Ala Ser Met Ile
                 405              410              415

     Gly His Leu Leu His Gln Phe Glu Trp Ser Leu Pro Glu Gly Thr Arg
                 420              425              430

     Pro Glu Asp Val Asn Met Met Glu Ser Asn Gly Val Val Thr Phe Met
             435              440              445

     Ser Thr Ser Leu Gln Val Ile Ala Lys Pro Arg Leu Asp Asn Pro Asp
         450              455              460

     Leu Tyr Lys Arg Phe Pro Val Glu Met
     465              470
```

```
<210> 21
<211> 1521
<212> DNA
<213> Lithospermum erythrorhizon

<220>
<221> CDS
<222> (1)..(1521)

<400> 21
atg gcc ctg cca gcc ata ccc cta gcc atc ata atc ttc ctc ata ata    48
Met Ala Leu Pro Ala Ile Pro Leu Ala Ile Ile Ile Phe Leu Ile Ile
  1               5                  10                  15

tcc tac aaa ctc tac caa aaa ctc cgg ttg aag ctg ccc ccc ggt cca    96
Ser Tyr Lys Leu Tyr Gln Lys Leu Arg Leu Lys Leu Pro Pro Gly Pro
                 20                  25                  30

cgc ccc ctc cca ata atc ggc aac atc tac caa gtg aaa ccc gtc aag   144
Arg Pro Leu Pro Ile Ile Gly Asn Ile Tyr Gln Val Lys Pro Val Lys
             35                  40                  45

ttc cgc tgc ttc tat aac tgg tcc aaa aca tac ggc ccc ata ttc tca   192
Phe Arg Cys Phe Tyr Asn Trp Ser Lys Thr Tyr Gly Pro Ile Phe Ser
         50                  55                  60

atc tac tat ggt tcc caa atg aac gta atc gtt tcg aca acg gaa ctt   240
```

```
          Ile Tyr Tyr Gly Ser Gln Met Asn Val Ile Val Ser Thr Thr Glu Leu
          65                  70                  75                  80

          gct aag gaa gtg ctt aaa gaa aat gat cag cat ttg gct gac agg ttt   288
          Ala Lys Glu Val Leu Lys Glu Asn Asp Gln His Leu Ala Asp Arg Phe
                           85                  90                  95

          cgg aca aga tca tct gct agt atg agt cga ggt ggt aag gac ttg att   336
          Arg Thr Arg Ser Ser Ala Ser Met Ser Arg Gly Gly Lys Asp Leu Ile
                       100                 105                 110

          tgg gca gat tat ggt ccg cat tat gtt aag gtt agg aag ctg tgt aat   384
          Trp Ala Asp Tyr Gly Pro His Tyr Val Lys Val Arg Lys Leu Cys Asn
                   115                 120                 125

          gtt gag ctg ttt tct ccc aag agg ctt gag gct att agg cct atg agg   432
          Val Glu Leu Phe Ser Pro Lys Arg Leu Glu Ala Ile Arg Pro Met Arg
               130                 135                 140

          gaa gat gag tat act gct ttg gtt gag tct att tat aag gat tgt act   480
          Glu Asp Glu Tyr Thr Ala Leu Val Glu Ser Ile Tyr Lys Asp Cys Thr
          145                 150                 155                 160

          aag cca gaa ttg aaa ggc aag agt ttg ttg gtg agg gag tat ttg agt   528
          Lys Pro Glu Leu Lys Gly Lys Ser Leu Leu Val Arg Glu Tyr Leu Ser
                           165                 170                 175

          tca gtg gca ttt aac aat att aca cgg ttg gct ttt ggg aaa agg ttt   576
          Ser Val Ala Phe Asn Asn Ile Thr Arg Leu Ala Phe Gly Lys Arg Phe
                       180                 185                 190

          atg gac tca aat gga gtg gta aat gag caa ggt caa gaa ttc aag aaa   624
          Met Asp Ser Asn Gly Val Val Asn Glu Gln Gly Gln Glu Phe Lys Lys
                       195                 200                 205

          atc aca cat gat ggc ata aag atc act gca aaa ctt tcg ata gca gag   672
          Ile Thr His Asp Gly Ile Lys Ile Thr Ala Lys Leu Ser Ile Ala Glu
                   210                 215                 220

          tac att cca tgg atc cgg tgg atg ttc aag gtc gaa cag gat gcg ctt   720
          Tyr Ile Pro Trp Ile Arg Trp Met Phe Lys Val Glu Gln Asp Ala Leu
          225                 230                 235                 240

          gat aag ttt gct gct gat aga gac cat ctt act aga gtg atc atg gaa   768
          Asp Lys Phe Ala Ala Asp Arg Asp His Leu Thr Arg Val Ile Met Glu
                       245                 250                 255

          gag cac att aag agt ggc aac acc aag cag cac ttt gtt gat gct ttg   816
          Glu His Ile Lys Ser Gly Asn Thr Lys Gln His Phe Val Asp Ala Leu
                       260                 265                 270

          ctc act ctt cag aag caa tat gat atc agt gaa gat acc att att gga   864
          Leu Thr Leu Gln Lys Gln Tyr Asp Ile Ser Glu Asp Thr Ile Ile Gly
                       275                 280                 285

          ctt ctt tgg gat atg att gct gct ggc atg gac acg gct acg atc tcc   912
          Leu Leu Trp Asp Met Ile Ala Ala Gly Met Asp Thr Ala Thr Ile Ser
                       290                 295                 300

          act gaa tgg gca atg gct gaa ctg gta agg aac cca aga gtg caa cga   960
          Thr Glu Trp Ala Met Ala Glu Leu Val Arg Asn Pro Arg Val Gln Arg
```

```
     305                      310                      315                      320
     aag gct caa gag gaa cta gac cgt gtg gtt gga cct gat cgc att atg        1008
     Lys Ala Gln Glu Glu Leu Asp Arg Val Val Gly Pro Asp Arg Ile Met
                     325                  330                  335

     act gaa gct gat gtc ccc aaa ttg ccc tac ttg caa tgc att gtc aaa        1056
     Thr Glu Ala Asp Val Pro Lys Leu Pro Tyr Leu Gln Cys Ile Val Lys
                 340                  345                  350

     gaa tca cta agg tta cat ccc cca acc cca ttg atg ctc cca cac aga        1104
     Glu Ser Leu Arg Leu His Pro Pro Thr Pro Leu Met Leu Pro His Arg
             355                  360                  365

     gcc agc gct aat gtg aaa att ggc ggg tat gac atc cct aaa gga tca        1152
     Ala Ser Ala Asn Val Lys Ile Gly Gly Tyr Asp Ile Pro Lys Gly Ser
             370                  375                  380

     atc gtc cat gtt aat gtc tgg gcc att gcg cgt gat cca gca tat tgg        1200
     Ile Val His Val Asn Val Trp Ala Ile Ala Arg Asp Pro Ala Tyr Trp
     385                  390                  395                  400

     aaa aac cct gag gaa ttc aga cct gaa aga ttc atg gag gaa gat ata        1248
     Lys Asn Pro Glu Glu Phe Arg Pro Glu Arg Phe Met Glu Glu Asp Ile
                     405                  410                  415

     gac atg aaa ggc aca gat tac aga ttg cta cca ttt ggt gct gga agg        1296
     Asp Met Lys Gly Thr Asp Tyr Arg Leu Leu Pro Phe Gly Ala Gly Arg
                 420                  425                  430

     agg ata tgt cct gga gct cag cta gct att aac ctg atc aca tct agt        1344
     Arg Ile Cys Pro Gly Ala Gln Leu Ala Ile Asn Leu Ile Thr Ser Ser
             435                  440                  445

     ctt ggc cat tta ttg cat cag ttt act tgg tcg cca cag cca ggg gtg        1392
     Leu Gly His Leu Leu His Gln Phe Thr Trp Ser Pro Gln Pro Gly Val
             450                  455                  460

     aag ccg gag gag att gac tta tcg gag aac ccc ggt aca gtt aca tac        1440
     Lys Pro Glu Glu Ile Asp Leu Ser Glu Asn Pro Gly Thr Val Thr Tyr
     465                  470                  475                  480

     atg cgc aac ccg gtg aag gct gtt gtt tca cct cgt tta tcc gct gtt        1488
     Met Arg Asn Pro Val Lys Ala Val Val Ser Pro Arg Leu Ser Ala Val
                 485                  490                  495

     cac tta tac aaa cga gtc gag tcg gac atg taa                           1521
     His Leu Tyr Lys Arg Val Glu Ser Asp Met
                 500                  505


     <210> 22
     <211> 506
     <212> PRT
     <213> Lithospermum erythrorhizon

     <400> 22
     Met Ala Leu Pro Ala Ile Pro Leu Ala Ile Ile Ile Phe Leu Ile Ile
       1                   5                  10                  15

     Ser Tyr Lys Leu Tyr Gln Lys Leu Arg Leu Lys Leu Pro Pro Gly Pro
```

```
                    20                  25                  30

         Arg Pro Leu Pro Ile Ile Gly Asn Ile Tyr Gln Val Lys Pro Val Lys
                 35                  40                  45

         Phe Arg Cys Phe Tyr Asn Trp Ser Lys Thr Tyr Gly Pro Ile Phe Ser
             50                  55                  60

         Ile Tyr Tyr Gly Ser Gln Met Asn Val Ile Val Ser Thr Thr Glu Leu
         65                  70                  75                  80

         Ala Lys Glu Val Leu Lys Glu Asn Asp Gln His Leu Ala Asp Arg Phe
                         85                  90                  95

         Arg Thr Arg Ser Ser Ala Ser Met Ser Arg Gly Gly Lys Asp Leu Ile
                     100                 105                 110

         Trp Ala Asp Tyr Gly Pro His Tyr Val Lys Val Arg Lys Leu Cys Asn
                 115                 120                 125

         Val Glu Leu Phe Ser Pro Lys Arg Leu Glu Ala Ile Arg Pro Met Arg
             130                 135                 140

         Glu Asp Glu Tyr Thr Ala Leu Val Glu Ser Ile Tyr Lys Asp Cys Thr
         145                 150                 155                 160

         Lys Pro Glu Leu Lys Gly Lys Ser Leu Leu Val Arg Glu Tyr Leu Ser
                     165                 170                 175

         Ser Val Ala Phe Asn Asn Ile Thr Arg Leu Ala Phe Gly Lys Arg Phe
                     180                 185                 190

         Met Asp Ser Asn Gly Val Val Asn Glu Gln Gly Gln Glu Phe Lys Lys
                     195                 200                 205

         Ile Thr His Asp Gly Ile Lys Ile Thr Ala Lys Leu Ser Ile Ala Glu
             210                 215                 220

         Tyr Ile Pro Trp Ile Arg Trp Met Phe Lys Val Glu Gln Asp Ala Leu
         225                 230                 235                 240

         Asp Lys Phe Ala Ala Asp Arg Asp His Leu Thr Arg Val Ile Met Glu
                     245                 250                 255

         Glu His Ile Lys Ser Gly Asn Thr Lys Gln His Phe Val Asp Ala Leu
                     260                 265                 270

         Leu Thr Leu Gln Lys Gln Tyr Asp Ile Ser Glu Asp Thr Ile Ile Gly
                 275                 280                 285

         Leu Leu Trp Asp Met Ile Ala Ala Gly Met Asp Thr Ala Thr Ile Ser
                 290                 295                 300

         Thr Glu Trp Ala Met Ala Glu Leu Val Arg Asn Pro Arg Val Gln Arg
         305                 310                 315                 320

         Lys Ala Gln Glu Glu Leu Asp Arg Val Val Gly Pro Asp Arg Ile Met
                     325                 330                 335

         Thr Glu Ala Asp Val Pro Lys Leu Pro Tyr Leu Gln Cys Ile Val Lys
                     340                 345                 350
```

```
Glu Ser Leu Arg Leu His Pro Pro Thr Pro Leu Met Leu Pro His Arg
        355             360             365

Ala Ser Ala Asn Val Lys Ile Gly Gly Tyr Asp Ile Pro Lys Gly Ser
        370             375             380

Ile Val His Val Asn Val Trp Ala Ile Ala Arg Asp Pro Ala Tyr Trp
385             390             395             400

Lys Asn Pro Glu Glu Phe Arg Pro Glu Arg Phe Met Glu Glu Asp Ile
            405             410             415

Asp Met Lys Gly Thr Asp Tyr Arg Leu Leu Pro Phe Gly Ala Gly Arg
            420             425             430

Arg Ile Cys Pro Gly Ala Gln Leu Ala Ile Asn Leu Ile Thr Ser Ser
        435             440             445

Leu Gly His Leu Leu His Gln Phe Thr Trp Ser Pro Gln Pro Gly Val
        450             455             460

Lys Pro Glu Glu Ile Asp Leu Ser Glu Asn Pro Gly Thr Val Thr Tyr
465             470             475             480

Met Arg Asn Pro Val Lys Ala Val Val Ser Pro Arg Leu Ser Ala Val
            485             490             495

His Leu Tyr Lys Arg Val Glu Ser Asp Met
            500             505
```

```
<210> 23
<211> 1536
<212> DNA
<213> Triticum aestivum

<220>
<221> CDS
<222> (1)..(1536)

<400> 23
atg gac gcg gcg tcg ctg ctc ccg ttc gcc atc gcg ctg gtg gcg atc      48
Met Asp Ala Ala Ser Leu Leu Pro Phe Ala Ile Ala Leu Val Ala Ile
  1               5              10                      15

ccg atc tcg ctg gcg ctg ctg gac cgg ctg cgg ctg ggc cgg ctg ccg      96
Pro Ile Ser Leu Ala Leu Leu Asp Arg Leu Arg Leu Gly Arg Leu Pro
                 20              25                      30

ccg ggc ccg cgc ccg tgg ccg gtg gtg ggc aac ctg cgg cag atc aag     144
Pro Gly Pro Arg Pro Trp Pro Val Val Gly Asn Leu Arg Gln Ile Lys
             35              40                      45

ccg gtg cgc cgc tgc ttc cag gag tgg gcg gag cgg tac ggg ccc atc     192
Pro Val Arg Arg Cys Phe Gln Glu Trp Ala Glu Arg Tyr Gly Pro Ile
         50              55                      60

atc tcc gtc tgg ttc ggc tcc tcg ctc acc gtc gtc gtc tcc acc tcc     240
Ile Ser Val Trp Phe Gly Ser Ser Leu Thr Val Val Val Ser Thr Ser
```

32

```
                65                    70                    75                    80
      gag ctg gcc aag gag gtg ctc aag gag cac gac cag cag ctc gcc gac   288
      Glu Leu Ala Lys Glu Val Leu Lys Glu His Asp Gln Gln Leu Ala Asp
                      85                    90                    95

      cgg ccc cgc aac cgc tcc acc cag cgc ttc agc cgc aac ggc cag gac   336
      Arg Pro Arg Asn Arg Ser Thr Gln Arg Phe Ser Arg Asn Gly Gln Asp
                      100                   105                   110

      ctc atc tgg gcc gac tac ggc ccg cac tac atc aag gtg cgc aag ctc   384
      Leu Ile Trp Ala Asp Tyr Gly Pro His Tyr Ile Lys Val Arg Lys Leu
                      115                   120                   125

      tgc aac ctc gag ctc ttc acc cag aag cgc ctc gag gcg ctg cgc tcc   432
      Cys Asn Leu Glu Leu Phe Thr Gln Lys Arg Leu Glu Ala Leu Arg Ser
                130                   135                   140

      atc cgc gag gac gag gtc acc gcc atg gtc gag tcc gtc cac cgc gcc   480
      Ile Arg Glu Asp Glu Val Thr Ala Met Val Glu Ser Val His Arg Ala
      145                   150                   155                   160

      gcc gcc ggc ccc ggt aat gaa gga aag cca ttg gta gtg agg aat cac   528
      Ala Ala Gly Pro Gly Asn Glu Gly Lys Pro Leu Val Val Arg Asn His
                      165                   170                   175

      ctt gct atg gtg tcc ttc aac aat ata aca agg cta gct ttt ggg aag   576
      Leu Ala Met Val Ser Phe Asn Asn Ile Thr Arg Leu Ala Phe Gly Lys
                      180                   185                   190

      cgg ttc atg aat gca aat ggt gac att gac gaa gaa ggg cag gag ttc   624
      Arg Phe Met Asn Ala Asn Gly Asp Ile Asp Glu Glu Gly Gln Glu Phe
                      195                   200                   205

      aag att att gtc aac aat ggt atc aaa att ggt gca tct ctc tct gtt   672
      Lys Ile Ile Val Asn Asn Gly Ile Lys Ile Gly Ala Ser Leu Ser Val
                      210                   215                   220

      gct gaa tac att tgg tac ttg aga tgg ctg tgt cca ctt aat gag gag   720
      Ala Glu Tyr Ile Trp Tyr Leu Arg Trp Leu Cys Pro Leu Asn Glu Glu
      225                   230                   235                   240

      ctt tac aag acc cac aat gag agg agg gat cgt ctg acc aag aag atc   768
      Leu Tyr Lys Thr His Asn Glu Arg Arg Asp Arg Leu Thr Lys Lys Ile
                      245                   250                   255

      att gaa gag cat gca cag gcc ctc cag gag aga ggt gcc aaa cag cac   816
      Ile Glu Glu His Ala Gln Ala Leu Gln Glu Arg Gly Ala Lys Gln His
                      260                   265                   270

      ttt gtg gat gca ctg ttc act ctc aga gag aag tat gac ctt agt gat   864
      Phe Val Asp Ala Leu Phe Thr Leu Arg Glu Lys Tyr Asp Leu Ser Asp
                      275                   280                   285

      gac aca gtt ttc gga ctt cta tgg gac atg atc acc gcc gga atg gac   912
      Asp Thr Val Phe Gly Leu Leu Trp Asp Met Ile Thr Ala Gly Met Asp
                      290                   295                   300

      acg aca gtc ata tca gtc gag tgg gcc atg gca gag ctg gtg agg aac   960
      Thr Thr Val Ile Ser Val Glu Trp Ala Met Ala Glu Leu Val Arg Asn
      305                   310                   315                   320
```

```
ccc agg gtg cag aag aag ctg cag gag gag ctg gac agc gtg gtc ggc    1008
Pro Arg Val Gln Lys Lys Leu Gln Glu Glu Leu Asp Ser Val Val Gly
                325             330             335

cgc gat cgc gtc atg tcc gag acc gac ttc ccg aac ctg ccc tac ctg    1056
Arg Asp Arg Val Met Ser Glu Thr Asp Phe Pro Asn Leu Pro Tyr Leu
            340             345             350

atg gcc gtc gtg aag gag tcc ctc cgc ctg cac ccg ccg acg ccg ctc    1104
Met Ala Val Val Lys Glu Ser Leu Arg Leu His Pro Pro Thr Pro Leu
        355             360             365

atg ctg ccc cac aag gcc agc gcg agc gtc aag gtc ggc ggc tac agc    1152
Met Leu Pro His Lys Ala Ser Ala Ser Val Lys Val Gly Gly Tyr Ser
    370             375             380

atc ccc aag ggc gcc aac gtg atg gtg aac gtg tgg gcg gtg gcc cgg    1200
Ile Pro Lys Gly Ala Asn Val Met Val Asn Val Trp Ala Val Ala Arg
385             390             395             400

gac ccc aag gtg tgg agc agc ccg ctg gag ttc cgg ccg gag cgg ttc    1248
Asp Pro Lys Val Trp Ser Ser Pro Leu Glu Phe Arg Pro Glu Arg Phe
                405             410             415

ctg gag gag agc atc gac atc aag ggc agc gac ttc agg gtg ctg ccc    1296
Leu Glu Glu Ser Ile Asp Ile Lys Gly Ser Asp Phe Arg Val Leu Pro
                420             425             430

ttc ggc gcg ggc cgg agg gtg tgc ccc ggc gcg cag ctc ggg atc aac    1344
Phe Gly Ala Gly Arg Arg Val Cys Pro Gly Ala Gln Leu Gly Ile Asn
        435             440             445

ctc gtc gcc tcc atg atc ggg cac atg ctg cac cac ttc gag tgg tct    1392
Leu Val Ala Ser Met Ile Gly His Met Leu His His Phe Glu Trp Ser
    450             455             460

ctg ccg gag ggc gcc agg ccg gag gac atc agc atg atg gag tcc ccc    1440
Leu Pro Glu Gly Ala Arg Pro Glu Asp Ile Ser Met Met Glu Ser Pro
465             470             475             480

ggg ctc gtc acc ttc atg ggc acg ccg ctg cag gcc gtc gcc acg ccg    1488
Gly Leu Val Thr Phe Met Gly Thr Pro Leu Gln Ala Val Ala Thr Pro
                485             490             495

cgc ctg gag aat gag gag ctg tac aag agg gtc ccg gtt gag atc tga    1536
Arg Leu Glu Asn Glu Glu Leu Tyr Lys Arg Val Pro Val Glu Ile  *
            500             505          *       510
```

<210> 24
<211> 511
<212> PRT
<213> Triticum aestivum

<400> 24

```
Met Asp Ala Ala Ser Leu Leu Pro Phe Ala Ile Ala Leu Val Ala Ile
1               5               10              15

Pro Ile Ser Leu Ala Leu Leu Asp Arg Leu Arg Leu Gly Arg Leu Pro
            20              25              30
```

```
Pro Gly Pro Arg Pro Trp Pro Val Val Gly Asn Leu Arg Gln Ile Lys
        35                  40                  45

Pro Val Arg Arg Cys Phe Gln Glu Trp Ala Glu Arg Tyr Gly Pro Ile
    50                  55                  60

Ile Ser Val Trp Phe Gly Ser Ser Leu Thr Val Val Val Ser Thr Ser
65                  70                  75                  80

Glu Leu Ala Lys Glu Val Leu Lys Glu His Asp Gln Gln Leu Ala Asp
            85                  90                  95

Arg Pro Arg Asn Arg Ser Thr Gln Arg Phe Ser Arg Asn Gly Gln Asp
            100                 105                 110

Leu Ile Trp Ala Asp Tyr Gly Pro His Tyr Ile Lys Val Arg Lys Leu
        115                 120                 125

Cys Asn Leu Glu Leu Phe Thr Gln Lys Arg Leu Glu Ala Leu Arg Ser
    130                 135                 140

Ile Arg Glu Asp Glu Val Thr Ala Met Val Glu Ser Val His Arg Ala
145                 150                 155                 160

Ala Ala Gly Pro Gly Asn Glu Gly Lys Pro Leu Val Val Arg Asn His
            165                 170                 175

Leu Ala Met Val Ser Phe Asn Asn Ile Thr Arg Leu Ala Phe Gly Lys
            180                 185                 190

Arg Phe Met Asn Ala Asn Gly Asp Ile Asp Glu Glu Gly Gln Glu Phe
        195                 200                 205

Lys Ile Ile Val Asn Asn Gly Ile Lys Ile Gly Ala Ser Leu Ser Val
    210                 215                 220

Ala Glu Tyr Ile Trp Tyr Leu Arg Trp Leu Cys Pro Leu Asn Glu Glu
225                 230                 235                 240

Leu Tyr Lys Thr His Asn Glu Arg Arg Asp Arg Leu Thr Lys Lys Ile
            245                 250                 255

Ile Glu Glu His Ala Gln Ala Leu Gln Glu Arg Gly Ala Lys Gln His
            260                 265                 270

Phe Val Asp Ala Leu Phe Thr Leu Arg Glu Lys Tyr Asp Leu Ser Asp
        275                 280                 285

Asp Thr Val Phe Gly Leu Leu Trp Asp Met Ile Thr Ala Gly Met Asp
    290                 295                 300

Thr Thr Val Ile Ser Val Glu Trp Ala Met Ala Glu Leu Val Arg Asn
305                 310                 315                 320

Pro Arg Val Gln Lys Lys Leu Gln Glu Glu Leu Asp Ser Val Val Gly
            325                 330                 335

Arg Asp Arg Val Met Ser Glu Thr Asp Phe Pro Asn Leu Pro Tyr Leu
        340                 345                 350
```

```
Met Ala Val Val Lys Glu Ser Leu Arg Leu His Pro Pro Thr Pro Leu
        355             360             365

Met Leu Pro His Lys Ala Ser Ala Ser Val Lys Val Gly Gly Tyr Ser
        370             375             380

Ile Pro Lys Gly Ala Asn Val Met Val Asn Val Trp Ala Val Ala Arg
385             390             395             400

Asp Pro Lys Val Trp Ser Ser Pro Leu Glu Phe Arg Pro Glu Arg Phe
            405             410             415

Leu Glu Glu Ser Ile Asp Ile Lys Gly Ser Asp Phe Arg Val Leu Pro
        420             425             430

Phe Gly Ala Gly Arg Arg Val Cys Pro Gly Ala Gln Leu Gly Ile Asn
        435             440             445

Leu Val Ala Ser Met Ile Gly His Met Leu His His Phe Glu Trp Ser
        450             455             460

Leu Pro Glu Gly Ala Arg Pro Glu Asp Ile Ser Met Met Glu Ser Pro
465             470             475             480

Gly Leu Val Thr Phe Met Gly Thr Pro Leu Gln Ala Val Ala Thr Pro
            485             490             495

Arg Leu Glu Asn Glu Glu Leu Tyr Lys Arg Val Pro Val Glu Ile
        500             505             510
```

<210> 25
<211> 1539
<212> DNA
<213> Triticum aestivum

<220>
<221> CDS
<222> (1)..(1539)

<400> 25

```
atg gac gtg gcg tcg ctg ctc ccg ttc gcc atc gcg ctg gtg gcg atc    48
Met Asp Val Ala Ser Leu Leu Pro Phe Ala Ile Ala Leu Val Ala Ile
  1               5              10              15

ccg atc tcg ctg gcg ctg ctg gac cgg ctg cgg ctg ggg cgg ctg ccg    96
Pro Ile Ser Leu Ala Leu Leu Asp Arg Leu Arg Leu Gly Arg Leu Pro
            20              25              30

ccg ggc ccg cgc ccg tgg ccg gtg gtg ggc aac ctg cgg cag atc aag   144
Pro Gly Pro Arg Pro Trp Pro Val Val Gly Asn Leu Arg Gln Ile Lys
            35              40              45

ccg gtg cgg tgc cgc tgc ttc cag gag tgg gcg gcg cgg tac ggg ccc   192
Pro Val Arg Cys Arg Cys Phe Gln Glu Trp Ala Ala Arg Tyr Gly Pro
        50              55              60

atc atc tcc gtc tgg ttc ggc tcc tcg ctc acc gtc gtc gtc tcc acc   240
Ile Ile Ser Val Trp Phe Gly Ser Ser Leu Thr Val Val Val Ser Thr
 65              70              75              80
```

36

tcc gag ctg gcc aag gag gtg ctc aag gag cac gac cag cag ctc gcc   288
Ser Glu Leu Ala Lys Glu Val Leu Lys Glu His Asp Gln Gln Leu Ala
            85               90               95

gac cgg ccc cgc aac cgc tcc acc cag cgc ttc agc cgc aac ggc cag   336
Asp Arg Pro Arg Asn Arg Ser Thr Gln Arg Phe Ser Arg Asn Gly Gln
            100              105            110

gac ctc atc tgg gcc gac tac ggc ccg cac tac atc aag gtg cgc aag   384
Asp Leu Ile Trp Ala Asp Tyr Gly Pro His Tyr Ile Lys Val Arg Lys
            115              120            125

ctc tgc aac ctc gag ctc ttc acc cag aag cgc ctc gag gcg ctg cgc   432
Leu Cys Asn Leu Glu Leu Phe Thr Gln Lys Arg Leu Glu Ala Leu Arg
            130              135            140

ccc atc cgc gag gac gag gtc acc gcc atg gtc gag tcc gtc cac cgc   480
Pro Ile Arg Glu Asp Glu Val Thr Ala Met Val Glu Ser Val His Arg
145               150              155            160

gcc gcc gcc ggc ccc ggt aat gaa gga aag cca ttg gta gtg agg aat   528
Ala Ala Ala Gly Pro Gly Asn Glu Gly Lys Pro Leu Val Val Arg Asn
            165              170            175

cac ctt gct atg gtg tcc ttc aac aat ata aca agg cta gct ttt ggg   576
His Leu Ala Met Val Ser Phe Asn Asn Ile Thr Arg Leu Ala Phe Gly
            180              185            190

aag cgg ttc atg aat gca gat ggt gac att gac gaa gaa ggg cag gaa   624
Lys Arg Phe Met Asn Ala Asp Gly Asp Ile Asp Glu Glu Gly Gln Glu
            195              200            205

ttc aag ctt att gtc aac aat ggg atc aaa att ggt gca tct ctt tct   672
Phe Lys Leu Ile Val Asn Asn Gly Ile Lys Ile Gly Ala Ser Leu Ser
            210              215            220

gtt gct gaa tac att tgg tac ttg aga tgg ctg tgt cca ctt aat gag   720
Val Ala Glu Tyr Ile Trp Tyr Leu Arg Trp Leu Cys Pro Leu Asn Glu
225               230              235            240

gag ctt tac aat acc cac aat gag aga agg gat cgt ctg acc aag aag   768
Glu Leu Tyr Asn Thr His Asn Glu Arg Arg Asp Arg Leu Thr Lys Lys
            245              250            255

atc att gaa gag cat gca cag gcc ctc agg gag agt ggt gcc aaa cag   816
Ile Ile Glu Glu His Ala Gln Ala Leu Arg Glu Ser Gly Ala Lys Gln
            260              265            270

cac ttt gtg gat gct ctg ttc act ctc aga gag aag tat gac ctt agt   864
His Phe Val Asp Ala Leu Phe Thr Leu Arg Glu Lys Tyr Asp Leu Ser
            275              280            285

gat gac aca gtt ttc gga ctt cta tgg gac atg atc acc gcc gga atg   912
Asp Asp Thr Val Phe Gly Leu Leu Trp Asp Met Ile Thr Ala Gly Met
            290              295            300

gac acg aca gtc ata tca gtc gaa tgg gcc atg gca gag ctg gtc cgg   960
Asp Thr Thr Val Ile Ser Val Glu Trp Ala Met Ala Glu Leu Val Arg
305               310              315            320

```
aac ccc agg gtg cag aag aag ctg caa gag gag ctg gac agc gtg gtc     1008
Asn Pro Arg Val Gln Lys Lys Leu Gln Glu Glu Leu Asp Ser Val Val
            325             330             335

ggc cgc gac cgc gtc atg tcc gag acc gac ttc cag aac ctg ccc tac     1056
Gly Arg Asp Arg Val Met Ser Glu Thr Asp Phe Gln Asn Leu Pro Tyr
            340             345             350

ctg atg gcc gtc gtg aag gag tcc ctc cgc ctg cac ccg ccg acg ccg     1104
Leu Met Ala Val Val Lys Glu Ser Leu Arg Leu His Pro Pro Thr Pro
        355             360             365

ctc atg ctg ccc cac aag gcc agc gcg agc gtc aag gtc ggc ggc tac     1152
Leu Met Leu Pro His Lys Ala Ser Ala Ser Val Lys Val Gly Gly Tyr
    370             375             380

agc atc ccc aag ggc gcc aac gtg atg gtg aac gtg tgg gcg gtg gcc     1200
Ser Ile Pro Lys Gly Ala Asn Val Met Val Asn Val Trp Ala Val Ala
385             390             395             400

cgg gac ccc aag gtg tgg agc agc ccg ctg gag ttc cgg ccg gag cgg     1248
Arg Asp Pro Lys Val Trp Ser Ser Pro Leu Glu Phe Arg Pro Glu Arg
            405             410             415

ttc ctg gag gag agc atc gac atc aag ggc agc gac ttc agg gtg ctg     1296
Phe Leu Glu Glu Ser Ile Asp Ile Lys Gly Ser Asp Phe Arg Val Leu
            420             425             430

ccc ttc ggc gcc ggc cgg agg gtg tgc ccc ggc gcg cag ctc ggg atc     1344
Pro Phe Gly Ala Gly Arg Arg Val Cys Pro Gly Ala Gln Leu Gly Ile
        435             440             445

aac ctc gtc gcc tcc atg atc ggc cac atg ctg cac cac ttc gag tgg     1392
Asn Leu Val Ala Ser Met Ile Gly His Met Leu His His Phe Glu Trp
    450             455             460

tct ctg ccg gag ggc gcc agg ccg gag gac atc agc atg atg gag tcc     1440
Ser Leu Pro Glu Gly Ala Arg Pro Glu Asp Ile Ser Met Met Glu Ser
465             470             475             480

ccc ggg ctc gtc acc ttc atg ggc acg ctg ctg cag gcc gtc gcc acg     1488
Pro Gly Leu Val Thr Phe Met Gly Thr Leu Leu Gln Ala Val Ala Thr
            485             490             495

ccg cgc ctg gag aat gag gag ctc tac aag agg gtc ccg gtc gag atc     1536
Pro Arg Leu Glu Asn Glu Glu Leu Tyr Lys Arg Val Pro Val Glu Ile
            500             505             510

tga                                                                 1539
```

<210> 26
<211> 512
<212> PRT
<213> Triticum aestivum

<400> 26
Met Asp Val Ala Ser Leu Leu Pro Phe Ala Ile Ala Leu Val Ala Ile
1               5               10              15

```
Pro Ile Ser Leu Ala Leu Leu Asp Arg Leu Arg Leu Gly Arg Leu Pro
        20                  25                  30

Pro Gly Pro Arg Pro Trp Pro Val Val Gly Asn Leu Arg Gln Ile Lys
        35                  40                  45

Pro Val Arg Cys Arg Cys Phe Gln Glu Trp Ala Ala Arg Tyr Gly Pro
        50                  55                  60

Ile Ile Ser Val Trp Phe Gly Ser Ser Leu Thr Val Val Val Ser Thr
65                  70                  75                  80

Ser Glu Leu Ala Lys Glu Val Leu Lys Glu His Asp Gln Gln Leu Ala
                85                  90                  95

Asp Arg Pro Arg Asn Arg Ser Thr Gln Arg Phe Ser Arg Asn Gly Gln
            100                 105                 110

Asp Leu Ile Trp Ala Asp Tyr Gly Pro His Tyr Ile Lys Val Arg Lys
        115                 120                 125

Leu Cys Asn Leu Glu Leu Phe Thr Gln Lys Arg Leu Glu Ala Leu Arg
    130                 135                 140

Pro Ile Arg Glu Asp Glu Val Thr Ala Met Val Glu Ser Val His Arg
145                 150                 155                 160

Ala Ala Ala Gly Pro Gly Asn Glu Gly Lys Pro Leu Val Val Arg Asn
            165                 170                 175

His Leu Ala Met Val Ser Phe Asn Asn Ile Thr Arg Leu Ala Phe Gly
            180                 185                 190

Lys Arg Phe Met Asn Ala Asp Gly Asp Ile Asp Glu Glu Gly Gln Glu
        195                 200                 205

Phe Lys Leu Ile Val Asn Asn Gly Ile Lys Ile Gly Ala Ser Leu Ser
    210                 215                 220

Val Ala Glu Tyr Ile Trp Tyr Leu Arg Trp Leu Cys Pro Leu Asn Glu
225                 230                 235                 240

Glu Leu Tyr Asn Thr His Asn Glu Arg Arg Asp Arg Leu Thr Lys Lys
            245                 250                 255

Ile Ile Glu Glu His Ala Gln Ala Leu Arg Glu Ser Gly Ala Lys Gln
            260                 265                 270

His Phe Val Asp Ala Leu Phe Thr Leu Arg Glu Lys Tyr Asp Leu Ser
    275                 280                 285

Asp Asp Thr Val Phe Gly Leu Leu Trp Asp Met Ile Thr Ala Gly Met
    290                 295                 300

Asp Thr Thr Val Ile Ser Val Glu Trp Ala Met Ala Glu Leu Val Arg
305                 310                 315                 320

Asn Pro Arg Val Gln Lys Lys Leu Gln Glu Glu Leu Asp Ser Val Val
            325                 330                 335

Gly Arg Asp Arg Val Met Ser Glu Thr Asp Phe Gln Asn Leu Pro Tyr
```

```
                    340                  345                      350

       Leu Met Ala Val Val Lys Glu Ser Leu Arg Leu His Pro Pro Thr Pro
               355                  360                  365

       Leu Met Leu Pro His Lys Ala Ser Ala Ser Val Lys Val Gly Gly Tyr
           370                  375                  380

       Ser Ile Pro Lys Gly Ala Asn Val Met Val Asn Val Trp Ala Val Ala
       385                  390                  395                  400

       Arg Asp Pro Lys Val Trp Ser Ser Pro Leu Glu Phe Arg Pro Glu Arg
                       405                  410                  415

       Phe Leu Glu Glu Ser Ile Asp Ile Lys Gly Ser Asp Phe Arg Val Leu
                   420                  425                  430

       Pro Phe Gly Ala Gly Arg Arg Val Cys Pro Gly Ala Gln Leu Gly Ile
                   435                  440                  445

       Asn Leu Val Ala Ser Met Ile Gly His Met Leu His His Phe Glu Trp
           450                  455                  460

       Ser Leu Pro Glu Gly Ala Arg Pro Glu Asp Ile Ser Met Met Glu Ser
       465                  470                  475                  480

       Pro Gly Leu Val Thr Phe Met Gly Thr Leu Leu Gln Ala Val Ala Thr
                       485                  490                  495

       Pro Arg Leu Glu Asn Glu Glu Leu Tyr Lys Arg Val Pro Val Glu Ile
                   500                  505                  510


       <210> 27
       <211> 1530
       <212> DNA
       <213> Triticum aestivum

       <220>
       <221> CDS
       <222> (1)..(1530)

       <400> 27
       atg gac acg gcc gcc ttg ctc ccg gtg gcg ctg gcc ctc atg gcc atc    48
       Met Asp Thr Ala Ala Leu Leu Pro Val Ala Leu Ala Leu Met Ala Ile
        1               5                  10                  15

       ccc gtc acg gcg ctc ctc ctc agc cgg ctc cgc ttc ggc agg ctg cca    96
       Pro Val Thr Ala Leu Leu Leu Ser Arg Leu Arg Phe Gly Arg Leu Pro
                       20                  25                  30

       ccg ggg ccg cgc ccg tgg ccg gtg ctg ggc aac ctc ttc caa atc cag   144
       Pro Gly Pro Arg Pro Trp Pro Val Leu Gly Asn Leu Phe Gln Ile Gln
                   35                  40                  45

       ccc gtg cgc tgc cgg tgc ttc gcc gag tgg gcg gcg cgg tac ggg ccc   192
       Pro Val Arg Cys Arg Cys Phe Ala Glu Trp Ala Ala Arg Tyr Gly Pro
               50                  55                  60

       atc atg acg gtc tgg ttc ggc tcg acg ccg atg gtg gtg gtg tcc acg   240
```

```
        Ile Met Thr Val Trp Phe Gly Ser Thr Pro Met Val Val Val Ser Thr
        65                  70                  75                  80

ccg gag ctg gcg cag gag gtg ctc agg acc cac gac cag cac ctg gcc        288
Pro Glu Leu Ala Gln Glu Val Leu Arg Thr His Asp Gln His Leu Ala
                85                  90                  95

gac cgc tcc cgg aac cgc tcc tcc gag cgc ttc agc cgc ggc ggc atg        336
Asp Arg Ser Arg Asn Arg Ser Ser Glu Arg Phe Ser Arg Gly Gly Met
            100                 105                 110

gac ctc atc tgg gcc gac tac ggc ccg cac tac atc aag gtg cgc aag        384
Asp Leu Ile Trp Ala Asp Tyr Gly Pro His Tyr Ile Lys Val Arg Lys
        115                 120                 125

ctc tgt aac ctc gag ctc ttc acg ccc cgc cgc ctc gag gcg ctc cgc        432
Leu Cys Asn Leu Glu Leu Phe Thr Pro Arg Arg Leu Glu Ala Leu Arg
        130                 135                 140

ccc gtc cgc gag gac gag gtc acc gcc atg gtc gag tcc gtg cac gcg        480
Pro Val Arg Glu Asp Glu Val Thr Ala Met Val Glu Ser Val His Ala
145                 150                 155                 160

gcc ggc aag gga ggc agg ccc gtg gcg gtg cgg gag ttc ctc gct atg        528
Ala Gly Lys Gly Gly Arg Pro Val Ala Val Arg Glu Phe Leu Ala Met
                165                 170                 175

gtc ggc ttc aac aac atc acg cgg ctc gcc ttc ggg aag cgg ttc ctg        576
Val Gly Phe Asn Asn Ile Thr Arg Leu Ala Phe Gly Lys Arg Phe Leu
            180                 185                 190

acc gcg gcc ggc gag ctg gac gag caa ggg cgc gag ttc aag gag atc        624
Thr Ala Ala Gly Glu Leu Asp Glu Gln Gly Arg Glu Phe Lys Glu Ile
        195                 200                 205

gtc aac aac ggc atc aag atc ggc gcg tcg ctg tcc atc gcc gag cac        672
Val Asn Asn Gly Ile Lys Ile Gly Ala Ser Leu Ser Ile Ala Glu His
        210                 215                 220

atc cgg tgg ctc cgg tgg ctc acg ccc gtc gac gag gtg gcc tat caa        720
Ile Arg Trp Leu Arg Trp Leu Thr Pro Val Asp Glu Val Ala Tyr Gln
225                 230                 235                 240

gcc cac ggc gac cgg cgc gat cgg ctc acc gtc aag atc atg gag gag        768
Ala His Gly Asp Arg Arg Asp Arg Leu Thr Val Lys Ile Met Glu Glu
                245                 250                 255

cac gcc agc gcc ctc gag cgg agc ggc gcc agc aag cag cat ttc gtc        816
His Ala Ser Ala Leu Glu Arg Ser Gly Ala Ser Lys Gln His Phe Val
            260                 265                 270

gac gcg ctc ttc acg ctc cgg gac aag tac gac ctc agc gac gac acc        864
Asp Ala Leu Phe Thr Leu Arg Asp Lys Tyr Asp Leu Ser Asp Asp Thr
        275                 280                 285

gtc att ggc ctc ctc tgg gac atg atc acc gcc ggc acg gac acg acg        912
Val Ile Gly Leu Leu Trp Asp Met Ile Thr Ala Gly Thr Asp Thr Thr
        290                 295                 300

gtg ata acc gtg gag tgg gcg gta gcg gag ctg gtg agg aac ccg acg        960
Val Ile Thr Val Glu Trp Ala Val Ala Glu Leu Val Arg Asn Pro Thr
```

305               310               315               320

```
gtg cag cac aag gtg cag gag gag ctg gac cgg gtg gtc ggc cgc gac    1008
Val Gln His Lys Val Gln Glu Glu Leu Asp Arg Val Val Gly Arg Asp
                325                 330                 335

cgg gtg ctg tcg gag acg gac ttc ccc aac ctg ccc tac ctg cag gcc    1056
Arg Val Leu Ser Glu Thr Asp Phe Pro Asn Leu Pro Tyr Leu Gln Ala
                340                 345                 350

atc gtc aag gag tcg ctc cgg ctg cac ccg ccg acg ccg ctg atg ctg    1104
Ile Val Lys Glu Ser Leu Arg Leu His Pro Pro Thr Pro Leu Met Leu
                355                 360                 365

ccg cac agg gcc agc gcc gcc gtg aag gtc gcc ggc tac gac atc ccc    1152
Pro His Arg Ala Ser Ala Ala Val Lys Val Ala Gly Tyr Asp Ile Pro
                370                 375                 380

aag gga gcc agc gtc acg gtg aac gtg tgg gcg atc gcg cgc gac ccg    1200
Lys Gly Ala Ser Val Thr Val Asn Val Trp Ala Ile Ala Arg Asp Pro
385                 390                 395                 400

gag gcg tgg gac agc ccg ctc gag ttc cgg ccc gag cgc ttc ctc cac    1248
Glu Ala Trp Asp Ser Pro Leu Glu Phe Arg Pro Glu Arg Phe Leu His
                405                 410                 415

gac aac atc gac atc aag ggg tgc gac tac cgc gtg ctg ccg ttc ggc    1296
Asp Asn Ile Asp Ile Lys Gly Cys Asp Tyr Arg Val Leu Pro Phe Gly
                420                 425                 430

gcc ggc cgc cgg gtg tgc ccc ggc gcg cag ctc ggg atc aac ctc gtg    1344
Ala Gly Arg Arg Val Cys Pro Gly Ala Gln Leu Gly Ile Asn Leu Val
                435                 440                 445

gcg tcc atg atc ggc cac ctc ctg cac cac ttc acc tgg gcg ctg ccg    1392
Ala Ser Met Ile Gly His Leu Leu His His Phe Thr Trp Ala Leu Pro
                450                 455                 460

gac ggg acc cgg ccg gag gac atc gac atg atg gag tcc ccc ggg ctc    1440
Asp Gly Thr Arg Pro Glu Asp Ile Asp Met Met Glu Ser Pro Gly Leu
465                 470                 475                 480

atc acc ttc atg cgc acg ccg ctg cag gtc gtc gcc acg ccg cgc ctc    1488
Ile Thr Phe Met Arg Thr Pro Leu Gln Val Val Ala Thr Pro Arg Leu
                485                 490                 495

gaa aag gag gag ctc tac aag cgg gtg gcc gcc gag atg tga            1530
Glu Lys Glu Glu Leu Tyr Lys Arg Val Ala Ala Glu Met
                500                 505                 510
```

<210> 28
<211> 509
<212> PRT
<213> Triticum aestivum

<400> 28
```
Met Asp Thr Ala Ala Leu Leu Pro Val Ala Leu Ala Leu Met Ala Ile
  1               5                  10                  15

Pro Val Thr Ala Leu Leu Leu Ser Arg Leu Arg Phe Gly Arg Leu Pro
```

|  |  |  | 20 |  |  |  | 25 |  |  |  |  | 30 |  |  |  |
|---|---|---|---|---|---|---|---|---|---|---|---|---|---|---|---|

Pro Gly Pro Arg Pro Trp Pro Val Leu Gly Asn Leu Phe Gln Ile Gln
          35              40                45

Pro Val Arg Cys Arg Cys Phe Ala Glu Trp Ala Ala Arg Tyr Gly Pro
      50          55              60

Ile Met Thr Val Trp Phe Gly Ser Thr Pro Met Val Val Val Ser Thr
65              70              75                  80

Pro Glu Leu Ala Gln Glu Val Leu Arg Thr His Asp Gln His Leu Ala
              85              90                95

Asp Arg Ser Arg Asn Arg Ser Ser Glu Arg Phe Ser Arg Gly Gly Met
          100             105             110

Asp Leu Ile Trp Ala Asp Tyr Gly Pro His Tyr Ile Lys Val Arg Lys
      115             120             125

Leu Cys Asn Leu Glu Leu Phe Thr Pro Arg Arg Leu Glu Ala Leu Arg
      130             135             140

Pro Val Arg Glu Asp Glu Val Thr Ala Met Val Glu Ser Val His Ala
145             150             155             160

Ala Gly Lys Gly Gly Arg Pro Val Ala Val Arg Glu Phe Leu Ala Met
          165             170             175

Val Gly Phe Asn Asn Ile Thr Arg Leu Ala Phe Gly Lys Arg Phe Leu
          180             185             190

Thr Ala Ala Gly Glu Leu Asp Glu Gln Gly Arg Glu Phe Lys Glu Ile
      195             200             205

Val Asn Asn Gly Ile Lys Ile Gly Ala Ser Leu Ser Ile Ala Glu His
      210             215             220

Ile Arg Trp Leu Arg Trp Leu Thr Pro Val Asp Glu Val Ala Tyr Gln
225             230             235             240

Ala His Gly Asp Arg Arg Asp Arg Leu Thr Val Lys Ile Met Glu Glu
          245             250             255

His Ala Ser Ala Leu Glu Arg Ser Gly Ala Ser Lys Gln His Phe Val
          260             265             270

Asp Ala Leu Phe Thr Leu Arg Asp Lys Tyr Asp Leu Ser Asp Asp Thr
      275             280             285

Val Ile Gly Leu Leu Trp Asp Met Ile Thr Ala Gly Thr Asp Thr Thr
      290             295             300

Val Ile Thr Val Glu Trp Ala Val Ala Glu Leu Val Arg Asn Pro Thr
305             310             315             320

Val Gln His Lys Val Gln Glu Glu Leu Asp Arg Val Val Gly Arg Asp
          325             330             335

Arg Val Leu Ser Glu Thr Asp Phe Pro Asn Leu Pro Tyr Leu Gln Ala
          340             345             350

```
Ile Val Lys Glu Ser Leu Arg Leu His Pro Pro Thr Pro Leu Met Leu
        355             360             365

Pro His Arg Ala Ser Ala Ala Val Lys Val Ala Gly Tyr Asp Ile Pro
        370             375             380

Lys Gly Ala Ser Val Thr Val Asn Val Trp Ala Ile Ala Arg Asp Pro
385             390             395             400

Glu Ala Trp Asp Ser Pro Leu Glu Phe Arg Pro Glu Arg Phe Leu His
                405             410             415

Asp Asn Ile Asp Ile Lys Gly Cys Asp Tyr Arg Val Leu Pro Phe Gly
            420             425             430

Ala Gly Arg Arg Val Cys Pro Gly Ala Gln Leu Gly Ile Asn Leu Val
        435             440             445

Ala Ser Met Ile Gly His Leu Leu His His Phe Thr Trp Ala Leu Pro
    450             455             460

Asp Gly Thr Arg Pro Glu Asp Ile Asp Met Met Glu Ser Pro Gly Leu
465             470             475             480

Ile Thr Phe Met Arg Thr Pro Leu Gln Val Val Ala Thr Pro Arg Leu
            485             490             495

Glu Lys Glu Glu Leu Tyr Lys Arg Val Ala Ala Glu Met
        500             505
```

```
<210> 29
<211> 1539
<212> DNA
<213> Ocimum basilicum

<220>
<221> CDS
<222> (1)..(1539)

<400> 29
atg gca gct ctc ctc ctc ctc ctc ctc ctt ccc ctc ctc ctt ccc gcc    48
Met Ala Ala Leu Leu Leu Leu Leu Leu Leu Pro Leu Leu Leu Pro Ala
  1               5                  10                  15

att ttc ctc ctc cac cac ctc tat tac cgc ctt cgc ttc cgc ctc cct    96
Ile Phe Leu Leu His His Leu Tyr Tyr Arg Leu Arg Phe Arg Leu Pro
                20                  25                  30

ccg ggc ccc cgc cct ctt ccc atc gtc ggc aac ctc tac gac gtc aaa   144
Pro Gly Pro Arg Pro Leu Pro Ile Val Gly Asn Leu Tyr Asp Val Lys
            35                  40                  45

ccc gtc cgt ttc cgc tgc ttc gcc gac tgg gct cag tcc tat ggc ccc   192
Pro Val Arg Phe Arg Cys Phe Ala Asp Trp Ala Gln Ser Tyr Gly Pro
        50                  55                  60

atc att tcc gtc tgg ttc gga tcc acc tta aac gtc atc gtt tcc aac   240
Ile Ile Ser Val Trp Phe Gly Ser Thr Leu Asn Val Ile Val Ser Asn
```

|  | 65 |  |  |  |  | 70 |  |  |  |  | 75 |  |  |  |  | 80 |  |  |
|---|---|---|---|---|---|---|---|---|---|---|---|---|---|---|---|---|---|---|

```
acc gaa ttg gcc aaa gaa gtc ctc aag gag aag gac cag cag ctg gcc    288
Thr Glu Leu Ala Lys Glu Val Leu Lys Glu Lys Asp Gln Gln Leu Ala
                85                  90                  95

gac cgc cat cgc agc cga tcc gcc gcc aag ttc agc agg gac ggc cag    336
Asp Arg His Arg Ser Arg Ser Ala Ala Lys Phe Ser Arg Asp Gly Gln
            100                 105                 110

gat ctg att tgg gcg gat tat ggg ccg cac tac gtc aag gtc aga aaa    384
Asp Leu Ile Trp Ala Asp Tyr Gly Pro His Tyr Val Lys Val Arg Lys
        115                 120                 125

gtt tgt acg ctg gag cta ttt tcc ccc aag cga ctt gaa gct ttg agg    432
Val Cys Thr Leu Glu Leu Phe Ser Pro Lys Arg Leu Glu Ala Leu Arg
    130                 135                 140

cca atc agg gaa gat gaa gtc acc gct atg gtt gaa tca att tac cac    480
Pro Ile Arg Glu Asp Glu Val Thr Ala Met Val Glu Ser Ile Tyr His
145                 150                 155                 160

gac tgc act gct cca gat aac gca ggg aag agc ttg cta gtg aag aag    528
Asp Cys Thr Ala Pro Asp Asn Ala Gly Lys Ser Leu Leu Val Lys Lys
                165                 170                 175

tat ctt gga gca gtg gca ttc aac aac att acc aga ctc gca ttt ggg    576
Tyr Leu Gly Ala Val Ala Phe Asn Asn Ile Thr Arg Leu Ala Phe Gly
            180                 185                 190

aaa cga ttc gtg aat tct gaa gga att ata gac aag caa ggg ctg gaa    624
Lys Arg Phe Val Asn Ser Glu Gly Ile Ile Asp Lys Gln Gly Leu Glu
        195                 200                 205

ttc aag gcc att gtt tcc aat gga cta aag ctt ggt gct tcc cta gct    672
Phe Lys Ala Ile Val Ser Asn Gly Leu Lys Leu Gly Ala Ser Leu Ala
        210                 215                 220

atg gct gag cat atc cca tcg ctc cgc tgg atg ttc cca ctc gat gaa    720
Met Ala Glu His Ile Pro Ser Leu Arg Trp Met Phe Pro Leu Asp Glu
225                 230                 235                 240

gac gct ttt gcc aaa cat gga gca cgc cgc gac caa ctc act cgc gag    768
Asp Ala Phe Ala Lys His Gly Ala Arg Arg Asp Gln Leu Thr Arg Glu
                245                 250                 255

ata atg gaa gag cac aca cgt gct cgt gaa gaa agt gga ggc gcc aaa    816
Ile Met Glu Glu His Thr Arg Ala Arg Glu Glu Ser Gly Gly Ala Lys
                260                 265                 270

caa cac ttt ttt gat gct ttg ctt aca ctt aaa gat aaa tac gac cta    864
Gln His Phe Phe Asp Ala Leu Leu Thr Leu Lys Asp Lys Tyr Asp Leu
            275                 280                 285

agt gag gac acc atc att ggt ctt ctc tgg gac atg ata act gca ggg    912
Ser Glu Asp Thr Ile Ile Gly Leu Leu Trp Asp Met Ile Thr Ala Gly
        290                 295                 300

atg gac aca act gca ata tct gtt gaa tgg gcc atg gcc gaa ctg atc    960
Met Asp Thr Thr Ala Ile Ser Val Glu Trp Ala Met Ala Glu Leu Ile
305                 310                 315                 320
```

```
aag aat cct cga gtc caa caa aag gct cag gag gag cta gac cgt gta    1008
Lys Asn Pro Arg Val Gln Gln Lys Ala Gln Glu Glu Leu Asp Arg Val
                325                 330                 335

att ggt tat gaa cgc gtg atg act gaa ttg gac ttt tca aac ctt cct    1056
Ile Gly Tyr Glu Arg Val Met Thr Glu Leu Asp Phe Ser Asn Leu Pro
                340                 345                 350

tat ctg caa tgt gtg gcc aag gaa gca ctg agg ttg cac cct cca act    1104
Tyr Leu Gln Cys Val Ala Lys Glu Ala Leu Arg Leu His Pro Pro Thr
            355                 360                 365

cca ctt atg ctc cct cac cga tcg aat tca aac gtg aag att ggt ggc    1152
Pro Leu Met Leu Pro His Arg Ser Asn Ser Asn Val Lys Ile Gly Gly
        370                 375                 380

tac gat ata ccc aag gga tca aat gtg cat gtc aat gtg tgg gcg gta    1200
Tyr Asp Ile Pro Lys Gly Ser Asn Val His Val Asn Val Trp Ala Val
385                 390                 395                 400

gcc cgt gat cct gct gtg tgg aag aac cct tgt gag ttt agg ccg gag    1248
Ala Arg Asp Pro Ala Val Trp Lys Asn Pro Cys Glu Phe Arg Pro Glu
                405                 410                 415

aga ttc ttg gaa gag gat gtt gac atg aaa ggg cac gat ttt cga ctt    1296
Arg Phe Leu Glu Glu Asp Val Asp Met Lys Gly His Asp Phe Arg Leu
                420                 425                 430

ctt cca ttt ggt gcg gga aga aga gtg tgc cca ggt gcc caa ctg ggt    1344
Leu Pro Phe Gly Ala Gly Arg Arg Val Cys Pro Gly Ala Gln Leu Gly
            435                 440                 445

ata aat ttg gtg aca tct atg ata gga cac ctc ttg cac cac ttc aac    1392
Ile Asn Leu Val Thr Ser Met Ile Gly His Leu Leu His His Phe Asn
        450                 455                 460

tgg gct cct ccc agt gga gtg agc agc gac gag ttg gac atg gga gag    1440
Trp Ala Pro Pro Ser Gly Val Ser Ser Asp Glu Leu Asp Met Gly Glu
465                 470                 475                 480

aac ccg ggt ctc gtt acc tat atg agg act cca ttg gag gca gtg cct    1488
Asn Pro Gly Leu Val Thr Tyr Met Arg Thr Pro Leu Glu Ala Val Pro
                485                 490                 495

act cct aga ttg ccc tcc gat ctc tac aag cgt att gct gtg gac ttg    1536
Thr Pro Arg Leu Pro Ser Asp Leu Tyr Lys Arg Ile Ala Val Asp Leu
            500                 505                 510

taa                                                                 1539
```

```
<210> 30
<211> 512
<212> PRT
<213> Ocimum basilicum

<400> 30
Met Ala Ala Leu Leu Leu Leu Leu Leu Leu Pro Leu Leu Leu Pro Ala
 1               5                   10                  15
```

```
Ile Phe Leu Leu His His Leu Tyr Tyr Arg Leu Arg Phe Arg Leu Pro
            20              25                  30

Pro Gly Pro Arg Pro Leu Pro Ile Val Gly Asn Leu Tyr Asp Val Lys
        35              40                  45

Pro Val Arg Phe Arg Cys Phe Ala Asp Trp Ala Gln Ser Tyr Gly Pro
        50              55                  60

Ile Ile Ser Val Trp Phe Gly Ser Thr Leu Asn Val Ile Val Ser Asn
65              70                  75                      80

Thr Glu Leu Ala Lys Glu Val Leu Lys Glu Lys Asp Gln Gln Leu Ala
                85                  90                  95

Asp Arg His Arg Ser Arg Ser Ala Ala Lys Phe Ser Arg Asp Gly Gln
            100             105                 110

Asp Leu Ile Trp Ala Asp Tyr Gly Pro His Tyr Val Lys Val Arg Lys
            115             120                 125

Val Cys Thr Leu Glu Leu Phe Ser Pro Lys Arg Leu Glu Ala Leu Arg
        130             135                 140

Pro Ile Arg Glu Asp Glu Val Thr Ala Met Val Glu Ser Ile Tyr His
145             150                 155                 160

Asp Cys Thr Ala Pro Asp Asn Ala Gly Lys Ser Leu Leu Val Lys Lys
                165                 170                 175

Tyr Leu Gly Ala Val Ala Phe Asn Asn Ile Thr Arg Leu Ala Phe Gly
            180             185                 190

Lys Arg Phe Val Asn Ser Glu Gly Ile Ile Asp Lys Gln Gly Leu Glu
        195             200                 205

Phe Lys Ala Ile Val Ser Asn Gly Leu Lys Leu Gly Ala Ser Leu Ala
        210             215                 220

Met Ala Glu His Ile Pro Ser Leu Arg Trp Met Phe Pro Leu Asp Glu
225             230                 235                 240

Asp Ala Phe Ala Lys His Gly Ala Arg Arg Asp Gln Leu Thr Arg Glu
                245             250                 255

Ile Met Glu Glu His Thr Arg Ala Arg Glu Glu Ser Gly Gly Ala Lys
            260             265                 270

Gln His Phe Phe Asp Ala Leu Leu Thr Leu Lys Asp Lys Tyr Asp Leu
        275             280                 285

Ser Glu Asp Thr Ile Ile Gly Leu Leu Trp Asp Met Ile Thr Ala Gly
        290             295                 300

Met Asp Thr Thr Ala Ile Ser Val Glu Trp Ala Met Ala Glu Leu Ile
305             310                 315                 320

Lys Asn Pro Arg Val Gln Gln Lys Ala Gln Glu Glu Leu Asp Arg Val
                325             330                 335
```

47

```
Ile Gly Tyr Glu Arg Val Met Thr Glu Leu Asp Phe Ser Asn Leu Pro
            340                 345                 350

Tyr Leu Gln Cys Val Ala Lys Glu Ala Leu Arg Leu His Pro Pro Thr
            355                 360                 365

Pro Leu Met Leu Pro His Arg Ser Asn Ser Asn Val Lys Ile Gly Gly
        370             375                 380

Tyr Asp Ile Pro Lys Gly Ser Asn Val His Val Asn Val Trp Ala Val
385             390                 395                     400

Ala Arg Asp Pro Ala Val Trp Lys Asn Pro Cys Glu Phe Arg Pro Glu
            405                 410                 415

Arg Phe Leu Glu Glu Asp Val Asp Met Lys Gly His Asp Phe Arg Leu
            420                 425                 430

Leu Pro Phe Gly Ala Gly Arg Arg Val Cys Pro Gly Ala Gln Leu Gly
            435                 440                 445

Ile Asn Leu Val Thr Ser Met Ile Gly His Leu Leu His His Phe Asn
        450                 455                 460

Trp Ala Pro Pro Ser Gly Val Ser Ser Asp Glu Leu Asp Met Gly Glu
465                 470                 475                     480

Asn Pro Gly Leu Val Thr Tyr Met Arg Thr Pro Leu Glu Ala Val Pro
            485                 490                 495

Thr Pro Arg Leu Pro Ser Asp Leu Tyr Lys Arg Ile Ala Val Asp Leu
            500                 505                 510
```

```
<210> 31
<211> 1530
<212> DNA
<213> Ocimum basilicum

<220>
<221> CDS
<222> (1)..(1530)

<400> 31
atg gca gct ctc ctc ctc ctt ctc ctc ctc ctt ccc gcc att ttc ctc    48
Met Ala Ala Leu Leu Leu Leu Leu Leu Leu Pro Ala Ile Phe Leu
  1               5                  10                  15

ctc cac cac ctc tat tac cgc ctt cgc ttc cgc ctc cct ccg ggc ccc    96
Leu His His Leu Tyr Tyr Arg Leu Arg Phe Arg Leu Pro Pro Gly Pro
                20                  25                  30

cgc cct ctt ccc gtc gtc ggc aac ctc tac gac gtc aaa ccc gtc cgt   144
Arg Pro Leu Pro Val Val Gly Asn Leu Tyr Asp Val Lys Pro Val Arg
            35                  40                  45

ttc cgc tgc ttc gcc gac tgg gct cag tcc tat ggc ccc atc att tcc   192
Phe Arg Cys Phe Ala Asp Trp Ala Gln Ser Tyr Gly Pro Ile Ile Ser
        50                  55                  60
```

```
gtc tgg ttc gga tcc acc tta aac gtc atc gtt tcc aac acc gaa ttg    240
Val Trp Phe Gly Ser Thr Leu Asn Val Ile Val Ser Asn Thr Glu Leu
 65              70              75              80

gcc aaa gaa gtc ctc aag gag aag gac cag cag ctg gcc gac cgc cat    288
Ala Lys Glu Val Leu Lys Glu Lys Asp Gln Gln Leu Ala Asp Arg His
             85              90              95

cgc agc cga tcc gcc gcc aag ttc agc agg gac ggc cag gat ctg att    336
Arg Ser Arg Ser Ala Ala Lys Phe Ser Arg Asp Gly Gln Asp Leu Ile
         100             105             110

tgg gcg gat tat ggg ccg cac tac gtc aag gtc aga aaa gtt tgt atg    384
Trp Ala Asp Tyr Gly Pro His Tyr Val Lys Val Arg Lys Val Cys Met
         115             120             125

ctg gag cta ttt tcc ccc aag cga ctt gaa gct ttg agg cca atc agg    432
Leu Glu Leu Phe Ser Pro Lys Arg Leu Glu Ala Leu Arg Pro Ile Arg
     130             135             140

gag gat gaa gtc acc gct atg gtt gag tca att tac cac gat tgc act    480
Glu Asp Glu Val Thr Ala Met Val Glu Ser Ile Tyr His Asp Cys Thr
145             150             155             160

gct cca gat aac gca ggg aag agc ttg cta gtg aag aag tat ctt gga    528
Ala Pro Asp Asn Ala Gly Lys Ser Leu Leu Val Lys Lys Tyr Leu Gly
             165             170             175

gca gtg gca ttc aac aac att acc aga ctc gca ttt ggg aaa cga ttt    576
Ala Val Ala Phe Asn Asn Ile Thr Arg Leu Ala Phe Gly Lys Arg Phe
         180             185             190

gtg aat tct gaa gga ata ata gac aag caa ggg ctg gaa ttc aag gcc    624
Val Asn Ser Glu Gly Ile Ile Asp Lys Gln Gly Leu Glu Phe Lys Ala
         195             200             205

att gtt tcc aat gga cta aag ctt ggt gct tcc cta gct atg gct gag    672
Ile Val Ser Asn Gly Leu Lys Leu Gly Ala Ser Leu Ala Met Ala Glu
     210             215             220

cat atc cca tgg ctc cgc tgg atg ttc cca ctc gat gaa gac gct ttc    720
His Ile Pro Trp Leu Arg Trp Met Phe Pro Leu Asp Glu Asp Ala Phe
225             230             235             240

gcc aaa cat gga gca cgc cgc gac caa ctc act cgc gag ata atg gaa    768
Ala Lys His Gly Ala Arg Arg Asp Gln Leu Thr Arg Glu Ile Met Glu
             245             250             255

gag cac act cgt gct cgt gaa gaa agt gga ggc gcc aaa caa cac ttt    816
Glu His Thr Arg Ala Arg Glu Glu Ser Gly Gly Ala Lys Gln His Phe
         260             265             270

ttt gat gct ttg ctt aca ctt aaa gat aaa tac gac cta agt gag gac    864
Phe Asp Ala Leu Leu Thr Leu Lys Asp Lys Tyr Asp Leu Ser Glu Asp
         275             280             285

acc atc att ggt ctt ctc tgg gac atg ata act gca ggg atg gac aca    912
Thr Ile Ile Gly Leu Leu Trp Asp Met Ile Thr Ala Gly Met Asp Thr
         290             295             300

act gca ata tct gtc gaa tgg gcc atg gcc gaa ctg atc aag aat ccg    960
```

```
      Thr Ala Ile Ser Val Glu Trp Ala Met Ala Glu Leu Ile Lys Asn Pro
      305                 310                 315                 320

      cga gtc caa caa aag gct cag gag gag cta gac cgc gta att ggt tac   1008
      Arg Val Gln Gln Lys Ala Gln Glu Glu Leu Asp Arg Val Ile Gly Tyr
                      325                 330                 335

      gaa cgc gtg atg act gaa ttg gac ttt tca aac ctt cct tat ctg caa   1056
      Glu Arg Val Met Thr Glu Leu Asp Phe Ser Asn Leu Pro Tyr Leu Gln
                  340                 345                 350

      tgt gtg gcc aag gaa gca ctg agg ttg cac cct cca act cca ctt atg   1104
      Cys Val Ala Lys Glu Ala Leu Arg Leu His Pro Pro Thr Pro Leu Met
                  355                 360                 365

      ctc cct cac cga tcg aat tca aac gtg aag att ggt ggc tac gat ata   1152
      Leu Pro His Arg Ser Asn Ser Asn Val Lys Ile Gly Gly Tyr Asp Ile
              370                 375                 380

      ccc aag gga tca aat gtg cat gtc aat gtg tgg gcg gta gcc cgt gat   1200
      Pro Lys Gly Ser Asn Val His Val Asn Val Trp Ala Val Ala Arg Asp
      385                 390                 395                 400

      cct gct gtg tgg aag aac cct tct gag ttt agg ccg gag aga ttc ttg   1248
      Pro Ala Val Trp Lys Asn Pro Ser Glu Phe Arg Pro Glu Arg Phe Leu
                      405                 410                 415

      gaa gag gat gtt gac atg aaa ggg cac gat ttt cga ctt ctt cca ttt   1296
      Glu Glu Asp Val Asp Met Lys Gly His Asp Phe Arg Leu Leu Pro Phe
                  420                 425                 430

      ggt gcg gga aga aga gtg tgc ccg ggt gcc caa ctg ggt ata aat ttg   1344
      Gly Ala Gly Arg Arg Val Cys Pro Gly Ala Gln Leu Gly Ile Asn Leu
              435                 440                 445

      gtg aca tct atg ata gga cac ctc ttg cac cac ttc aac tgg gct cct   1392
      Val Thr Ser Met Ile Gly His Leu Leu His His Phe Asn Trp Ala Pro
              450                 455                 460

      ccc agt gga gtg agc acc gac gag ttg gac atg gga gag aac ccg ggt   1440
      Pro Ser Gly Val Ser Thr Asp Glu Leu Asp Met Gly Glu Asn Pro Gly
      465                 470                 475                 480

      ctc gtt acc tat atg agg act cca ttg gaa gcg gtg cct act cct aga   1488
      Leu Val Thr Tyr Met Arg Thr Pro Leu Glu Ala Val Pro Thr Pro Arg
                      485                 490                 495

      ttg ccc tcc gat ctc tac aag cgt att gct gtg gac ttg taa           1530
      Leu Pro Ser Asp Leu Tyr Lys Arg Ile Ala Val Asp Leu
                  500                 505                 510
```

```
<210> 32
<211> 509
<212> PRT
<213> Ocimum basilicum

<400> 32
Met Ala Ala Leu Leu Leu Leu Leu Leu Leu Leu Pro Ala Ile Phe Leu
  1               5                  10                  15
```

```
Leu His His Leu Tyr Tyr Arg Leu Arg Phe Arg Leu Pro Pro Gly Pro
         20              25              30

Arg Pro Leu Pro Val Val Gly Asn Leu Tyr Asp Val Lys Pro Val Arg
         35              40              45

Phe Arg Cys Phe Ala Asp Trp Ala Gln Ser Tyr Gly Pro Ile Ile Ser
     50              55              60

Val Trp Phe Gly Ser Thr Leu Asn Val Ile Val Ser Asn Thr Glu Leu
 65              70              75              80

Ala Lys Glu Val Leu Lys Glu Lys Asp Gln Gln Leu Ala Asp Arg His
             85              90              95

Arg Ser Arg Ser Ala Ala Lys Phe Ser Arg Asp Gly Gln Asp Leu Ile
         100             105             110

Trp Ala Asp Tyr Gly Pro His Tyr Val Lys Val Arg Lys Val Cys Met
         115             120             125

Leu Glu Leu Phe Ser Pro Lys Arg Leu Glu Ala Leu Arg Pro Ile Arg
     130             135             140

Glu Asp Glu Val Thr Ala Met Val Glu Ser Ile Tyr His Asp Cys Thr
145             150             155             160

Ala Pro Asp Asn Ala Gly Lys Ser Leu Leu Val Lys Lys Tyr Leu Gly
             165             170             175

Ala Val Ala Phe Asn Asn Ile Thr Arg Leu Ala Phe Gly Lys Arg Phe
         180             185             190

Val Asn Ser Glu Gly Ile Ile Asp Lys Gln Gly Leu Glu Phe Lys Ala
         195             200             205

Ile Val Ser Asn Gly Leu Lys Leu Gly Ala Ser Leu Ala Met Ala Glu
     210             215             220

His Ile Pro Trp Leu Arg Trp Met Phe Pro Leu Asp Glu Asp Ala Phe
225             230             235             240

Ala Lys His Gly Ala Arg Arg Asp Gln Leu Thr Arg Glu Ile Met Glu
             245             250             255

Glu His Thr Arg Ala Arg Glu Glu Ser Gly Gly Ala Lys Gln His Phe
         260             265             270

Phe Asp Ala Leu Leu Thr Leu Lys Asp Lys Tyr Asp Leu Ser Glu Asp
         275             280             285

Thr Ile Ile Gly Leu Leu Trp Asp Met Ile Thr Ala Gly Met Asp Thr
     290             295             300

Thr Ala Ile Ser Val Glu Trp Ala Met Ala Glu Leu Ile Lys Asn Pro
305             310             315             320

Arg Val Gln Gln Lys Ala Gln Glu Glu Leu Asp Arg Val Ile Gly Tyr
             325             330             335

Glu Arg Val Met Thr Glu Leu Asp Phe Ser Asn Leu Pro Tyr Leu Gln
```

```
                    340                    345                        350

         Cys Val Ala Lys Glu Ala Leu Arg Leu His Pro Pro Thr Pro Leu Met
             355                 360                 365

         Leu Pro His Arg Ser Asn Ser Asn Val Lys Ile Gly Gly Tyr Asp Ile
             370                 375                 380

         Pro Lys Gly Ser Asn Val His Val Asn Val Trp Ala Val Ala Arg Asp
         385                 390                 395                 400

         Pro Ala Val Trp Lys Asn Pro Ser Glu Phe Arg Pro Glu Arg Phe Leu
                         405                 410                 415

         Glu Glu Asp Val Asp Met Lys Gly His Asp Phe Arg Leu Leu Pro Phe
                     420                 425                 430

         Gly Ala Gly Arg Arg Val Cys Pro Gly Ala Gln Leu Gly Ile Asn Leu
                 435                 440                 445

         Val Thr Ser Met Ile Gly His Leu Leu His His Phe Asn Trp Ala Pro
             450                 455                 460

         Pro Ser Gly Val Ser Thr Asp Glu Leu Asp Met Gly Glu Asn Pro Gly
         465                 470                 475                 480

         Leu Val Thr Tyr Met Arg Thr Pro Leu Glu Ala Val Pro Thr Pro Arg
                         485                 490                 495

         Leu Pro Ser Asp Leu Tyr Lys Arg Ile Ala Val Asp Leu
                     500                 505


         <210> 33
         <211> 1524
         <212> DNA
         <213> Solenostemon scutellarioides

         <220>
         <221> CDS
         <222> (1)..(1524)

         <400> 33
         atg act tct cta ctc gct cat cct ctt ctc cct ccc cct cgc cgt cgt    48
         Met Thr Ser Leu Leu Ala His Pro Leu Leu Pro Pro Pro Arg Arg Arg
          1               5                  10                  15

         cct cta cca ccg ctt cta ccg cct ccg gta tcg cat ccc gcc ggc cct    96
         Pro Leu Pro Pro Leu Leu Pro Pro Pro Val Ser His Pro Ala Gly Pro
                     20                  25                  30

         cgc cct ggc cgg tgg tgg agg aat ctc tac gat gtc aag ccg gtt cga   144
         Arg Pro Gly Arg Trp Trp Arg Asn Leu Tyr Asp Val Lys Pro Val Arg
                 35                  40                  45

         ttc cgc tgc ttc gcg gag tgg gcc cag ttg ttc ggt cct acc ttt tcg   192
         Phe Arg Cys Phe Ala Glu Trp Ala Gln Leu Phe Gly Pro Thr Phe Ser
             50                  55                  60

         gtg tgg ttt ggc tcc acg ctt aat gtt atc gtt tcg agc tcg gag ctg   240
```

52

```
              Val Trp Phe Gly Ser Thr Leu Asn Val Ile Val Ser Ser Ser Glu Leu
              65              70              75                  80

gct aag gag gtg ttg aag gag aag gac ggg cag ctg gct gac ccg ccc      288
Ala Lys Glu Val Leu Lys Glu Lys Asp Gly Gln Leu Ala Asp Pro Pro
                85              90                  95

cgc agc cga tct gcg gtg aag ctg agc aga gac ggg aag gat ttg atc      336
Arg Ser Arg Ser Ala Val Lys Leu Ser Arg Asp Gly Lys Asp Leu Ile
            100             105             110

tgg gcg gat tat ggg cct cac tat gtg aag gtg agg aag gtt tgc acg      384
Trp Ala Asp Tyr Gly Pro His Tyr Val Lys Val Arg Lys Val Cys Thr
            115             120             125

gtg gtg ctg ttt tcg ccg aag agg ctg gag ctg ttg cgg ccg att agg      432
Val Val Leu Phe Ser Pro Lys Arg Leu Glu Leu Leu Arg Pro Ile Arg
        130             135             140

gaa gat gag att acg gtt atg gtg gaa tcg att tat caa gat tca gct      480
Glu Asp Glu Ile Thr Val Met Val Glu Ser Ile Tyr Gln Asp Ser Ala
145             150             155             160

gct tct ggg aag agc gtg gtg ata aag aaa tac ctt gca tca atg gcg      528
Ala Ser Gly Lys Ser Val Val Ile Lys Lys Tyr Leu Ala Ser Met Ala
                165             170             175

ttc cac aac atc acg agg ctg gta ttt ggg aag cgt ttc gtg aat tct      576
Phe His Asn Ile Thr Arg Leu Val Phe Gly Lys Arg Phe Val Asn Ser
            180             185             190

gaa ggg gaa gtt gac aag caa ggg cag gag ttc aag gcc att gct atc      624
Glu Gly Glu Val Asp Lys Gln Gly Gln Glu Phe Lys Ala Ile Ala Ile
        195             200             205

aac ggt ctg aag ctg ggt gct tct ctc gcc gtg tcg gag cac atc ccc      672
Asn Gly Leu Lys Leu Gly Ala Ser Leu Ala Val Ser Glu His Ile Pro
        210             215             220

tgg ctc cgg tgg atg ttc cca ctc gat gaa gac gcc ttc acc cag cac      720
Trp Leu Arg Trp Met Phe Pro Leu Asp Glu Asp Ala Phe Thr Gln His
225             230             235             240

ggc gtt cgg atg gag cgt ctc acc cga gaa atc atg caa gaa cac acc      768
Gly Val Arg Met Glu Arg Leu Thr Arg Glu Ile Met Gln Glu His Thr
            245             250             255

ctc gcc cgc cag aaa acc ggc ggc gcc aag cag cat ttc ttc gac gcg      816
Leu Ala Arg Gln Lys Thr Gly Gly Ala Lys Gln His Phe Phe Asp Ala
            260             265             270

ctg ctc act ctc aag gat gaa tac gat ctc agc gag gac acc atc atc      864
Leu Leu Thr Leu Lys Asp Glu Tyr Asp Leu Ser Glu Asp Thr Ile Ile
            275             280             285

gcc ctt ctc tgg gat atg atc gcg gcg ggg atg gac acg ccg gcg ata      912
Ala Leu Leu Trp Asp Met Ile Ala Ala Gly Met Asp Thr Pro Ala Ile
        290             295             300

tcg gtg gag tgg gcg atg gcg gag ctg gtg agg aac ccg agg gtg caa      960
Ser Val Glu Trp Ala Met Ala Glu Leu Val Arg Asn Pro Arg Val Gln
```

53

```
         305                    310                    315                    320

gag aaa gtg cag gag gag ttg gac cgt gtg ata ggc cat gaa cgg atc    1008
Glu Lys Val Gln Glu Glu Leu Asp Arg Val Ile Gly His Glu Arg Ile
                325                    330                    335

atg acg gag cta gac atc cca aac ctt ccc tac cta caa tgc gtg gtg    1056
Met Thr Glu Leu Asp Ile Pro Asn Leu Pro Tyr Leu Gln Cys Val Val
                340                    345                    350

aag gag tct ctc aga ctt cac cct cca act cct gtc atg ctt ccc cac    1104
Lys Glu Ser Leu Arg Leu His Pro Pro Thr Pro Val Met Leu Pro His
                355                    360                    365

cgc tcc aac gcc gat gtc aag atc ggt ggc tac gac atc cca aag ggc    1152
Arg Ser Asn Ala Asp Val Lys Ile Gly Gly Tyr Asp Ile Pro Lys Gly
            370                    375                    380

tcg aat gtg cac gtg aat gta tgg gcc atc gca cgt gac ccc aag tca    1200
Ser Asn Val His Val Asn Val Trp Ala Ile Ala Arg Asp Pro Lys Ser
385                    390                    395                    400

tgg aag gtt cca ctc gag ttc cgg ccg gag agg ttc ttg gag gag gat    1248
Trp Lys Val Pro Leu Glu Phe Arg Pro Glu Arg Phe Leu Glu Glu Asp
                405                    410                    415

gtg gat atc aag gga cat gat ttc cgg ctt ctg cgt ttt ggt gct ggg    1296
Val Asp Ile Lys Gly His Asp Phe Arg Leu Leu Arg Phe Gly Ala Gly
                420                    425                    430

aga aga gtg tgc ccc ggt gca cag cta ggt atc gac ctc gcc act tcg    1344
Arg Arg Val Cys Pro Gly Ala Gln Leu Gly Ile Asp Leu Ala Thr Ser
            435                    440                    445

atg ata ggt cac ctt ctg cac cac ttc cgg tgg act cct ccg act gga    1392
Met Ile Gly His Leu Leu His His Phe Arg Trp Thr Pro Pro Thr Gly
            450                    455                    460

gtg agg gcg gag gac atc gac atg ggg gag aat ccg ggg acg gtg acg    1440
Val Arg Ala Glu Asp Ile Asp Met Gly Glu Asn Pro Gly Thr Val Thr
465                    470                    475                    480

tac atg agg act ccg gtg gag gca gtt cct act ccc aga ttg ccg gcc    1488
Tyr Met Arg Thr Pro Val Glu Ala Val Pro Thr Pro Arg Leu Pro Ala
                485                    490                    495

aac ttg tat aag cgt gtg gct gtg gtg gat att tga    1524
Asn Leu Tyr Lys Arg Val Ala Val Val Asp Ile
            500                    505


<210> 34
<211> 507
<212> PRT
<213> Solenostemon scutellarioides

<400> 34
Met Thr Ser Leu Leu Ala His Pro Leu Leu Pro Pro Pro Arg Arg Arg
 1               5                   10                  15

Pro Leu Pro Pro Leu Leu Pro Pro Pro Val Ser His Pro Ala Gly Pro
```

54

```
                     20                        25                        30

      Arg Pro Gly Arg Trp Trp Arg Asn Leu Tyr Asp Val Lys Pro Val Arg
              35                  40                  45

      Phe Arg Cys Phe Ala Glu Trp Ala Gln Leu Phe Gly Pro Thr Phe Ser
          50                  55                  60

      Val Trp Phe Gly Ser Thr Leu Asn Val Ile Val Ser Ser Ser Glu Leu
      65                  70                  75                      80

      Ala Lys Glu Val Leu Lys Glu Lys Asp Gly Gln Leu Ala Asp Pro Pro
                      85                  90                      95

      Arg Ser Arg Ser Ala Val Lys Leu Ser Arg Asp Gly Lys Asp Leu Ile
                  100             105                 110

      Trp Ala Asp Tyr Gly Pro His Tyr Val Lys Val Arg Lys Val Cys Thr
              115                 120                 125

      Val Val Leu Phe Ser Pro Lys Arg Leu Glu Leu Leu Arg Pro Ile Arg
          130                 135                 140

      Glu Asp Glu Ile Thr Val Met Val Glu Ser Ile Tyr Gln Asp Ser Ala
      145                 150                 155                 160

      Ala Ser Gly Lys Ser Val Val Ile Lys Lys Tyr Leu Ala Ser Met Ala
                      165                 170                 175

      Phe His Asn Ile Thr Arg Leu Val Phe Gly Lys Arg Phe Val Asn Ser
                  180                 185                 190

      Glu Gly Glu Val Asp Lys Gln Gly Gln Glu Phe Lys Ala Ile Ala Ile
                  195                 200                 205

      Asn Gly Leu Lys Leu Gly Ala Ser Leu Ala Val Ser Glu His Ile Pro
          210                 215                 220

      Trp Leu Arg Trp Met Phe Pro Leu Asp Glu Asp Ala Phe Thr Gln His
      225                 230                 235                 240

      Gly Val Arg Met Glu Arg Leu Thr Arg Glu Ile Met Gln Glu His Thr
                      245                 250                 255

      Leu Ala Arg Gln Lys Thr Gly Gly Ala Lys Gln His Phe Phe Asp Ala
                  260                 265                 270

      Leu Leu Thr Leu Lys Asp Glu Tyr Asp Leu Ser Glu Asp Thr Ile Ile
                  275                 280                 285

      Ala Leu Leu Trp Asp Met Ile Ala Ala Gly Met Asp Thr Pro Ala Ile
                  290                 295                 300

      Ser Val Glu Trp Ala Met Ala Glu Leu Val Arg Asn Pro Arg Val Gln
      305                 310                 315                 320

      Glu Lys Val Gln Glu Glu Leu Asp Arg Val Ile Gly His Glu Arg Ile
                      325                 330                 335

      Met Thr Glu Leu Asp Ile Pro Asn Leu Pro Tyr Leu Gln Cys Val Val
                  340                 345                 350
```

```
Lys Glu Ser Leu Arg Leu His Pro Pro Thr Pro Val Met Leu Pro His
        355             360             365

Arg Ser Asn Ala Asp Val Lys Ile Gly Gly Tyr Asp Ile Pro Lys Gly
    370             375             380

Ser Asn Val His Val Asn Val Trp Ala Ile Ala Arg Asp Pro Lys Ser
385             390             395             400

Trp Lys Val Pro Leu Glu Phe Arg Pro Glu Arg Phe Leu Glu Glu Asp
            405             410             415

Val Asp Ile Lys Gly His Asp Phe Arg Leu Leu Arg Phe Gly Ala Gly
            420             425             430

Arg Arg Val Cys Pro Gly Ala Gln Leu Gly Ile Asp Leu Ala Thr Ser
        435             440             445

Met Ile Gly His Leu Leu His His Phe Arg Trp Thr Pro Pro Thr Gly
    450             455             460

Val Arg Ala Glu Asp Ile Asp Met Gly Glu Asn Pro Gly Thr Val Thr
465             470             475             480

Tyr Met Arg Thr Pro Val Glu Ala Val Pro Thr Pro Arg Leu Pro Ala
            485             490             495

Asn Leu Tyr Lys Arg Val Ala Val Val Asp Ile
            500             505
```

```
<210> 35
<211> 1539
<212> DNA
<213> Pinus taeda

<220>
<221> CDS
<222> (1)..(1539)

<400> 35
atg tct gtt cct gaa atg ggt ccg ttt gga gtt gtg gtg act ggt att      48
Met Ser Val Pro Glu Met Gly Pro Phe Gly Val Val Val Thr Gly Ile
  1               5                   10                  15

gtg gcc att gcc ata gtt tac aag ttg gtg caa cgg tgg aga ttc aag      96
Val Ala Ile Ala Ile Val Tyr Lys Leu Val Gln Arg Trp Arg Phe Lys
                20                  25                  30

ctg cca cca ggt ccc aga cca tgg cct gtg gtg ggg aat ctg cta cag     144
Leu Pro Pro Gly Pro Arg Pro Trp Pro Val Val Gly Asn Leu Leu Gln
            35                  40                  45

ata gaa cct gtg aga ttt aga tgc ttc tgg gac tgg tcc aag aaa tat     192
Ile Glu Pro Val Arg Phe Arg Cys Phe Trp Asp Trp Ser Lys Lys Tyr
        50                  55                  60

ggg cca atc atg tcc gtg tgg ttt gga tcc aca ctc aat gta gtt gtg     240
Gly Pro Ile Met Ser Val Trp Phe Gly Ser Thr Leu Asn Val Val Val
```

56

|  | 65 |  |  |  |  | 70 |  |  |  |  | 75 |  |  |  |  | 80 |  |  |
|---|---|---|---|---|---|---|---|---|---|---|---|---|---|---|---|---|---|---|

```
tcc aat aca gag ctg gct aag gag gtt ctc aag gag cac gat caa cag    288
Ser Asn Thr Glu Leu Ala Lys Glu Val Leu Lys Glu His Asp Gln Gln
                85                  90                  95

ctt gcc gac agg ccc aga tcc aga tcg gct gag aaa ttt agc aga aat    336
Leu Ala Asp Arg Pro Arg Ser Arg Ser Ala Glu Lys Phe Ser Arg Asn
            100                 105                 110

ggc caa gac ttg att tgg gct gac tat ggt cct cat tat gtg aag gtt    384
Gly Gln Asp Leu Ile Trp Ala Asp Tyr Gly Pro His Tyr Val Lys Val
            115                 120                 125

agg aag gtc tgt act ctg gag ctc ttc tct ccc aag aga ttg gag gcc    432
Arg Lys Val Cys Thr Leu Glu Leu Phe Ser Pro Lys Arg Leu Glu Ala
        130                 135                 140

ctc agg cct atc aga gaa gac gag gtt gca gcc atg gta gaa tcc ata    480
Leu Arg Pro Ile Arg Glu Asp Glu Val Ala Ala Met Val Glu Ser Ile
145                 150                 155                 160

ttc aat gac tgt ggc aaa caa gaa gga atc ggc aag ccc ttg gtg gtg    528
Phe Asn Asp Cys Gly Lys Gln Glu Gly Ile Gly Lys Pro Leu Val Val
                165                 170                 175

aag aga tat ctg tca ggt gtg gca ttc aac aat atc acc agg cct gct    576
Lys Arg Tyr Leu Ser Gly Val Ala Phe Asn Asn Ile Thr Arg Pro Ala
            180                 185                 190

ttt ggg aaa aga ttt gtg aat gaa gaa ggg aag atg gat cca caa ggt    624
Phe Gly Lys Arg Phe Val Asn Glu Glu Gly Lys Met Asp Pro Gln Gly
            195                 200                 205

gtg gag ttt aag gaa att gtt gca aca ggg ttg aag ctg ggg gct tcc    672
Val Glu Phe Lys Glu Ile Val Ala Thr Gly Leu Lys Leu Gly Ala Ser
        210                 215                 220

ctc acc atg gcc gag cac att ccc tat ctc cgc tgg atg ttt cca ttg    720
Leu Thr Met Ala Glu His Ile Pro Tyr Leu Arg Trp Met Phe Pro Leu
225                 230                 235                 240

gaa gag ggt gcg ttc gcc aag cat ggt gca agg aga gac aat gtc acc    768
Glu Glu Gly Ala Phe Ala Lys His Gly Ala Arg Arg Asp Asn Val Thr
                245                 250                 255

aaa gct atc atg gaa gag cat aca ctt gcc agg cag acg agt ggt gcc    816
Lys Ala Ile Met Glu Glu His Thr Leu Ala Arg Gln Thr Ser Gly Ala
            260                 265                 270

aaa caa cat ttt gtg gat gca ttg ctc act cta caa gaa aag tat gat    864
Lys Gln His Phe Val Asp Ala Leu Leu Thr Leu Gln Glu Lys Tyr Asp
            275                 280                 285

ctt agt gag gat act att att ggc ctc ctc tgg gat atg atc aca gca    912
Leu Ser Glu Asp Thr Ile Ile Gly Leu Leu Trp Asp Met Ile Thr Ala
            290                 295                 300

ggt atg gat aca aca gca ata act gta gaa tgg gcc atg gct gaa ctt    960
Gly Met Asp Thr Thr Ala Ile Thr Val Glu Trp Ala Met Ala Glu Leu
305                 310                 315                 320
```

```
gtt cgc aat ccc cga att caa cag aaa gca cag gaa gag ata gat aga    1008
Val Arg Asn Pro Arg Ile Gln Gln Lys Ala Gln Glu Glu Ile Asp Arg
            325             330             335

gtg gtt ggc agg gac aga gtg atg aat gaa act gat ttt cct cat cta    1056
Val Val Gly Arg Asp Arg Val Met Asn Glu Thr Asp Phe Pro His Leu
            340             345             350

cca tac ctg caa tgc atc aca aaa gag gct ctc cgc ttg cat cca ccc    1104
Pro Tyr Leu Gln Cys Ile Thr Lys Glu Ala Leu Arg Leu His Pro Pro
            355             360             365

act cct ctg atg ctt cca cat aaa gca acg cag aat gtc aag ata ggt    1152
Thr Pro Leu Met Leu Pro His Lys Ala Thr Gln Asn Val Lys Ile Gly
            370             375             380

ggc tat gat ata ccc aaa gga tcc aat gta cat gta aat gtg tgg gcc    1200
Gly Tyr Asp Ile Pro Lys Gly Ser Asn Val His Val Asn Val Trp Ala
385             390             395             400

att gct cgg gat ccg gct gta tgg aag gac cca gtg acc ttc agg cct    1248
Ile Ala Arg Asp Pro Ala Val Trp Lys Asp Pro Val Thr Phe Arg Pro
            405             410             415

gag aga ttt ctt gag gaa gat gtt gat att aag ggc cat gat tat aga    1296
Glu Arg Phe Leu Glu Glu Asp Val Asp Ile Lys Gly His Asp Tyr Arg
            420             425             430

cta ctg cca ttt ggt gca ggg cgc agg atc tgt cct ggt gca caa ttg    1344
Leu Leu Pro Phe Gly Ala Gly Arg Arg Ile Cys Pro Gly Ala Gln Leu
            435             440             445

ggt att aat tta gtt cag tct atg ttg gga cac ctg ctt cat cat ttc    1392
Gly Ile Asn Leu Val Gln Ser Met Leu Gly His Leu Leu His His Phe
            450             455             460

gta tgg gca cct cct gag gga atg cag gca gaa gac ata gat ctc aca    1440
Val Trp Ala Pro Pro Glu Gly Met Gln Ala Glu Asp Ile Asp Leu Thr
465             470             475             480

gag aat cca ggg ctt gtt act ttc atg gcc aag cct gtg cag gcc att    1488
Glu Asn Pro Gly Leu Val Thr Phe Met Ala Lys Pro Val Gln Ala Ile
            485             490             495

gct att cct cga ttg cct gat cat ctc tac aag cga caa cca ctc aat    1536
Ala Ile Pro Arg Leu Pro Asp His Leu Tyr Lys Arg Gln Pro Leu Asn
            500             505             510

tga                                                                1539
```

```
<210> 36
<211> 512
<212> PRT
<213> Pinus taeda

<400> 36
Met Ser Val Pro Glu Met Gly Pro Phe Gly Val Val Val Thr Gly Ile
1               5               10              15
```

```
Val Ala Ile Ala Ile Val Tyr Lys Leu Val Gln Arg Trp Arg Phe Lys
            20                  25                  30

Leu Pro Pro Gly Pro Arg Pro Trp Pro Val Val Gly Asn Leu Leu Gln
            35                  40                  45

Ile Glu Pro Val Arg Phe Arg Cys Phe Trp Asp Trp Ser Lys Lys Tyr
        50                  55                  60

Gly Pro Ile Met Ser Val Trp Phe Gly Ser Thr Leu Asn Val Val Val
65                  70                  75                  80

Ser Asn Thr Glu Leu Ala Lys Glu Val Leu Lys Glu His Asp Gln Gln
                85                  90                  95

Leu Ala Asp Arg Pro Arg Ser Arg Ser Ala Glu Lys Phe Ser Arg Asn
            100                 105                 110

Gly Gln Asp Leu Ile Trp Ala Asp Tyr Gly Pro His Tyr Val Lys Val
            115                 120                 125

Arg Lys Val Cys Thr Leu Glu Leu Phe Ser Pro Lys Arg Leu Glu Ala
    130                 135                 140

Leu Arg Pro Ile Arg Glu Asp Glu Val Ala Ala Met Val Glu Ser Ile
145                 150                 155                 160

Phe Asn Asp Cys Gly Lys Gln Glu Gly Ile Gly Lys Pro Leu Val Val
                165                 170                 175

Lys Arg Tyr Leu Ser Gly Val Ala Phe Asn Asn Ile Thr Arg Pro Ala
            180                 185                 190

Phe Gly Lys Arg Phe Val Asn Glu Glu Gly Lys Met Asp Pro Gln Gly
            195                 200                 205

Val Glu Phe Lys Glu Ile Val Ala Thr Gly Leu Lys Leu Gly Ala Ser
    210                 215                 220

Leu Thr Met Ala Glu His Ile Pro Tyr Leu Arg Trp Met Phe Pro Leu
225                 230                 235                 240

Glu Glu Gly Ala Phe Ala Lys His Gly Ala Arg Arg Asp Asn Val Thr
                245                 250                 255

Lys Ala Ile Met Glu Glu His Thr Leu Ala Arg Gln Thr Ser Gly Ala
            260                 265                 270

Lys Gln His Phe Val Asp Ala Leu Leu Thr Leu Gln Glu Lys Tyr Asp
            275                 280                 285

Leu Ser Glu Asp Thr Ile Ile Gly Leu Leu Trp Asp Met Ile Thr Ala
    290                 295                 300

Gly Met Asp Thr Thr Ala Ile Thr Val Glu Trp Ala Met Ala Glu Leu
305                 310                 315                 320

Val Arg Asn Pro Arg Ile Gln Gln Lys Ala Gln Glu Glu Ile Asp Arg
                325                 330                 335
```

```
Val Val Gly Arg Asp Arg Val Met Asn Glu Thr Asp Phe Pro His Leu
        340             345             350

Pro Tyr Leu Gln Cys Ile Thr Lys Glu Ala Leu Arg Leu His Pro Pro
        355             360             365

Thr Pro Leu Met Leu Pro His Lys Ala Thr Gln Asn Val Lys Ile Gly
    370             375             380

Gly Tyr Asp Ile Pro Lys Gly Ser Asn Val His Val Asn Val Trp Ala
385             390             395             400

Ile Ala Arg Asp Pro Ala Val Trp Lys Asp Pro Val Thr Phe Arg Pro
            405             410             415

Glu Arg Phe Leu Glu Glu Asp Val Asp Ile Lys Gly His Asp Tyr Arg
        420             425             430

Leu Leu Pro Phe Gly Ala Gly Arg Arg Ile Cys Pro Gly Ala Gln Leu
        435             440             445

Gly Ile Asn Leu Val Gln Ser Met Leu Gly His Leu Leu His His Phe
    450             455             460

Val Trp Ala Pro Pro Glu Gly Met Gln Ala Glu Asp Ile Asp Leu Thr
465             470             475             480

Glu Asn Pro Gly Leu Val Thr Phe Met Ala Lys Pro Val Gln Ala Ile
            485             490             495

Ala Ile Pro Arg Leu Pro Asp His Leu Tyr Lys Arg Gln Pro Leu Asn
            500             505             510


<210> 37
<211> 1530
<212> DNA
<213> Ammi majus

<220>
<221> CDS
<222> (1)..(1530)

<400> 37
atg gct ctc ttc ctc tac ctg atc atc cct tgc acc ata atc ctc ctc      48
Met Ala Leu Phe Leu Tyr Leu Ile Ile Pro Cys Thr Ile Ile Leu Leu
  1               5                  10                  15

cac cag ctc tac cac aag cta aga ttc aag ctc cca ccc ggc cct cgt      96
His Gln Leu Tyr His Lys Leu Arg Phe Lys Leu Pro Pro Gly Pro Arg
            20                  25                  30

cca tgg cct atc gta ggc aat ctt tac gac atc gaa ccg gtg agg ttc     144
Pro Trp Pro Ile Val Gly Asn Leu Tyr Asp Ile Glu Pro Val Arg Phe
        35                  40                  45

cgg tgc ttc aac aac tgg tcc aag act tat ggt cct ata ata tca gtt     192
Arg Cys Phe Asn Asn Trp Ser Lys Thr Tyr Gly Pro Ile Ile Ser Val
    50                  55                  60
```

```
tgg ttt gga tca act ctg aat gtt att gtg aat aac act gag cta gct    240
Trp Phe Gly Ser Thr Leu Asn Val Ile Val Asn Asn Thr Glu Leu Ala
 65              70              75                      80

aaa gaa gtg ttg aaa gac aag gat caa cag ctg gct gat cgg cat cga    288
Lys Glu Val Leu Lys Asp Lys Asp Gln Gln Leu Ala Asp Arg His Arg
             85              90                      95

agc cga tca gca gct aag ttt agt aga gat ggc cag gat tta ata tgg    336
Ser Arg Ser Ala Ala Lys Phe Ser Arg Asp Gly Gln Asp Leu Ile Trp
            100             105             110

gct gat tat gga cct cat tat gtt aag gtt agg aaa gtt tgt aca ctt    384
Ala Asp Tyr Gly Pro His Tyr Val Lys Val Arg Lys Val Cys Thr Leu
            115             120             125

gag ctg ttt act cct aag aga ctt gaa gct atc agg cct gtt aga gaa    432
Glu Leu Phe Thr Pro Lys Arg Leu Glu Ala Ile Arg Pro Val Arg Glu
        130             135             140

gat gaa gtt act gct atg gtg gaa tcc att tac aaa gat tgt act aac    480
Asp Glu Val Thr Ala Met Val Glu Ser Ile Tyr Lys Asp Cys Thr Asn
145             150             155             160

tca gat acg ata ggg aaa agt ttg ctg gtg agg cag tat cta gga ggt    528
Ser Asp Thr Ile Gly Lys Ser Leu Leu Val Arg Gln Tyr Leu Gly Gly
            165             170             175

gtt gca ttc aac aac atc acc agg ctt gct ttt gga aaa cga ttc gtg    576
Val Ala Phe Asn Asn Ile Thr Arg Leu Ala Phe Gly Lys Arg Phe Val
            180             185             190

aac tcg gag ggg gtg atg gat gag caa ggc aag gag ttc aag tct att    624
Asn Ser Glu Gly Val Met Asp Glu Gln Gly Lys Glu Phe Lys Ser Ile
        195             200             205

att gct aat ggt cta aaa ctt ggt gcc tct ctt gca aca gct gag cac    672
Ile Ala Asn Gly Leu Lys Leu Gly Ala Ser Leu Ala Thr Ala Glu His
        210             215             220

att caa tgg ctc cga tgg ttg ttt cca ctg gaa gag gaa gca ttt gca    720
Ile Gln Trp Leu Arg Trp Leu Phe Pro Leu Glu Glu Glu Ala Phe Ala
225             230             235             240

aaa cat gga gcg cgc agg gat aat ctc act cgt gcc atc atg gaa gaa    768
Lys His Gly Ala Arg Arg Asp Asn Leu Thr Arg Ala Ile Met Glu Glu
            245             250             255

cac act ctt gct cgc caa aag tcc ggt ggc acc aaa caa cat ttt gtt    816
His Thr Leu Ala Arg Gln Lys Ser Gly Gly Thr Lys Gln His Phe Val
            260             265             270

gat gca ttg ctc acc ctt caa tct aaa tat gac ctt agt gag gac acc    864
Asp Ala Leu Leu Thr Leu Gln Ser Lys Tyr Asp Leu Ser Glu Asp Thr
            275             280             285

att att ggt cta ctt tgg gat atg att acg gca gga gcg gac aca act    912
Ile Ile Gly Leu Leu Trp Asp Met Ile Thr Ala Gly Ala Asp Thr Thr
        290             295             300

gca att gta gta gaa tgg ggc atg gca gaa ctc att aag aac cca agg    960
```

```
       Ala Ile Val Val Glu Trp Gly Met Ala Glu Leu Ile Lys Asn Pro Arg
       305                 310                 315                 320

       gtg caa gaa aaa gcc caa gag gag ctt gac cgt gta ata gga tat gag    1008
       Val Gln Glu Lys Ala Gln Glu Glu Leu Asp Arg Val Ile Gly Tyr Glu
                       325                 330                 335

       cgt gtg tta act gaa ctg gat ttc tcg aat ctc ccc tac ctt caa tgt    1056
       Arg Val Leu Thr Glu Leu Asp Phe Ser Asn Leu Pro Tyr Leu Gln Cys
                   340                 345                 350

       gtg gct aaa gag gct cta cga cta cat ccc cca act ccc ctc atg ctc    1104
       Val Ala Lys Glu Ala Leu Arg Leu His Pro Pro Thr Pro Leu Met Leu
                   355                 360                 365

       cca cac cgt gcc aat gcc aat gtc aaa ata ggt gga tat gac att ccc    1152
       Pro His Arg Ala Asn Ala Asn Val Lys Ile Gly Gly Tyr Asp Ile Pro
           370                 375                 380

       aag ggt tca aat gtg cat gta aac gta tgg gcc gtg gct cgt gac cct    1200
       Lys Gly Ser Asn Val His Val Asn Val Trp Ala Val Ala Arg Asp Pro
       385                 390                 395                 400

       gct gtt tgg aaa aat cca tta gag ttc agg cct gaa cgg ttc tta gag    1248
       Ala Val Trp Lys Asn Pro Leu Glu Phe Arg Pro Glu Arg Phe Leu Glu
                       405                 410                 415

       gag gat gtt gac atg aaa ggg cat gat tat cgt cta cta cca ttc gga    1296
       Glu Asp Val Asp Met Lys Gly His Asp Tyr Arg Leu Leu Pro Phe Gly
                   420                 425                 430

       gct gga aga aga gtt tgc cct ggg gca caa tta ggg atc aac ttg gta    1344
       Ala Gly Arg Arg Val Cys Pro Gly Ala Gln Leu Gly Ile Asn Leu Val
                   435                 440                 445

       aca tca atg ttg ggt cat ctc ttg cac cac tac tca tgg gca cca cct    1392
       Thr Ser Met Leu Gly His Leu Leu His His Tyr Ser Trp Ala Pro Pro
           450                 455                 460

       agc ggg ttg agc tca gat gag att gac atg tca gag agc cct gga atg    1440
       Ser Gly Leu Ser Ser Asp Glu Ile Asp Met Ser Glu Ser Pro Gly Met
       465                 470                 475                 480

       gtg act tac atg aaa aca ccc ctg caa gct gtt cca aca ccg agg ttg    1488
       Val Thr Tyr Met Lys Thr Pro Leu Gln Ala Val Pro Thr Pro Arg Leu
                       485                 490                 495

       cca agt cag ttg tac aaa cgt ttg gca gtg gag gac atg taa           1530
       Pro Ser Gln Leu Tyr Lys Arg Leu Ala Val Glu Asp Met
                   500                 505                 510


       <210> 38
       <211> 509
       <212> PRT
       <213> Ammi majus

       <400> 38
       Met Ala Leu Phe Leu Tyr Leu Ile Ile Pro Cys Thr Ile Ile Leu Leu
       1               5                   10                  15
```

```
His Gln Leu Tyr His Lys Leu Arg Phe Lys Leu Pro Pro Gly Pro Arg
            20                  25              30

Pro Trp Pro Ile Val Gly Asn Leu Tyr Asp Ile Glu Pro Val Arg Phe
            35                  40              45

Arg Cys Phe Asn Asn Trp Ser Lys Thr Tyr Gly Pro Ile Ile Ser Val
    50                  55                  60

Trp Phe Gly Ser Thr Leu Asn Val Ile Val Asn Asn Thr Glu Leu Ala
65                  70                  75                  80

Lys Glu Val Leu Lys Asp Lys Asp Gln Gln Leu Ala Asp Arg His Arg
                85                  90                  95

Ser Arg Ser Ala Ala Lys Phe Ser Arg Asp Gly Gln Asp Leu Ile Trp
                100                 105                 110

Ala Asp Tyr Gly Pro His Tyr Val Lys Val Arg Lys Val Cys Thr Leu
            115                 120                 125

Glu Leu Phe Thr Pro Lys Arg Leu Glu Ala Ile Arg Pro Val Arg Glu
        130                 135                 140

Asp Glu Val Thr Ala Met Val Glu Ser Ile Tyr Lys Asp Cys Thr Asn
145                 150                 155                 160

Ser Asp Thr Ile Gly Lys Ser Leu Leu Val Arg Gln Tyr Leu Gly Gly
                165                 170                 175

Val Ala Phe Asn Asn Ile Thr Arg Leu Ala Phe Gly Lys Arg Phe Val
                180                 185                 190

Asn Ser Glu Gly Val Met Asp Glu Gln Gly Lys Glu Phe Lys Ser Ile
                195                 200                 205

Ile Ala Asn Gly Leu Lys Leu Gly Ala Ser Leu Ala Thr Ala Glu His
        210                 215                 220

Ile Gln Trp Leu Arg Trp Leu Phe Pro Leu Glu Glu Glu Ala Phe Ala
225                 230                 235                 240

Lys His Gly Ala Arg Arg Asp Asn Leu Thr Arg Ala Ile Met Glu Glu
                245                 250                 255

His Thr Leu Ala Arg Gln Lys Ser Gly Gly Thr Lys Gln His Phe Val
                260                 265                 270

Asp Ala Leu Leu Thr Leu Gln Ser Lys Tyr Asp Leu Ser Glu Asp Thr
            275                 280                 285

Ile Ile Gly Leu Leu Trp Asp Met Ile Thr Ala Gly Ala Asp Thr Thr
        290                 295                 300

Ala Ile Val Val Glu Trp Gly Met Ala Glu Leu Ile Lys Asn Pro Arg
305                 310                 315                 320

Val Gln Glu Lys Ala Gln Glu Glu Leu Asp Arg Val Ile Gly Tyr Glu
                325                 330                 335

Arg Val Leu Thr Glu Leu Asp Phe Ser Asn Leu Pro Tyr Leu Gln Cys
```

```
                340                      345                         350

      Val Ala Lys Glu Ala Leu Arg Leu His Pro Pro Thr Pro Leu Met Leu
              355             360                 365

      Pro His Arg Ala Asn Ala Asn Val Lys Ile Gly Gly Tyr Asp Ile Pro
          370             375                 380

      Lys Gly Ser Asn Val His Val Asn Val Trp Ala Val Ala Arg Asp Pro
      385             390                 395                     400

      Ala Val Trp Lys Asn Pro Leu Glu Phe Arg Pro Glu Arg Phe Leu Glu
                  405             410                 415

      Glu Asp Val Asp Met Lys Gly His Asp Tyr Arg Leu Leu Pro Phe Gly
              420             425                 430

      Ala Gly Arg Arg Val Cys Pro Gly Ala Gln Leu Gly Ile Asn Leu Val
              435             440                 445

      Thr Ser Met Leu Gly His Leu Leu His His Tyr Ser Trp Ala Pro Pro
          450             455                 460

      Ser Gly Leu Ser Ser Asp Glu Ile Asp Met Ser Glu Ser Pro Gly Met
      465             470                 475                     480

      Val Thr Tyr Met Lys Thr Pro Leu Gln Ala Val Pro Thr Pro Arg Leu
                  485             490                 495

      Pro Ser Gln Leu Tyr Lys Arg Leu Ala Val Glu Asp Met
                  500             505
```

```
<210> 39
<211> 1530
<212> DNA
<213> Camptotheca acuminata

<220>
<221> CDS
<222> (1)..(1530)

<400> 39
atg gcg gtg ctt cta tta ttc ctc cct ctc atc ttc ata ttc ctt gct      48
Met Ala Val Leu Leu Leu Phe Leu Pro Leu Ile Phe Ile Phe Leu Ala
  1               5                  10                  15

tac aaa ctc tat caa cgg ctt aga ttc aag ctc ccg ccg gga ccc cgc      96
Tyr Lys Leu Tyr Gln Arg Leu Arg Phe Lys Leu Pro Pro Gly Pro Arg
              20                  25                  30

cct ttg ccg ttc gtc gga aac ctt tac agc gtg aaa ccg gtg aag ttc     144
Pro Leu Pro Phe Val Gly Asn Leu Tyr Ser Val Lys Pro Val Lys Phe
          35                  40                  45

cgg tgc ttc gcc gaa tgg gca caa gtt tat ggt ccg atc atg tcg gtg     192
Arg Cys Phe Ala Glu Trp Ala Gln Val Tyr Gly Pro Ile Met Ser Val
      50                  55                  60

tgg ttc ggt tcg act tta aac gta gtg atc tca aac tca gag ttg gct     240
```

```
        Trp Phe Gly Ser Thr Leu Asn Val Val Ile Ser Asn Ser Glu Leu Ala
        65                  70                  75                  80

aaa gaa gta ctc aag gaa aat gat caa cat tta gca gat agg gaa aga       288
Lys Glu Val Leu Lys Glu Asn Asp Gln His Leu Ala Asp Arg Glu Arg
                85                  90                  95

aat aga tca tca aat tca ttc agc aga ggt ggg aag gat ctg ata tgg       336
Asn Arg Ser Ser Asn Ser Phe Ser Arg Gly Gly Lys Asp Leu Ile Trp
            100                 105                 110

gct gat tat ggc cct cac tat gtc aag gtt aga aag ctg tgt act gtt       384
Ala Asp Tyr Gly Pro His Tyr Val Lys Val Arg Lys Leu Cys Thr Val
            115                 120                 125

gaa ctc ttc act cca aag agg ctt gaa gct ctt aga cct att cga gaa       432
Glu Leu Phe Thr Pro Lys Arg Leu Glu Ala Leu Arg Pro Ile Arg Glu
        130                 135                 140

gat gaa gtt act gcc atg gtt gac tcc att ttc aaa gac tcg gcc aat       480
Asp Glu Val Thr Ala Met Val Asp Ser Ile Phe Lys Asp Ser Ala Asn
145                 150                 155                 160

cct gat aat tat ggg aaa agc ttg ttg gtg aag cag tac ata gga gca       528
Pro Asp Asn Tyr Gly Lys Ser Leu Leu Val Lys Gln Tyr Ile Gly Ala
            165                 170                 175

gta gca ttc aac aac ata acg aga ctt gta ttt ggg aag cgg ttt atg       576
Val Ala Phe Asn Asn Ile Thr Arg Leu Val Phe Gly Lys Arg Phe Met
            180                 185                 190

aac tcg gag ggg gtg atg gat gag caa ggg aag gaa ttc aga ggg att       624
Asn Ser Glu Gly Val Met Asp Glu Gln Gly Lys Glu Phe Arg Gly Ile
            195                 200                 205

gtg gcc aat ggc gtc aag att ggt gga ttg cgt ttc att gga gag cac       672
Val Ala Asn Gly Val Lys Ile Gly Gly Leu Arg Phe Ile Gly Glu His
            210                 215                 220

att cca tgg ctt cgt tgc atg ttc cca cag gag gaa gag ata ttg gtt       720
Ile Pro Trp Leu Arg Cys Met Phe Pro Gln Glu Glu Glu Ile Leu Val
225                 230                 235                 240

aaa cat gaa gca cgt agg gat cga ctc acc agg gcc atc atg gaa gag       768
Lys His Glu Ala Arg Arg Asp Arg Leu Thr Arg Ala Ile Met Glu Glu
            245                 250                 255

cac aca ctt gcc cgc aag cac tct ggc ggt gcc aag cag cat ttt gtc       816
His Thr Leu Ala Arg Lys His Ser Gly Gly Ala Lys Gln His Phe Val
            260                 265                 270

gat gcc ttg ctt act ctt cag aag gaa tat gaa ctc agt gat gac act       864
Asp Ala Leu Leu Thr Leu Gln Lys Glu Tyr Glu Leu Ser Asp Asp Thr
            275                 280                 285

gtc att ggc ctc ctt tgg gac atg atc act gca ggc atg gac aca acg       912
Val Ile Gly Leu Leu Trp Asp Met Ile Thr Ala Gly Met Asp Thr Thr
            290                 295                 300

tcg atc tca gtt gag tgg gcg atg gcc gag ctg atc aag aac cca aga       960
Ser Ile Ser Val Glu Trp Ala Met Ala Glu Leu Ile Lys Asn Pro Arg
```

```
         305                    310                    315                    320
gtg caa cag aag gct caa gag gag cta gac cgc gtg att gga tat gaa    1008
Val Gln Gln Lys Ala Gln Glu Glu Leu Asp Arg Val Ile Gly Tyr Glu
                325                    330                    335

cgt atc atg tct gaa act gac ttc ccg aac ctc cca tac cta caa tgt    1056
Arg Ile Met Ser Glu Thr Asp Phe Pro Asn Leu Pro Tyr Leu Gln Cys
                340                    345                    350

gta gct aag gaa gcg ctg agg ttg cac cct cca acc cca ctc atg ctt    1104
Val Ala Lys Glu Ala Leu Arg Leu His Pro Pro Thr Pro Leu Met Leu
                355                    360                    365

cca cac aag gcc aat tcc aat gtc aag att ggt ggc tat gac atc cct    1152
Pro His Lys Ala Asn Ser Asn Val Lys Ile Gly Gly Tyr Asp Ile Pro
                370                    375                    380

aaa ggc tct att gtg cac gta aat gta tgg gcc att gct cgt gat cca    1200
Lys Gly Ser Ile Val His Val Asn Val Trp Ala Ile Ala Arg Asp Pro
385                    390                    395                    400

gca acc tgg aaa gac cct cac gag ttc cgg cct gaa cgt ttc ctt gag    1248
Ala Thr Trp Lys Asp Pro His Glu Phe Arg Pro Glu Arg Phe Leu Glu
                405                    410                    415

gaa gat gtt gac atg aag ggt cat gat ttt agg cta cta ccc ttt ggt    1296
Glu Asp Val Asp Met Lys Gly His Asp Phe Arg Leu Leu Pro Phe Gly
                420                    425                    430

gca gga agg cgt atc tgc cct ggt gct cag atc gcc att aac ttg atc    1344
Ala Gly Arg Arg Ile Cys Pro Gly Ala Gln Ile Ala Ile Asn Leu Ile
                435                    440                    445

aca tcg atg ctt ggt cac ctg ttg cac cat ttt tct tgg gca cca cct    1392
Thr Ser Met Leu Gly His Leu Leu His His Phe Ser Trp Ala Pro Pro
                450                    455                    460

gaa ggg gtc aag cca gaa gaa atc gac atg act gag aac cct gga ttg    1440
Glu Gly Val Lys Pro Glu Glu Ile Asp Met Thr Glu Asn Pro Gly Leu
465                    470                    475                    480

gtt act ttt atg aag aca cca gta caa gct gtt gct aaa cca aga ttg    1488
Val Thr Phe Met Lys Thr Pro Val Gln Ala Val Ala Lys Pro Arg Leu
                485                    490                    495

cct tca cat ttg tac aaa cgt gtg gct gtg ggt gct gtg tga            1530
Pro Ser His Leu Tyr Lys Arg Val Ala Val Gly Ala Val
                500                    505                    510
```

<210> 40
<211> 509
<212> PRT
<213> Camptotheca acuminata

<400> 40
Met Ala Val Leu Leu Leu Phe Leu Pro Leu Ile Phe Ile Phe Leu Ala
 1               5                   10                  15

Tyr Lys Leu Tyr Gln Arg Leu Arg Phe Lys Leu Pro Pro Gly Pro Arg

```
                      20                        25                        30

         Pro Leu Pro Phe Val Gly Asn Leu Tyr Ser Val Lys Pro Val Lys Phe
                 35                  40                  45

         Arg Cys Phe Ala Glu Trp Ala Gln Val Tyr Gly Pro Ile Met Ser Val
                 50                  55                  60

         Trp Phe Gly Ser Thr Leu Asn Val Val Ile Ser Asn Ser Glu Leu Ala
         65                  70                  75                  80

         Lys Glu Val Leu Lys Glu Asn Asp Gln His Leu Ala Asp Arg Glu Arg
                         85                  90                  95

         Asn Arg Ser Ser Asn Ser Phe Ser Arg Gly Gly Lys Asp Leu Ile Trp
                     100                 105                 110

         Ala Asp Tyr Gly Pro His Tyr Val Lys Val Arg Lys Leu Cys Thr Val
                     115                 120                 125

         Glu Leu Phe Thr Pro Lys Arg Leu Glu Ala Leu Arg Pro Ile Arg Glu
                     130                 135                 140

         Asp Glu Val Thr Ala Met Val Asp Ser Ile Phe Lys Asp Ser Ala Asn
         145                 150                 155                 160

         Pro Asp Asn Tyr Gly Lys Ser Leu Leu Val Lys Gln Tyr Ile Gly Ala
                     165                 170                 175

         Val Ala Phe Asn Asn Ile Thr Arg Leu Val Phe Gly Lys Arg Phe Met
                     180                 185                 190

         Asn Ser Glu Gly Val Met Asp Glu Gln Gly Lys Glu Phe Arg Gly Ile
                     195                 200                 205

         Val Ala Asn Gly Val Lys Ile Gly Gly Leu Arg Phe Ile Gly Glu His
                     210                 215                 220

         Ile Pro Trp Leu Arg Cys Met Phe Pro Gln Glu Glu Glu Ile Leu Val
         225                 230                 235                 240

         Lys His Glu Ala Arg Arg Asp Arg Leu Thr Arg Ala Ile Met Glu Glu
                     245                 250                 255

         His Thr Leu Ala Arg Lys His Ser Gly Gly Ala Lys Gln His Phe Val
                     260                 265                 270

         Asp Ala Leu Leu Thr Leu Gln Lys Glu Tyr Glu Leu Ser Asp Asp Thr
                     275                 280                 285

         Val Ile Gly Leu Leu Trp Asp Met Ile Thr Ala Gly Met Asp Thr Thr
                     290                 295                 300

         Ser Ile Ser Val Glu Trp Ala Met Ala Glu Leu Ile Lys Asn Pro Arg
         305                 310                 315                 320

         Val Gln Gln Lys Ala Gln Glu Glu Leu Asp Arg Val Ile Gly Tyr Glu
                     325                 330                 335

         Arg Ile Met Ser Glu Thr Asp Phe Pro Asn Leu Pro Tyr Leu Gln Cys
                     340                 345                 350
```

```
Val Ala Lys Glu Ala Leu Arg Leu His Pro Pro Thr Pro Leu Met Leu
        355             360             365

Pro His Lys Ala Asn Ser Asn Val Lys Ile Gly Gly Tyr Asp Ile Pro
    370             375             380

Lys Gly Ser Ile Val His Val Asn Val Trp Ala Ile Ala Arg Asp Pro
385             390             395             400

Ala Thr Trp Lys Asp Pro His Glu Phe Arg Pro Glu Arg Phe Leu Glu
            405             410             415

Glu Asp Val Asp Met Lys Gly His Asp Phe Arg Leu Leu Pro Phe Gly
            420             425             430

Ala Gly Arg Arg Ile Cys Pro Gly Ala Gln Ile Ala Ile Asn Leu Ile
        435             440             445

Thr Ser Met Leu Gly His Leu Leu His His Phe Ser Trp Ala Pro Pro
    450             455             460

Glu Gly Val Lys Pro Glu Glu Ile Asp Met Thr Glu Asn Pro Gly Leu
465             470             475             480

Val Thr Phe Met Lys Thr Pro Val Gln Ala Val Ala Lys Pro Arg Leu
            485             490             495

Pro Ser His Leu Tyr Lys Arg Val Ala Val Gly Ala Val
            500             505
```